(19) Europäisches Patentamt / European Patent Office / Office européen des brevets

(11) **EP 3 904 883 A1**

(12) **EUROPEAN PATENT APPLICATION**

(43) Date of publication:
**03.11.2021 Bulletin 2021/44**

(51) Int Cl.:
***G01N 33/68*** *(2006.01)*

(21) Application number: **20168527.8**

(22) Date of filing: **07.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sciomics GmbH**
**69115 Heidelberg (DE)**

(72) Inventors:
• **Griesbeck, Anne**
**69221 Dossenheim (DE)**
• **Lowy, Camille**
**69123 Heidelberg (DE)**

• **Skwirblies, Florian**
**69239 Neckarsteinach (DE)**
• **Klein, Marco**
**69151 Neckargemünd (DE)**
• **Schröder, Christoph**
**69118 Heidelberg (DE)**

(74) Representative: **Fuchs Patentanwälte Partnerschaft mbB**
**Westhafenplatz 1**
**60327 Frankfurt am Main (DE)**

Remarks:
The complete document including Reference Tables and the Sequence Listing can be downloaded from the EPO website

(54) **PREDICTION AND EARLY DIAGNOSIS OF ACUTE KIDNEY INJURY**

(57) The present invention relates to prediction and early diagnosis of acute kidney injury (AKI). The method provides biomarkers that correlate with a patient's risk of developing AKI, or with the presence of early stage AKI. The invention also provides devices and kits for predicting the risk of occurrence of AKI and for diagnosing AKI.

EP 3 904 883 A1

## Description

Summary

The present invention pertains to the field of organ failure prediction and early diagnosis. In an aspect, the invention relates to a method for predicting the risk of acute kidney injury (AKI) and/or diagnosing AKI at an early time point.

The invention comprises the steps of determining the amount of at least one biomarker selected from the biomarkers shown in Table 1, and comparing the amount of said at least one biomarker with a reference amount for said at least one biomarker, whereby the risk of occurrence of AKI is predicted and/or AKI is timely diagnosed. The inventors identified different sets of biomarkers.

In an aspect, the present invention relates to a method for predicting the risk of AKI and/or diagnosing AKI at an early time point upon a medical intervention. More preferably, the present invention relates to a method for predicting the risk of AKI and/or diagnosing AKI at an early time point upon a surgical intervention. More preferably, the present invention relates to a method for predicting the risk of AKI and/or diagnosing AKI at an early time point upon solid organ transplantation (sTx) and/or upon cardiac surgery. More preferably, the present invention relates to a method for predicting the risk of AKI and/or diagnosing AKI at an early time point upon lung transplantation (LuTx), upon kidney transplantation, upon heart valve repair or replacement, upon coronary artery bypass graft (CABG), upon transmyocardial laser revascularization and/or upon heart transplantion.

The present invention also contemplates a method for stratifying subjects for a change of therapy plan in order to improve patients' outcome. The method may include stratification for additional medication to stabilize kidney function. In an embodiment, the invention may contemplate a diagnostic test useful as a companion diagnostic to a therapeutic drug to determine its applicability to a specific person (companion diagnostic test). Encompassed are, furthermore, diagnostic devices and kits for carrying out the methods disclosed herein.

## Background of the invention

### Kidney/AKI

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. Renal disease and/or injury may be acute and/or chronic. Acute kidney injury (AKI) is an abrupt (typically detected within about 48 hours to 1 week) reduction in renal function with loss of filtration capacity. This results in retention of nitrogenous and non-nitrogenous waste products that are normally excreted by the kidney resulting in acid-base imbalance and electrolyte imbalance. This results in turn in a change in urine output, overhydration and an increase in non-nitrogenous and nitrogenous metabolic waste products such as the serum renal retention parameters creatinine and urea. The symptoms accompanying AKI are very diverse and may include decreased urine production, nausea and vomiting, high blood pressure, abdominal and chest pain, edema, shortness of breath, confusion, seizure or coma, confusion or fatigue. However, AKI can also be symptom-free, especially at early stages. AKI includes diagnosis codes N17.0, N17.1, N17.2, N17.8 and N17.9 according to ICD-10 (2019).

### Etiological groups

Clinically, AKI can be classified into 3 etiological groups:

prerenal - an adaptive response to severe renal hypoperfusion due to major bleeding, hypotension and ischemia reperfusion injury,

renal or intrinsic - a response to cytotoxic, ischemic or inflammatory injury of the kidney or autoimmune diseases affecting the kidney (i.e. glomerulonephritis, interstitial nephritis and others) with structural and functional damage, and

postrenal - the result of the mechanical obstruction of the urinary tract (i.e. prostate hypertrophy, obstructive uropathy, stones)

Prerenal AKI can be the consequence of hypovolemia resulting from conditions such as hemorrhage; impaired cardiac output resulting from congestive heart failure; decreased vascular resistance resulting from conditions such as sepsis or renal vasoconstriction from vasoconstrictive medications. Prerenal AKI often leads to intrinsic AKI if not treated correctly.

Renal etiologies of AKI are very diverse and can affect all four structures of the kidney, meaning that the damages can be tubular, glomerular, interstitial or vascular. The main causes of the renal etiology of AKI are ischemia, for example as a consequence of surgery; nephrotoxicity, for example resulting from radiocontrast media or certain types of antibiotics; acute glomerulonephritis; acute interstitial nephritis or vascular injury of the kidney due for example to malignant hypertension.

Causes of postrenal AKI are characterized by an obstruction of the urinary flow, which can be due for example to prostate or gynecological cancers, fibrosis or ureteral valve disease or kidney stones.

**Prevalence of AKI**

In the Western hemisphere, AKI has become primarily a nosocomial disease and it is a very common clinical condition in critically ill patients and after major surgeries. Indeed, AKI occurs in 5 - 7% of hospitalized patients (Basile et al., 2012). In addition, incidence of AKI is 5-7.5% for all acute care hospitalizations and accounts for up to 52.6% of all patients on intensive care units (ICUs) (Fuhrman et al., 2018). Surgical intervention is a major risk factor for AKI with 50% of the patients facing AKI during the peri-operative hospital stay (Hobson et al., 2016). After solid organ tx (tx = transplantation) incidence rates are between 25% (after kidney tx) and up to 65% (after liver tx) in the peri-operative period (Kalisvaart et al., 2018). After lung tx (LuTx) AKI incidence is of 50 - 65% (Wehbe et al., 2013; Wehbe et al., 2012). Moreover, incidence of AKI is up to 70% upon hematopoietic cell transplantation (Kogon & Hingorani, 2010). The incidence of AKI in men is 1.6-fold higher than in women (Brown et al., 2016). AKI also increases patients' morbidity and the length of in-hospital stay. Furthermore, long-term consequences of AKI such as chronic kidney disease (CKD) can lead to the need of dialysis, renal replacement therapy (RRT) or kidney transplantation. Therefore, AKI is associated with significant healthcare costs.

**Classification of AKI**

The first classification system for defining and detecting AKI emerged in 2002 and was published as RIFLE (Risk, Injury, Failure, Loss of kidney function, and ESKD-End-stage kidney disease) classification in 2004 (Bellomo et al., 2004). The RIFLE classification is based on three clinical parameters (SCreat: serum creatinine or GFR: glomerular filtration rate and UO: urine output) and defines three severity classes (Risk, Injury and Failure) and two outcome classes (Loss of kidney function and End-stage kidney disease, ESKD) as follows:

"Risk": SCreat 1.5 fold increased or GFR decreased > 25 % and UO < 0.5 ml/kg/h during 6 hours;

"Injury": SCreat 2.0 fold increased or GFR decreased > 50 % and UO < 0.5 ml/kg/h during 12 hours;

"Failure": SCreat 3.0 fold increased or SCreat $\geq$ 4 mg/dl and GFR decreased > 75 % and UO < 0.3 ml/kg/h during 24 hours or anuria for at least 12 hours;

"Loss": Persistent need for renal replacement therapy for more than 4 weeks;

"ESKD": need for dialysis for more than 3 months.

As an example, a patient with a UO of more than 0.5 ml/kg/h during 6 hours and a two-fold increase in SCreat will be categorized in the Injury class. The RIFLE classification leads to a high sensitivity and a low specificity for the mild classes (Risk and Injury), i.e. patients without renal failure will be falsely included in the Risk class. On the opposite, the End-stage kidney disease class has a high specificity and a low sensitivity. Therefore, it will miss some patients.

Although the RIFLE classification has been largely validated in terms of AKI diagnosis and prognosis in various clinical settings (Lopes & Jorge, 2013), it has a number of important limitations. Among those limitations are the requirement of a baseline SCreat value, the limited accuracy of the urine output measurement and the role of several confounding factors on the urine output measurement such as diabetes, the use of diuretics or the hydration status.

In an attempt to further improve the outcome of AKI patients, the AKI Network proposed a new classification in 2007 (Mehta et al., 2007). In this classification, the two outcome classes were removed and the Risk, Injury and Failure classes were replaced by the categories AKIN1, AKIN2 and AKIN3. Furthermore, some modifications were made compared to the RIFLE classification: before diagnosing AKI, an adequate status of hydration shall be achieved and urinary obstruction shall be ruled out. The AKIN1 class is broadened compared to RISK class as it includes an increase in SCreat of more than or equal to 0.3 mg/dl even if it does not reach 1.5-fold from the baseline. In addition, the use of GFR as a clinical parameter was discarded as this added additional complexity to the classification system.

**AKIN classification for AKI as proposed by Mehta et al., 2007.**

| Stage | Serum creatinine (SCreat) criteria | Urine output criteria |
|-------|-----------------------------------|-----------------------|
| AKIN1 | Increase in SCreat of more than or equal to 0.3 mg/dl (> 26.4 $\mu$mol/l) or increase to more than or equal to 150 % to 200 % (1.5- to 2-fold) from baseline | Less than 0.5 ml/kg per hour for more than 6 hours |
| AKIN2 | Increase in SCreat to more than 200 % to 300 % (> 2- to 3-fold) from baseline | Less than 0.5 ml/kg per hour for more than 12 hours |
| AKIN3 | Increase in SCreat to more than 300 % (> 3-fold) from baseline (or more than or equal to 4.0 mg/dl [$\geq$ 354 $\mu$mol/l] with an acute increase of at least 0.5 mg/dl [44 $\mu$mol/l] | Less than 0.3 ml/kg per hour for 24 hours or anuria for 12 hours |

The present invention relies on the AKIN classification system for classifying the patients into two groups: AKI, which corresponds to AKIN stages 1, 2 and 3; and control, corresponding to patients showing no sign of AKI according to the AKIN classification.

One of the major drawbacks of the AKIN classification system is the fact that AKI can only be diagnosed if an increase (SCreat) or a drop (UO) of the clinical parameters occurs within a 48 h period. Therefore, patients with an AKI that develops within a longer period might be missed by this classification system. The AKIN classification is a slightly modified version of the RIFLE classification and some of its limitations are very similar to those of the RIFLE classification. The major common limitation of both classification systems is the fact that the clinical parameters used for the staging of the patients are influenced by various confounding aspects such as gender, age or volume status, resulting in a limited reliability. In addition, the classification systems do not allow an early detection of AKI and today there is no assay available that allows predicting the individual risk for AKI prior to surgery or ICU admission.

**Biomarkers in the field of AKI**

So far, only a few biomarkers have been described for assessing the individual risk of AKI. As far as the diagnosis of AKI is concerned, current AKI diagnostics is mainly based on monitoring serum creatinine elevation and the decrease of the glomerular filtration rate or urine output. These parameters are influenced by gender, age, and volume status and therefore they are insufficient for early detection.

Currently, discussed biomarkers for AKI prediction and diagnosis are NGAL, Kim-1, IL18, L-FABP, AGT as well as TIMP-2 and IGFBP7, both of the latter are assessed by the US Food and Drug Administration (FDA)-approved NephroCheck® (Astute Medical, Inc.) test. NephroCheck® has limited reliability (sensitivity 76%, false positive rate ~50%) and other biomarkers have the disadvantage of being only transiently (NGAL) or late stage deregulated (KIM1).

Thus, there is still a strong need for more reliable biomarkers for prediction and early diagnosis of AKI. Moreover, diagnosis and further personalized treatment of subjects with AKI would be beneficial for improving patients' outcome and for the cost-efficiency of the health system.

Proteins, as the end product or the acting products of gene expression play a vital role in all activities of a cell. Proteins, as readily available through many body fluids such as plasma, urine and tissue extracts, provide the immediate option for clinical analysis. Proteomic technologies are important for the discovery of clinically relevant biomarkers in various indications.

Detailed description of the invention

The present invention methods of predicting AKI and methods of diagnosing early stage AKI.

**Method**

The method of this invention is suitable for predicting the risk of occurrence of acute kidney injury (AKI) or for early diagnosis of AKI in a subject, comprising determining in a sample obtained from the subject the amount of at least one biomarker, and comparing the amount of the biomarker with a reference amount for the biomarker. Preferably, the biomarkers of the present invention are of human origin, including human proteins and human carbohydrates.

In an aspect, the invention relates to a method for predicting the risk of occurrence of acute kidney injury (AKI) or for early diagnosis of AKI in a subject, comprising the steps of:

a. determining in a sample obtained from the subject the amount of at least one biomarker selected from the group consisting of

CD9 antigen, Prostaglandin G/H synthase 2, CD15, CD99 antigen, High affinity immunoglobulin epsilon receptor subunit alpha, Ligands of Tumor necrosis factor receptor superfamily member 1B including Tumor necrosis factor and Lymphotoxin-alpha, Tumor necrosis factor receptor superfamily member 6, Interferon alpha-1/13, Basigin, C-C motif, chemokine 7, Dickkopf-related protein 2, Hyaluronan mediated motility receptor, Interleukin-18, Interleukin-7, Major prion protein, Receptor-type tyrosine-protein phosphatase C, P-selectin glycoprotein ligand 1, Tumor necrosis factor ligand superfamily member 14, DNA topoisomerase 2-alpha, Brain-derived neurotrophic factor, Caspase-8, Eotaxin, C-C motif chemokine 3, C-C motif chemokine 5, Monocyte differentiation antigen CD14, Cytokine receptor-like factor 2, Lamin-B1, Cellular tumor antigen p53, Serine/threonine-protein kinase PAK 1, Caspase-9, Transforming growth factor-beta-induced protein ig-h3, Leukocyte surface antigen CD47, T-cell surface glycoprotein CD8 alpha chain, Dickkopf-related protein 3, Growth arrest-specific protein 6, Interleukin-15, Cytokine receptor common subunit beta, Keratin type II cytoskeletal 8, Leukosialin, MAP/microtubule affinity-regulating kinase 4, Melanophilin, Interstitial collagenase, Matrilysin, Prostaglandin G/H synthase 1, Myeloblastin, RNA-binding protein 3, Serum amyloid P-component, Tetraspanin-16, Urokinase-type plasminogen activator, CTP synthase 1, CD139, Max dimerization protein 4, Transmembrane protein 54, Actin cytoplasmic 1, Caspase-3, Complement decay-accelerating factor, High mobility group protein B2, Homeobox protein, Hox-C11, Intercellular adhesion molecule 1, Interleukin-12 subunit alpha, Krueppel-like factor 8, Galectin-4, Ragulator complex protein LAMTOR1, L-selectin, Mitogen-activated protein kinase 3, Mucin-5B, Nuclear factor of activated T-cells, Transforming growth factor beta-1 proprotein, Serine/threonine-protein kinase VRK1, Cyclin-dependent kinase inhibitor 3, Tissue factor pathway inhibitor 2, Microtubule-associated proteins 1A/1B light chain 3B, Phosphatidylinositol 3,4,5-trisphosphate 3-phosphatase and dual-specificity protein phosphatase PTEN, Interleukin-8, Rho guanine nucleotide exchange factor 2, CASP8 and FADD-like apoptosis regulator, CUE domain-containing protein 2, Death-associated protein kinase 1, Endothelin-1 receptor, Eukaryotic translation initiation factor 3 subunit B, DNA-binding protein inhibitor ID-2, Prelamin-A/C, CAD protein, Zinc finger protein 593, Mitogen-activated protein kinase 12, Cytochrome P450 1B1, Angiotensinogen, Adenomatous polyposis coli protein, POU domain class 2 transcription factor 1, Somatostatin receptor type 4, Tumor necrosis factor alpha-induced protein 3, and E3 ubiquitin-protein ligase TRIM22 as well as combinations thereof and isoforms, fragments and variants thereof (the list corresponds to SEQ ID No. 1 to 252 as well as CD15 and CD139); and

b. comparing the amount of the biomarker with a reference amount for the biomarker.

The present inventors identified 92 biomarkers (shown in Table 1). 79 thereof have proven to be particularly useful for predicting the risk of occurrence of AKI (shown in Table 2) and 26 thereof have proven to be particularly useful for early diagnosis of AKI (shown in Table 3), respectively. 12 biomarkers have proven to be particularly useful for both predicting the risk of occurrence of AKI and for early diagnosis of AKI (shown in Table 4). 35 biomarkers have shown a higher abundance in connection with AKI (shown in Table 5) and 68 biomarkers have shown lower abundance in connection with AKI (shown in Table 6).

In an embodiment, the method includes determining the amount of biomarkers that are downregulated in subjects having AKI or being at risk of developing AKI. In an embodiment, the biomarkers have a logFC of less than -0.7. In an embodiment, the method includes determining the amount of biomarkers that are upregulated in subjects having AKI or being at risk of developing AKI. In an embodiment, the biomarkers have a logFC of at least 0.7.

"logFC" is defined as the log fold change calculated for the basis 2 and represents the differences in protein abundance/amount between AKI patients and patients without AKI. The amount may be determined using an antibody microarray as disclosed herein, such as disclosed in the example section. A logFC = 1 means that AKI patients have on average a $2^1 = 2$ fold higher abundance of biomarker as compared to patients without AKI. logFC = -1 stands for $2^{-1}$ = 1/2 of the abundance of biomarker in AKI patients compared to patients without AKI.

All of the methods disclosed herein may include the step of predicting the risk of the subject of developing AKI based on a comparison of the amount of the biomarker with a reference amount of the biomarker. For biomarkers that are upregulated in subjects with AKI, an increased amount of the biomarker compared with the reference amount may indicate that the subject has AKI. For biomarkers that are upregulated in subjects with a risk of developing AKI, an increased amount of the biomarker compared with the reference amount may indicate that the subject is at risk of developing AKI. For biomarkers that are downregulated in subjects with AKI, a decreased amount of the biomarker compared with the reference amount may indicate that the subject has AKI. For biomarkers that are downregulated in subjects with a risk of developing AKI, a decreased amount of the biomarker compared with the reference amount may indicate that the subject is at risk of developing AKI.

Determining the amount of biomarker in a sample may include biomarker detection. Biomarker detection may include binding the biomarker to a binding agent. The binding agent may be selected from proteins that bind specifically to the respective biomarker. Examples of such proteins include antibodies. Another example is a receptor in case the biomarker is a ligand to the receptor. The binding agent can be immobilized on a substrate. For example, most of the biomarkers of this invention can be detected using immobilized antibodies.

Some of the biomarkers described herein are ligands of the Tumor necrosis factor receptor superfamily member 1B. For these markers, an immobilized Fc-Tumor necrosis factor receptor superfamily member 1B-Fusion protein can be used for capturing ligands of this receptor as an alternative to using antibodies. The ligands include Tumor necrosis factor and Lymphotoxin-alpha (SEQ ID No. 251-252). Examples of such ligands include marker no. 5 in table 1, marker no. 4 in table 2; and/or marker no. 5 in table 6. Evidence for the usefulness of all these biomarkers is given in the example section. Generally, all of these biomarkers are useful for predicting and diagnosing AKI.

In an embodiment, the method includes determining the amount of at least one biomarker that is upregulated in connection with AKI. In this embodiment, the invention relates to a method for predicting the risk of occurrence of acute kidney injury (AKI) or for early diagnosis of AKI in a subject, comprising the steps of:

a. determining in a sample obtained from the subject the amount of at least one biomarker that is upregulated in connection with AKI selected from the group consisting of

Interferon alpha-1/13, Cellular tumor antigen p53, Caspase-9, Interleukin 7, Interleukin-18, Serum amyloid P-component, Urokinase-type plasminogen activator, Keratin type II cytoskeletal 8, Eotaxin, Max dimerization protein 4, Interleukin-15, CTP synthase 1, Cytokine receptor-like factor 2, RNA-binding protein 3, Transmembrane protein 54, C-C motif chemokine 7, Homeobox protein Hox-C11, Ragulator complex protein LAMTOR1, Transforming growth factor beta-1 proprotein, High mobility group protein B2, Intercellular adhesion molecule 1, Complement decay-accelerating factor, Microtubule-associated proteins 1A/1B light chain 3B, Phosphatidylinositol 3,4,5-trisphosphate 3-phosphatase and dual-specificity protein phosphatase PTEN, Caspase-8, Growth arrest-specific protein 6, Interstitial collagenase, Eukaryotic translation initiation factor 3 subunit B, Zinc finger protein 593, Endothelin-1 receptor, CASP8 and FADD-like apoptosis regulator, DNA-binding protein inhibitor ID-2, Mitogen-activated protein kinase 12, POU domain class 2 transcription factor 1, and E3 ubiquitin-protein ligase TRIM22, as well as combinations thereof and isoforms, fragments and variants thereof, and

b. comparing the amount of the biomarker with a reference amount for the biomarker.

These biomarkers have shown higher abundance in connection with AKI. The listed upregulated biomarkers correspond to SEQ ID Nos. 13, 69-77, 80-83, 27-29, 25-26, 122, 127-128, 102-103, 61, 131, 98-99, 129-130, 65-67, 121, 132-134, 18, 145, 153, 184, 144, 146, 137-143, 190, 191-193, 52-60, 93-97, 112, 223-224, 233, 218-222, 198-212, 225, 234-235, 241-246, and 249-250, respectively.

As used in this description, a biomarker that is upregulated or downregulated "in connection with AKI" means that the biomarker is up- or downregulated, respectively, in subjects who are at risk of developing AKI, or who have (e.g. early stage) AKI.

In an embodiment, the method includes determining the amount of at least one biomarker that is downregulated in connection with AKI. In this embodiment, the invention relates to a method for predicting the risk of occurrence of acute kidney injury (AKI) or for early diagnosis of AKI in a subject, comprising the steps of:

a. determining in a sample obtained from the subject the amount of at least one biomarker that is downregulated in connection with AKI selected from the group consisting of

Prostaglandin G/H synthase 2, CD15, CD99 antigen, Tumor necrosis factor receptor superfamily member 6, Ligands of Tumor necrosis factor receptor superfamily member 1B including Tumor necrosis factor and Lymphotoxin-alpha, High affinity immunoglobulin epsilon receptor subunit alpha, CD9 antigen, Dickkopf-related protein 2, C-C motif chemokine 7, DNA topoisomerase 2-alpha, Receptor-type tyrosine-protein phosphatase C, Major prion protein, P-selectin glycoprotein ligand 1, Interleukin-7, Basigin, Hyaluronan mediated motility receptor, Interleukin-18, Tumor necrosis factor ligand superfamily member 14, Serine/threonine-protein kinase PAK 1, Lamin-B1, Monocyte differentiation antigen CD14, Eotaxin, Caspase-8, C-C motif chemokine 3, Brain-derived neurotrophic factor, C-C motif chemokine 5, Cytokine receptor-like factor 2, Leukocyte surface antigen CD47, Leukosialin, Interstitial collagenase, Melanophilin, Myeloblastin, Dickkopf-related protein 3, MAP/microtubule affinity-regulating kinase 4, Transforming growth factor-beta-induced protein ig-h3, Cytokine receptor common subunit beta, Prostaglandin G/H synthase 1, Growth arrest-specific protein 6, RNA-binding protein 3, T-cell surface glycoprotein CD8 alpha chain, Interleukin-15, Tetraspanin-16, Matrilysin, Interferon alpha-1/13, CD139, Nuclear factor of activated T-cells, L-selectin, Actin cytoplasmic 1, Krueppel-like factor 8, Interleukin-12 subunit alpha, Interleukin-8, Mitogen-activated protein kinase 3, Caspase-3, Galectin-4, Mucin-5B, Serine/threonine-protein kinase VRK1, Cyclin-dependent kinase inhibitor 3, Tissue factor pathway inhibitor 2, Rho guanine nucleotide exchange factor 2, Prelamin-A/C, CAD protein, Death-associated protein kinase 1, CUE domain-containing protein 2, Cytochrome P450 1B1, Somatostatin receptor type 4, Angiotensinogen, Adenomatous polyposis coli protein, and Tumor necrosis factor alpha-induced protein 3 as well as combinations thereof and isoforms, fragments and variants thereof, and

b. comparing the amount of the biomarker with a reference amount for the biomarker.

These biomarkers have shown lower abundance in AKI. The listed downregulated biomarkers correspond to SEQ ID Nos. 1,2-4, 6-12, 5, 19, 20, 18, 43-46, 31-38, 30, 39-40, 27-29, 14-17, 21-24, 25-26, 41-42, 78-79, 68, 64, 61, 52-60, 62, 47-51, 63, 65-67, 85-88, 104, 112, 107-111, 120, 92, 105-106, 84, 100-101, 114-119, 93-97, 121, 89-91, 98, 123-126, 113, 13, 160-183, 154-155, 135, 148-151, 147, 194, 156-158, 136, 152, 159, 185, 186, 188-189, 195-197, 226-231, 232, 214-217, 213, 236, 247, 237, 238-240, 248 and 251-252, respectively, as well as CD15 and CD139.

The following methods are preferred methods. The sets of biomarkers used in the preferred methods have shown to be particularly useful for predicting the risk of occurrence of AKI and/or early diagnosis of AKI. In the following passages only step a. is indicated. Preferably, the methods also include the following step subsequent to step a.:

b. comparing the amount of the biomarker with a reference amount for the biomarker.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of CD15 and Interferon alpha-1/13, as well as combinations thereof and isoforms, fragments and variants thereof. These biomarkers are showing an excellent quality score of 6.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of CD9 antigen, Dickkopf-related protein 2 and Hyaluronan mediated motility receptor, as well as combinations thereof and isoforms, fragments and variants thereof. These biomarkers are showing an excellent quality score of 5.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of Cytokine receptor-like factor 2, and Lamin-B1, as well as combinations thereof and isoforms, fragments and variants thereof. These biomarkers are showing a very high quality score of 4.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of Cytokine receptor common subunit beta, Leukosialin, MAP/microtubule affinity-regulating kinase 4, Melanophilin, Myeloblastin, RNA-binding protein 3, Serum amyloid P-component and Tetraspanin-16, as well as combinations thereof and isoforms, fragments and variants thereof. These biomarkers are showing a high quality score of 3.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of Dickkopf-related protein 2 and Interferon alpha-1/13, as well as combinations thereof and isoforms, fragments and variants thereof. These biomarkers are showing an excellent quality score of 5 to 6. They are also secreted biomarkers making them particularly suitable candidates for detection in urine, blood, plasma or serum.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of Dickkopf-related protein 2, Cytokine receptor-like factor 2 and Serum amyloid P-component, as well as combinations thereof and isoforms, fragments and variants thereof. These biomarkers are showing a very high to excellent quality score of 3 to 6. They are also secreted biomarkers making them particularly suitable candidates for detection in urine, blood, plasma or serum.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of CD9 antigen, CD15 and Hyaluronan mediated motility receptor, as well as combinations thereof and isoforms, fragments and variants thereof. These biomarkers are showing an excellent quality score of 5 to 6. They are also membrane biomarkers making them particularly suitable candidates for detection in urine, blood, plasma or serum.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of CD9 antigen, CD15, Hyaluronan mediated motility receptor, Myeloblastin and Tetraspanin-16, as well as combinations thereof and isoforms, fragments and variants thereof. These biomarkers are showing a very high to excellent quality score of 3 to 6. They are also membrane biomarkers making them particularly suitable candidates for detection in urine, blood, plasma or serum.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of CD9 antigen, CD15, Cytokine receptor-like factor 2, Lamin-B1, Melanophilin, Myeloblastin, Serum amyloid P-component, Krueppel-like factor 8, Nuclear factor of activated T-cells, Rho guanine nucleotide exchange factor 2, CASP8 and FADD-like apoptosis regulator, CUE domain-containing protein 2, Death-associated protein kinase 1, Prelamin-A/C, Cytochrome P450 1B1, Adenomatous polyposis coli protein and POU domain, class 2, transcription factor 1, as well as combinations thereof and isoforms, fragments and variants thereof. These biomarkers are showing distinct kidney expression. Distinct kidney expression refers to specific abundance within kidney substructures observed by the inventors. This indicates specific renal functions of the markers making them particularly suitable as biomarkers for kidney injury.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of CD9 antigen, CD15 and Myeloblastin, as well as combinations thereof and isoforms, fragments and variants thereof. These biomarkers are showing a high to excellent quality score of 3 to 6. They are also showing distinct kidney expression. Distinct kidney expression refers to specific abundance within kidney substructures observed by the inventors. This indicates specific renal functions of the markers making them particularly suitable as biomarkers for kidney injury. In addition, these three proteins are expressed by leukocytes; more specifically they are located on the cell membrane or in polymorphonuclear leukocyte granules. This is of special importance as leukocytes are known to be involved in the pathogenesis of acute kidney injury as the immune response to kidney damage during AKI is an important contributor to a prolonged lack of renal function and progression of kidney injury (Kinsey & Okusa, 2012).

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of CD15, Lamin-B1, MAP/microtubule affinity-regulating kinase 4, Dickkopf-related protein 2, Krueppel-like factor 8, Rho guanine nucleotide exchange factor 2, CUE domain-containing protein 2, Death-associated protein kinase 1, DNA-binding protein inhibitor ID-2, Prelamin-A/C, Adenomatous polyposis coli protein, POU domain class 2 transcription factor 1, Somatostatin receptor type 4 and Tumor necrosis factor alpha-induced protein 3, as well as combinations thereof and isoforms, fragments and variants thereof. These biomarkers were detected as differentially abundant in male patients and are particularly useful for predicting and/or diagnosing AKI in male patients.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of CD15, Lamin-B1 and MAP/microtubule affinity-regulating kinase 4, as well as combinations thereof and isoforms, fragments and variants thereof. These biomarkers are showing a high to excellent quality score of 3 to 6. They were also detected as differentially abundant in male patients and are particularly useful for predicting and/or diagnosing AKI in male patients.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of CD9 antigen, Tetraspanin-16, as well as combinations thereof and isoforms, fragments and variants thereof. These biomarkers belong to the tetraspanin family of integral membrane proteins. They interact with a variety of proteins and other tetraspanins and are required for the normal development and function of several organs including the eye, kidney and the immune system (Charrin et al., 2014).

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of Serum amyloid P-component, L-selectin and Galectin-4, as well as combinations thereof and isoforms, fragments and variants thereof. These biomarkers belong to the Lectin family. Lectins are specific carbohydrate binding proteins and one of their major functions is to facilitate cell-cell contacts. Selectins, for instance, are involved in leukocyte adhesion and signaling at the vascular wall being crucial steps during inflammation and immune response (McEver, 2015).

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of MAP/microtubule affinity-regulating kinase 4, Microtubule-associated proteins 1A/1B light chain 3B, Rho guanine nucleotide exchange factor 2, Adenomatous polyposis coli protein, as well as combinations thereof and isoforms, fragments and variants thereof. These biomarkers belong to the microtubuli binding proteins. In kidney tubular epithelial cells, the microtubule cytoskeleton plays a crucial role in the maintenance of cell polarity thereby influencing renal function (Drubin & Nelson, 1996).

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of MAP/microtubule affinity-regulating kinase 4, Mitogen-activated protein kinase 3, Serine/threonine-protein kinase VRK1, Death-associated protein kinase 1, Mitogen-activated protein kinase 12, as well as combinations thereof and isoforms, fragments and variants thereof. These biomarkers are showing protein serine/threonine kinase activity. Serine/Threonine kinases are key mediators in various signaling pathways including regulation of renal tubular transport (Satoh et al., 2015).

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of Cyclin-dependent kinase inhibitor 3, Phosphatidylinositol 3,4,5-trisphosphate 3-phosphatase and dual-specificity protein phosphatase PTEN, as well as combinations thereof and isoforms, fragments and variants thereof. These biomarkers are showing protein-tyrosine phosphatases activity. Protein tyrosine phosphorylation and dephosphorylation signaling is crucial in podocyte function and repair (Nezvitsky et al., 2014; Hsu et al., 2017).

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of CTP synthase 1 and CAD protein, as well as combinations thereof and isoforms, fragments and variants thereof. These biomarkers are involved in pyrimidine biosynthesis. Pyrimidines are fundamental for DNA replication, gene transcription, protein synthesis, and cellular metabolism.

In an aspect, the invention relates to a method for predicting the risk of occurrence of acute kidney injury (AKI) or for early diagnosis of AKI in a subject, comprising the step of:

a. determining in a sample obtained from the subject the amount of at least one biomarker selected from the group consisting of Cytokine receptor common subunit beta, Interferon alpha-1/13 and Cytokine receptor-like factor 2, as well as combinations thereof and isoforms, fragments and variants thereof. These biomarkers are proteins of the Jak-STAT signaling pathway. The Janus kinase/signal transducers and activators of transcription (JAK/STAT) pathway, in particular STAT1 and STAT3, was shown to be activated in renal and non-renal cells of kidney diseases, such as renal fibrosis and diabetic nephropathy (Chuang & He, 2010; Pang et al., 2010).

In an embodiment of the diagnostic method, further biomarkers are determined in the sample in addition to the biomarkers listed herein. The further biomarkers may be one, two, three or more biomarkers selected from the protein biomarkers, male patient biomarkers, predictive biomarkers, diagnostic biomarkers, or combined predictive and diagnostic biomarkers.

**Predictive method**

The following methods are preferred predictive methods, i.e. methods used to determine the risk that the subject will develop AKI. The sets of biomarkers used in the preferred methods have shown to be particularly useful for predicting the risk of occurrence of AKI. In the following passages only step a. is indicated. Preferably, the methods also include the following step subsequent to step a.:
b. comparing the amount of the biomarker with a reference amount for the biomarker.
In an aspect, the invention relates to a method for predicting the risk of occurrence of acute kidney injury (AKI) in a subject, comprising the step of:

a. determining in a sample obtained from the subject the amount of at least one biomarker that is upregulated in connection with imminent AKI, wherein the upregulated predictive biomarker is selected from the group consisting of

Cellular tumor antigen p53, Serum amyloid P-component, Urokinase-type plasminogen activator, Keratin type II cytoskeletal 8, Homeobox protein Hox-C11, Ragulator complex protein LAMTOR1, Transforming growth factor beta-1 proprotein, High mobility group protein B2, Intercellular adhesion molecule 1, Complement decay-accelerating factor, Eukaryotic translation initiation factor 3 subunit B, Zinc finger protein 593, Endothelin-1 receptor, CASP8 and FADD-like apoptosis regulator, DNA-binding protein inhibitor ID-2, and POU domain class 2 transcription factor 1, as well as combinations thereof and isoforms, fragments and variants thereof.
The listed upregulated predictive biomarkers have proven to be particularly useful for predicting the risk of occurrence of AKI. These biomarkers correspond to SEQ ID Nos. 69-77, 122, 127-128, 102-103, 145, 153, 184, 144, 146, 137-143, 223-224, 233, 218-222, 198-212, 225, and 241-246, respectively.
In an aspect, the invention relates to a method for predicting the risk of occurrence of acute kidney injury (AKI) in a subject, comprising the step of:

a. determining in a sample obtained from the subject the amount of at least one biomarker that is downregulated in connection with imminent AKI, wherein the downregulated predictive biomarker is selected from the group consisting of

CD15, CD99 antigen, Tumor necrosis factor receptor superfamily member 6, Ligands of Tumor necrosis factor receptor superfamily member 1B including Tumor necrosis factor and Lymphotoxin-alpha, High affinity immunoglobulin epsilon receptor subunit alpha, CD9 antigen, Dickkopf-related protein 2, C-C motif chemokine 7, DNA topoisomerase 2-alpha, Receptor-type tyrosine-protein phosphatase C, Major prion protein, P-selectin glycoprotein ligand 1, Interleukin-7, Basigin, Hyaluronan mediated motility receptor, Interleukin-18, Tumor necrosis factor ligand superfamily member 14, Serine/threonine-protein kinase PAK 1, Lamin-B1, Monocyte differentiation antigen CD14, Eotaxin, Caspase-8, C-C motif chemokine 3, Brain-derived neurotrophic factor, C-C motif chemokine 5, Cytokine receptor-like factor 2, Leukocyte surface antigen CD47, Leukosialin, Interstitial collagenase, Melanophilin, Myeloblastin, Dickkopf-related protein 3, MAP/microtubule affinity-regulating kinase 4, Transforming growth factor-beta-induced, protein ig-h3, Cytokine receptor common subunit beta, Prostaglandin G/H synthase 1, Growth arrest-specific protein 6, RNA-binding protein 3, T-cell surface glycoprotein CD8 alpha chain, Interleukin-15, Tetraspanin-16, Matrilysin, Interferon alpha-1/13, Nuclear factor of activated T-cells, L-selectin, Actin cytoplasmic 1, Krueppel-like factor 8, Interleukin-12 subunit alpha, Interleukin-8, Mitogen-activated protein kinase 3, Caspase-3, Galectin-4, Mucin-5B, Serine/threonine-protein kinase VRK1, Rho guanine nucleotide exchange factor 2, Prelamin-A/C, CAD protein, Death-associated protein kinase 1, CUE domain-containing protein 2, Somatostatin receptor type 4, Angiotensinogen, Adenomatous polyposis coli protein, and Tumor necrosis factor alpha-induced protein 3 as well as combinations thereof and isoforms, fragments and variants thereof.

The listed downregulated predictive biomarkers have proven to be particularly useful for predicting the risk of occurrence of AKI. These biomarkers correspond to SEQ ID Nos. 2-4, 6-12, 5, 19, 20, 18, 43-46, 31-38, 30, 39-40, 27-29, 14-17, 21-24, 25-26, 41-42, 78-79, 68, 64, 61, 52-60, 62, 47-51, 63, 65-67, 85-88, 104, 112, 107-111, 120, 92, 105-106, 84, 100-101, 114-119, 93-97, 121, 89-91, 98, 123-126, 113, 13, 160-183, 154-155, 135, 148-151, 147, 194, 156-158, 136, 152, 159, 185, 195-197, 226-231, 232, 214-217, 213, 247, 237, 238-240, 248 and 251-252 respectively, as well as CD15.

In an aspect, the invention relates to a method for predicting the risk of occurrence of acute kidney injury (AKI) in a subject, comprising the step of:

    a. determining in a sample obtained from the subject the amount of at least one predictive biomarker selected from the group consisting of CD15 and Interferon alpha-1/13, as well as combinations thereof and isoforms, fragments and variants thereof. These biomarkers are showing an excellent quality score of 6.

In an aspect, the invention relates to a method for predicting the risk of occurrence of acute kidney injury (AKI) in a subject, comprising the step of:

    a. determining in a sample obtained from the subject the amount of at least one predictive biomarker selected from the group consisting of CD9 antigen, Dickkopf-related protein 2, Hyaluronan mediated motility receptor, as well as combinations thereof and isoforms, fragments and variants thereof. These biomarkers are showing an excellent quality score of 5.

In an aspect, the invention relates to a method for predicting the risk of occurrence of acute kidney injury (AKI) in a subject, comprising the step of:

    a. determining in a sample obtained from the subject the amount of at least one predictive biomarker selected from the group consisting of Cytokine receptor-like factor 2 and Lamin-B1, as well as combinations thereof and isoforms, fragments and variants thereof. These biomarkers are showing a very high quality score of 4.

In an aspect, the invention relates to a method for predicting the risk of occurrence of acute kidney injury (AKI) in a subject, comprising the step of:

    a. determining in a sample obtained from the subject the amount of at least predictive biomarker selected from the group consisting of Cytokine receptor common subunit beta, Leukosialin, MAP/microtubule affinity-regulating kinase 4, Melanophilin, Myeloblastin, RNA-binding protein 3, Serum amyloid P-component and Tetraspanin-16, as well as combinations thereof and isoforms, fragments and variants thereof. These biomarkers are showing a high quality score of 3.

In an aspect, the invention relates to a method for predicting the risk of occurrence of acute kidney injury (AKI) in a subject, comprising the step of:

    a. determining in a sample obtained from the subject the amount of at least one predictive biomarker selected from the group consisting of Dickkopf-related protein 2 and Interferon alpha-1/13, as well as combinations thereof and isoforms, fragments and variants thereof. These biomarkers are showing an excellent quality score of 5 to 6. They are also secreted biomarkers making them particularly suitable candidates for detection in urine, blood, plasma or serum.

In an aspect, the invention relates to a method for predicting the risk of occurrence of acute kidney injury (AKI) in a subject, comprising the step of:

> a. determining in a sample obtained from the subject the amount of at least one predictive biomarker selected from the group consisting of Dickkopf-related protein 2, Cytokine receptor-like factor 2, and Serum amyloid P-component, as well as combinations thereof and isoforms, fragments and variants thereof. These biomarkers are showing a high to excellent quality score of 3 to 6. They are also secreted biomarkers making them particularly suitable candidates for detection in urine, blood, plasma or serum.

In an aspect, the invention relates to a method for predicting the risk of occurrence of acute kidney injury (AKI) in a subject, comprising the step of:

> a. determining in a sample obtained from the subject the amount of at least one predictive biomarker selected from the group consisting of CD9 antigen, CD15, Hyaluronan mediated motility receptor, as well as combinations thereof and isoforms, fragments and variants thereof. These biomarkers are showing an excellent quality score of 5 to 6. They are also membrane biomarkers making them particularly suitable candidates for detection in urine, blood, plasma or serum.

In an aspect, the invention relates to a method for predicting the risk of occurrence of acute kidney injury (AKI) in a subject, comprising the steps of:

> a. determining in a sample obtained from the subject the amount of at least one predictive biomarker selected from the group consisting of CD9 antigen, CD15, Hyaluronan mediated motility receptor, Myeloblastin, Tetraspanin-16 showing, as well as combinations thereof and isoforms, fragments and variants thereof. These biomarkers are showing a high to excellent quality score of 3 to 6. They are also membrane biomarkers making them particularly suitable candidates for detection in urine, blood, plasma or serum.

In an aspect, the invention relates to a method for predicting the risk of occurrence of acute kidney injury (AKI) in a subject, comprising the step of:

> a. determining in a sample obtained from the subject the amount of at least one predictive biomarker selected from the group consisting of CD9 antigen, CD15, Cytokine receptor-like factor 2, Lamin-B1, Melanophilin, Myeloblastin, Krueppel-like factor 8, Nuclear factor of activated T-cells, Rho guanine nucleotide exchange factor 2, CUE domain-containing protein 2, Death-associated protein kinase 1, Prelamin-A/C, Adenomatous polyposis coli protein, as well as combinations thereof and isoforms, fragments and variants thereof. These biomarkers are showing distinct kidney expression. Distinct kidney expression refers to specific abundance within kidney substructures observed by the inventors. This indicates specific renal functions of the markers making them particularly suitable as biomarkers for kidney injury.

In an aspect, the invention relates to a method for predicting the risk of occurrence of acute kidney injury (AKI) in a subject, comprising the step of:

> a. determining in a sample obtained from the subject the amount of at least one predictive biomarker selected from the group consisting of CD9 antigen, CD15, Hyaluronan mediated motility receptor, as well as combinations thereof and isoforms, fragments and variants thereof. These biomarkers are showing a high to excellent quality score of 3 to 6. They are also showing distinct kidney expression. Distinct kidney expression refers to specific abundance within kidney substructures observed by the inventors. This indicates specific renal functions of the markers making them particularly suitable as biomarkers for kidney injury.

In an aspect, the invention relates to a method for predicting the risk of occurrence of acute kidney injury (AKI) in a subject, comprising the step of:

> a. determining in a sample obtained from the subject the amount of at least one predictive biomarker selected from the group consisting of CD15, Lamin-B1, MAP/microtubule affinity-regulating kinase 4, Dickkopf-related protein 2, Krueppel-like factor 8, Rho guanine nucleotide exchange factor 2, Prelamin-A/C, Death-associated protein kinase 1, CUE domain-containing protein 2, Somatostatin receptor type 4, Adenomatous polyposis coli protein, DNA-binding protein inhibitor ID-2, POU domain class 2 transcription factor 1, Tumor necrosis factor alpha-induced protein 3, as well as combinations thereof and isoforms, fragments and variants thereof. These biomarkers were detected as differentially abundant in male patients and are particularly useful for predicting AKI in male patients. In an aspect, the invention relates to a method for predicting the risk of occurrence of acute kidney injury (AKI) in a subject, comprising the step of:

a. determining in a sample obtained from the subject the amount of at least one biomarker selected from the group consisting of CD15, Lamin-B1, MAP/microtubule affinity-regulating kinase 4, as well as combinations thereof and isoforms, fragments and variants thereof. These biomarkers are showing a high to excellent quality score of 3 to 6. They were also detected as differentially abundant in male patients and are particularly useful for predicting AKI in male patients.

In an aspect, the invention relates to a method for predicting the risk of occurrence of acute kidney injury (AKI) in a subject, comprising the step of:

a. determining in a sample obtained from the subject the amount of at least one predictive biomarker selected from the group consisting of CD9 antigen, Tetraspanin-16, as well as combinations thereof and isoforms, fragments and variants thereof. These biomarkers belong to the tetraspanin family of integral membrane proteins. They interact with a variety of proteins and other tetraspanins and are required for the normal development and function of several organs including the eye, and the immune system (Charrin et al., 2014).

In an aspect, the invention relates to a method for predicting the risk of occurrence of acute kidney injury (AKI) in a subject, comprising the step of:

a. determining in a sample obtained from the subject the amount of at least one predictive biomarker selected from the group consisting of Serum amyloid P-component, L-selectin and Galectin-4, as well as combinations thereof and isoforms, fragments and variants thereof. These biomarkers belong to the Lectin family. Lectins are specific carbohydrate binding proteins and one of their major functions is to facilitate cell-cell contacts. Selectins, for instance, are involved in leukocyte adhesion and signaling at the vascular wall being crucial steps during inflammation and immune response (McEver, 2015).

In an aspect, the invention relates to a method for predicting the risk of occurrence of acute kidney injury (AKI) in a subject, comprising the step of:

a. determining in a sample obtained from the subject the amount of at least one predictive biomarker selected from the group consisting of MAP/microtubule affinity-regulating kinase 4, Rho guanine nucleotide exchange factor 2, Adenomatous polyposis coli protein, as well as combinations thereof and isoforms, fragments and variants thereof. These biomarkers belong to the microtubuli binding proteins. In kidney tubular epithelial cells, the microtubule cytoskeleton plays a crucial role in the maintenance of cell polarity thereby influencing renal function (Drubin & Nelson, 1996).

In an aspect, the invention relates to a method for predicting the risk of occurrence of acute kidney injury (AKI) in a subject, comprising the step of:

a. determining in a sample obtained from the subject the amount of at least one predictive biomarker selected from the group consisting of MAP/microtubule affinity-regulating kinase 4, Mitogen-activated protein kinase 3, Serine/threonine-protein kinase VRK1, Death-associated protein kinase 1, as well as combinations thereof and isoforms, fragments and variants thereof. These biomarkers are showing protein serine/threonine kinase activity. Serine/Threonine kinases are key mediators in various signaling pathways including regulation of renal tubular transport (Satoh et al., 2015).

In an aspect, the invention relates to a method for predicting the risk of occurrence of acute kidney injury (AKI) in a subject, comprising the step of:

a. determining in a sample obtained from the subject the amount of one predictive biomarker selected from the group of CAD protein, as well as isoforms, fragments and variants thereof. These biomarkers are involved in pyrimidine biosynthesis. Pyrimidines are fundamental for DNA replication, gene transcription, protein synthesis, and cellular metabolism.

In an aspect, the invention relates to a method for predicting the risk of occurrence of acute kidney injury (AKI) in a subject, comprising the step of:

a. determining in a sample obtained from the subject the amount of at least one predictive biomarker selected from the group consisting of Cytokine receptor common subunit beta, Interferon alpha-1/13, Cytokine receptor-like factor 2, as well as combinations thereof and isoforms, fragments and variants thereof. These biomarkers are proteins of the Jak-STAT signaling pathway. The Janus kinase/signal transducers and activators of transcription (JAK/STAT) pathway is crucial for the kidney's response to injury and the progression of several renal diseases (Chuang & He, 2010; Pang et al., 2010).

In an embodiment of the predictive method, further biomarkers are determined in the sample in addition to the biomarkers listed herein. The further biomarkers may be one, two, three or more biomarkers selected from the protein biomarkers, male patient biomarkers, predictive biomarkers, diagnostic biomarkers, or combined predictive and diagnostic biomarkers.

**Diagnostic method**

The following methods are preferred diagnostic methods, the methods are used to determine whether a subject has (early stage) AKI. The sets of biomarkers used in the preferred methods have shown to be particularly useful for diagnosing AKI early. In the following passages only step a. is indicated. Preferably, the methods also include the following step subsequent to step a.:
b. comparing the amount of the biomarker with a reference amount for the biomarker.
In an aspect, the invention relates to a method for diagnosing acute kidney injury (AKI) in a subject, comprising the step of:

a. determining in a sample obtained from the subject the amount of at least one biomarker that is upregulated in connection with existing early AKI, wherein the upregulated diagnostic biomarker is selected from the group consisting of

Interferon alpha-1/13, Caspase-9, Interleukin-7, Interleukin-18, Eotaxin, Maxdimerization protein 4, Interleukin-15, CTP synthase 1, Cytokine receptor-like factor 2, RNA-binding protein 3, Transmembrane protein 54, C-C motif chemokine 7, Microtubule-associated proteins 1A/1B light chain 3B, Phosphatidylinositol 3,4,5-trisphosphate 3-phosphatase and dual-specificity protein phosphatase PTEN, Caspase-8, Growth arrest-specific protein 6, Interstitial collagenase, Mitogen-activated protein kinase 12, and E3 ubiquitin-protein ligase TRIM22, as well as combinations thereof and isoforms, fragments and variants thereof.
The listed upregulated diagnostic biomarkers have proven to be particularly useful for early diagnosis of AKI. These biomarkers correspond to SEQ ID Nos. (13, 80-83, 27-29, 25-26, 61, 131, 98-99, 129-130, 65-67, 121, 132-134, 18, 190, 191-193, 52-60, 93-97, 112, 234-235, and 249-250, respectively).
In an aspect, the invention relates to a method for diagnosing acute kidney injury (AKI) in a subject, comprising the step of:

a. determining in a sample obtained from the subject the amount of at least one biomarker that is downregulated in connection with existing early AKI, wherein the downregulated diagnostic biomarker is selected from the group consisting of

Prostaglandin G/H synthase 2, CD139, Cyclin-dependent kinase inhibitor 3, Tissue factor pathway inhibitor 2, Krueppel-like factor 8, and Cytochrome P450 1B1 as well as combinations thereof and isoforms, fragments and variants thereof.
The listed downregulated diagnostic biomarkers have proven to be particularly useful for early diagnosis of AKI. These biomarkers correspond to SEQ ID Nos. 1, 148-151, 186, 188-189, and 236, respectively, as well as CD139.
In an aspect, the invention relates to a method for early diagnosis of AKI in a subject, comprising the step of:

a. determining in a sample obtained from the subject the amount of one diagnostic biomarker selected from the group of Interferon alpha-1/13, isoforms, fragments and/or variants thereof. These biomarkers are showing an excellent quality score of 6.

In an aspect, the invention relates to a method for early diagnosis of AKI in a subject, comprising the steps of:

a. determining in a sample obtained from the subject the amount of one diagnostic biomarker selected from the group of Cytokine receptor-like factor 2, isoforms, fragments and/or variants thereof. These biomarkers are showing a very high quality score of 4.

In an aspect, the invention relates to a method for early diagnosis of AKI in a subject, comprising the step of:

a. determining in a sample obtained from the subject the amount of one diagnostic biomarker selected from the group of RNA-binding protein 3, isoforms, fragments and/or variants thereof. These biomarkers are showing a high quality score of 3.

In an aspect, the invention relates to a method for early diagnosis of AKI in a subject, comprising the step of:

a. determining in a sample obtained from the subject the amount of one diagnostic biomarker selected from the group consisting of Interferon alpha-1/13, as well as combinations thereof and isoforms, fragments and/or variants thereof. These biomarkers are showing an excellent quality score of 5 to 6. They are also secreted biomarkers making them particularly suitable candidates for detection in urine, blood, plasma or serum.

In an aspect, the invention relates to a method for early diagnosis of AKI in a subject, comprising the step of:

a. determining in a sample obtained from the subject the amount of at least one diagnostic biomarker selected from the group consisting of Cytokine receptor-like factor 2, as well as isoforms, fragments and variants thereof. These biomarkers are showing a high to excellent quality score of 5 to 6. They are also secreted biomarkers making them particularly suitable candidates for detection in urine, blood, plasma or serum.

In an aspect, the invention relates to a method for early diagnosis of AKI in a subject, comprising the step of:

a. determining in a sample obtained from the subject the amount of at least one diagnostic biomarker selected from the group consisting of Cytokine receptor-like factor 2, Krueppel-like factor 8, Cytochrome P450 1B1, as well as combinations thereof and isoforms, fragments and variants thereof. These biomarkers are showing distinct kidney expression. Distinct kidney expression refers to specific abundance within kidney substructures observed by the inventors. This indicates specific renal functions of the markers making them particularly suitable as biomarkers for kidney injury.

In an aspect, the invention relates to a method for early diagnosis of AKI in a subject, comprising the step of:

a. determining in a sample obtained from the subject the amount of at least one diagnostic biomarker selected from the group consisting of Krueppel-like factor 8, as well as isoforms, fragments and variants thereof. These biomarkers were detected as differentially abundant in male patients and are particularly useful for diagnosing AKI in male patients.

In an aspect, the invention relates to a method for early diagnosis of AKI in a subject, comprising the step of:

a. determining in a sample obtained from the subject the amount of at least one diagnostic biomarker selected from the group consisting of Microtubule-associated proteins 1A/1B light chain 3B, as well as combinations thereof and fragments and variants thereof. These biomarkers belong to the microtubuli binding proteins. In kidney tubular epithelial cells, the microtubule cytoskeleton plays a crucial role in the maintenance of cell polarity thereby influencing renal function (Drubin & Nelson, 1996).

In an aspect, the invention relates to a method for early diagnosis of AKI in a subject, comprising the step of:

a. determining in a sample obtained from the subject the amount of at least one diagnostic biomarker selected from the group consisting of Mitogen-activated protein kinase 12, as well as fragments and variants thereof. These biomarkers are showing protein serine/threonine kinase activity. Serine/Threonine kinases are key mediators in various signaling pathways including regulation of renal tubular transport (Satoh et al., 2015).

In an aspect, the invention relates to a method for early diagnosis of AKI in a subject, comprising the step of:

a. determining in a sample obtained from the subject the amount of at least one diagnostic biomarker selected from the group consisting of Cyclin-dependent kinase inhibitor 3, Phosphatidylinositol 3,4,5-trisphosphate 3-phosphatase and dual-specificity protein phosphatase PTEN, as well as combinations thereof and isoforms, fragments and variants thereof. These biomarkers are showing protein-tyrosine phosphatases activity. Protein tyrosine phosphorylation and dephosphorylation signaling is crucial in podocyte function and repair (Nezvitsky et al. 2014; Hsu et al. 2017).

In an aspect, the invention relates to a method for early diagnosis of AKI in a subject, comprising the step of:

a. determining in a sample obtained from the subject the amount of at least one diagnostic biomarker selected from the group consisting of CTP synthase 1, as well as fragments and variants thereof. These biomarkers are involved in pyrimidine biosynthesis. Pyrimidines are fundamental for DNA replication, gene transcription, protein synthesis, and cellular metabolism.

In an aspect, the invention relates to a method for early diagnosis of AKI in a subject, comprising the step of:

a. determining in a sample obtained from the subject the amount of at least one diagnostic biomarker selected from the group consisting of Cytokine receptor common subunit beta, Interferon alpha-1/13, Cytokine receptor-like factor 2, as well as combinations thereof and isoforms, fragments and variants thereof. These biomarkers are proteins of the Jak-STAT signaling pathway. The Janus kinase/signal transducers and activators of transcription (JAK/STAT) pathway is crucial for the kidney's response to injury and the progression of several renal diseases (Chuang & He, 2010; Pang et al., 2010)

In an embodiment of the diagnostic method, further biomarkers are determined in the sample in addition to the biomarkers listed herein. The further biomarkers may be one, two, three or more biomarkers selected from the protein biomarkers, male patient biomarkers, predictive biomarkers, diagnostic biomarkers, or combined predictive and diagnostic biomarkers.

**Preferred groups of biomarkers**

The biomarkers as used herein include a polypeptide according to SEQ ID No. 1 to 252 or fragments or variants of such polypeptides being associated with the risk of occurrence or to the occurrence of AKI. "Polypeptide" and "protein" are used interchangeably herein. In the tables, all protein biomarkers are uniquely described by the Uniprot entry name, the Uniprot accession number as well as the respective gene name and official protein name as provided by the Uniprot database. For more information on the protein, see the UniProt Database, in particular, the UniProt release 2019_02 of February 13, 2019, see also The UniProt Consortium (2017). The sequences of all protein biomarkers of the invention are listed in the sequence listing under SEQ ID No. 1 to 252.

Variants and/or isoforms of the biomarkers disclosed herein include polypeptides which differ in their amino acid sequences, e.g. due to the presence of conservative amino acid substitutions. Preferably, such variants and/or isoforms have an amino acid sequence being at least 70%, at least 80%, at least 90%, at least 95%, at least 98%, or at least 99% identical over the entire sequence region to the amino acid sequences of the aforementioned specific polypeptides given in the sequence listing. Variants may be allelic variants, splice variants or any other species specific homologs, paralogs, or orthologs. Preferably, the percent identity can be determined by the algorithms of Needleman and Wunsch or Smith and Waterman. Programs and algorithms to carry out sequence alignments are well known by a skilled artisan. To carry out the sequence alignments, the program PileUp (Feng & Doolittle, 1987; Higgins & Sharp, 1989) or the programs Gap and BestFit (Needleman & Wunsch, 1970; Smith & Waterman, 1981), which are part of the GCG software packet (Genetics Computer Group, 575 Science Drive, Madison, Wisconsin, USA 53711, Version 1991), may be used. The sequence identity values recited above in percent (%) may be determined using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments. In an embodiment, the variants of biomarkers include any isoforms of the respective biomarker.

The biomarkers of the present invention include protein biomarkers and non-protein biomarkers. Thus, not all of the 92 biomarkers of the present invention are proteins. The invention also includes non-protein biomarkers, namely CD15 and CD139.

In an aspect, the invention relates to a method for early diagnosis of AKI in a subject, comprising the step of:

a. determining in a sample obtained from the subject the amount of at least one protein biomarker, male patient biomarker, predictive biomarker, diagnostic biomarker, or combined predictive and diagnostic biomarker, as well as combinations thereof and fragments and variants thereof, and

b. comparing the amount of the biomarker with a reference amount for the biomarker.

The "protein biomarkers" of the present invention are CD9 antigen, Prostaglandin G/H synthase 2, CD99 antigen, High affinity immunoglobulin epsilon receptor subunit alpha, ligands of Tumor necrosis factor receptor superfamily member 1B including Tumor necrosis factor and Lymphotoxin-alpha, Tumor necrosis factor receptor superfamily member 6, Interferon alpha-1/13, Basigin, C-C motif, chemokine 7, Dickkopf-related protein 2, Hyaluronan mediated motility receptor, Interleukin-18, Interleukin-7, Major prion protein, Receptor-type tyrosine-protein phosphatase C, P-selectin glycoprotein ligand 1, Tumor necrosis factor ligand superfamily member 14, DNA topoisomerase 2-alpha, Brain-derived neurotrophic factor, Caspase-8, Eotaxin, C-C motif chemokine 3, C-C motif chemokine 5, Monocyte differentiation antigen CD14, Cytokine receptor-like factor 2, Lamin-B1, Cellular tumor antigen p53, Serine/threonine-protein kinase PAK 1, Caspase-9, Transforming growth factor-beta-induced protein ig-h3, Leukocyte surface antigen CD47, T-cell surface glycoprotein CD8 alpha chain, Dickkopf-related protein 3, Growth arrest-specific protein 6, Interleukin-15, Cytokine receptor common subunit beta, Keratin, type II cytoskeletal 8, Leukosialin, MAP/microtubule affinity-regulating kinase 4, Melanophilin, Interstitial collagenase, Matrilysin, Prostaglandin G/H synthase 1, Myeloblastin, RNA-binding protein 3, Serum amyloid P-component, Tetraspanin-16, Urokinase-type plasminogen activator, CTP synthase 1, Max dimerization protein 4, Transmembrane protein 54, Actin, cytoplasmic 1, Caspase-3, Complement decay-accelerating factor, High mobility group protein B2, Homeobox protein, Hox-C11, Intercellular adhesion molecule 1, Interleukin-12 subunit alpha, Krueppel-like factor 8, Galectin-4, Ragulator complex protein LAMTOR1, L-selectin, Mitogen-activated protein kinase 3, Mucin-5B, Nuclear factor of activated T-cells, Transforming growth factor beta-1 proprotein, Serine/threonine-protein kinase VRK1, Cyclin-dependent kinase inhibitor 3, Tissue factor pathway inhibitor 2, Microtubule-associated proteins 1A/1B light chain 3B, Phosphatidylinositol 3,4,5-trisphosphate 3-phosphatase and dual-specificity protein phosphatase PTEN, Interleukin-8, Rho guanine nucleotide exchange factor 2, CASP8 and FADD-like apoptosis regulator, CUE domain-containing protein 2, Death-associated protein kinase 1, Endothelin-1 receptor, Eukaryotic translation initiation factor 3 subunit B, DNA-binding protein inhibitor ID-2, Prelamin-A/C, CAD protein, Zinc finger protein 593, Mitogen-activated protein kinase 12, Cytochrome P450 1B1, Angiotensinogen, Adenomatous polyposis coli protein, POU domain class 2 transcription factor 1, Somatostatin receptor type 4, Tumor necrosis factor alpha-induced protein 3, and E3 ubiquitin-protein ligase TRIM22 as well as isoforms thereof.

"Male patient biomarkers" of the present invention are CD15, ligands of Tumor necrosis factor receptor superfamily member 1B including Tumor necrosis factor and Lymphotoxin-alpha, Lamin-B1, MAP/microtubule affinity-regulating kinase 4, Dickkopf-related protein 2, Krueppel-like factor 8, Rho guanine nucleotide exchange factor 2, CUE domain-containing protein 2, Death-associated protein kinase 1, DNA-binding protein inhibitor ID-2, Prelamin-A/C, Adenomatous polyposis coli protein, POU domain class 2 transcription factor 1, Somatostatin receptor type 4, and Tumor necrosis factor alpha-induced protein 3 as well as isoforms thereof. The male patient biomarkers are particularly useful for predicting the risk of occurrence of AKI and for early diagnosis of AKI, respectively, in male subjects. Thus, in an embodiment of this invention the method includes determining an amount of at least one biomarker selected from SEQ ID No. 68, 105,106, 20, 148,149,150,151, 195, 196, 197, 213, 214, 215, 216, 217, 225, 226, 227, 228, 229, 230, 231, 238, 239, 240, 241, 242, 243, 244, 245, 246, 247, 248 as well as CD15, or fragments or variants thereof, in a sample from a male subject, and comparing the amount of said at least one biomarker with a reference amount for said at least one biomarker. The reference amount should preferably be determined in male subjects.

"Predictive biomarkers" of this invention are CD15, CD99 antigen, High affinity immunoglobulin epsilon receptor subunit alpha, Ligands of Tumor necrosis factor receptor superfamily member 1B including Tumor necrosis factor and Lymphotoxin-alpha, Tumor necrosis factor receptor superfamily member 6, Basigin, C-C motif chemokine 7, CD9 antigen, Dickkopf-related protein 2, Hyaluronan mediated motility receptor, Interleukin-18, Interleukin-7, Major prion protein, Receptor-type tyrosine-protein phosphatase C, P-selectin glycoprotein ligand 1, Tumor necrosis factor ligand superfamily member 14, DNA topoisomerase 2-alpha, Brain-derived neurotrophic factor, Caspase-8, Eotaxin, C-C motif chemokine 3, C-C motif chemokine 5, Monocyte differentiation antigen CD14, Cytokine receptor-like factor 2, Lamin-B1, Cellular tumor antigen p53, Serine/threonine-protein kinase PAK 1, Transforming growth factor-beta-induced protein ig-h3, Leukocyte surface antigen CD47, T-cell surface glycoprotein CD8 alpha chain, Dickkopf-related protein 3, Growth arrest-specific protein 6, Interferon alpha-1/13, Interleukin-15, Cytokine receptor common subunit beta, Keratin type II cytoskeletal 8, Leukosialin, MAP/microtubule affinity-regulating kinase 4, Melanophilin, Interstitial collagenase, Matrilysin, Prostaglandin G/H synthase 1, Myeloblastin, RNA-binding protein 3, Serum amyloid P-component, Tetraspanin-16, Urokinase-type plasminogen activator, Actin cytoplasmic 1, Caspase-3, Complement decay-accelerating factor, High mobility group protein B2, Homeobox protein Hox-C11, Intercellular adhesion molecule 1, Interleukin-12 subunit alpha, Interleukin-8, Krueppel-like factor 8, Galectin-4, Ragulator complex protein, LAMTOR1, L-selectin, Mitogen-activated protein kinase 3, Mucin-5B, Nuclear factor of activated T-cells cytoplasmic 4, Transforming growth factor beta-1 proprotein, Serine/threonine-protein kinase VRK1, Rho guanine nucleotide exchange factor, CASP8 and FADD-like apoptosis regulator, CUE domain-containing protein 2, Death-associated protein kinase 1, Endothelin-1 receptor, Eukaryotic translation initiation factor 3 subunit, DNA-binding protein inhibitor ID-2, Prelamin-A/C, CAD protein, Zinc finger protein 593, Angiotensinogen, Adenomatous polyposis coli protein, POU domain, class 2, transcription factor 1, Somatostatin receptor type 4, and Tumor necrosis factor alpha-induced protein 3 as well as isoforms thereof (corresponding to SEQ ID No.

2-79, 84-97, 98-128, 135-185, 194-233, 237-248, 251-252 as well as CD15). It was found that these biomarkers are particularly useful for predicting AKI in a subject. The sample may be taken prior to a planned surgical intervention. Even more preferred, the predictive biomarker is selected from the group consisting of CD9 antigen, CD15, Myeloblastin, or fragments or variants thereof. More preferably, the biomarker is CD9 antigen having a sequence of SEQ ID No.19.

"Diagnostic biomarkers" of this invention are Cytokine receptor-like factor 2, Prostaglandin G/H synthase 2, Interferon alpha-1/13, Caspase-9, Interleukin-18, Interleukin-7, CTP synthase 1, C-C motif chemokine 7, CD139, Eotaxin, Max dimerization protein 4, Interleukin-15, RNA-binding protein 3, Transmembrane protein 54, Krueppel-like factor 8, Interstitial collagenase, Cyclin-dependent kinase inhibitor 3, Tissue factor pathway inhibitor 2, Growth arrest-specific protein 6, Microtubule-associated proteins 1A/1B light chain 3B, Caspase-8, Phosphatidylinositol 3,4,5-trisphosphate 3-phosphatase and dual-specificity protein phosphatase PTEN, Mitogen-activated protein kinase 12, Cytochrome P450 1B1, and E3 ubiquitin-protein ligase TRIM22 as well as isoforms thereof (corresponding to SEQ ID No.1, 13, 80-83, 25-29, 129, 130, 18, 61-67, 131, 98, 99, 121, 132-134, 148-15, 112, 186-189, 93-97, 190, 52-60, 191-193, 234-250, as well as CD139). It was found that these biomarkers are particularly useful for early diagnosis of AKI in a subject. The sample may be taken within 24 hours, 36 hours or 48 hours after surgical intervention.

"Combined predictive and diagnostic biomarkers" of this invention are Cytokine receptor-like factor 2, Caspase-8 , Eotaxin, C-C motif chemokine 7, Growth arrest-specific protein 6, Interferon alpha-1/13, Interleukin-15, Interleukin-18, Interleukin-7, Krueppel-like factor 8, Interstitial collagenase, and RNA-binding protein 3 as well as isoforms thereof (corresponding to SEQ ID No. 52-61, 18, 65-67, 93-97, 13, 98, 99, 25-29, 148-151, 112, 121). It was found that these biomarkers are particularly useful for both predicting the risk of occurrence of AKI in a subject and early diagnosis of AKI.

Furthermore, in the context of the present invention, it is particularly envisaged to determine the amount of more than one biomarker, e.g., for predicting the risk of occurrence of AKI or for early diagnosis of AKI. The combined determination of biomarkers is advantageous since it allows for a higher specificity and sensitivity, e.g., when predicting the risk of occurrence of AKI or when diagnosing AKI timely.

The following combinations of biomarkers are particularly preferred in accordance with the methods, kits, devices, and uses of the present invention. Hence, in preferred embodiments, the invention includes determining in a sample obtained from the subject at least the amount of (optionally including isoforms thereof):

A) CD9 and CD15,

B) CD9 antigen, CD15 and Myeloblastin,

C) CD9 antigen, CD15 and Dickkopf-related protein 2,

D) CD15 and Dickkopf-related protein 2,

E) CD15 and Interstitial collagenase,

F) CD15 and Cytokine receptor-like factor 2,

G) CD15, Dickkopf-related protein 2 and CD99 antigen,

H) CD15, Dickkopf-related protein 2 and Dickkopf-related protein 3,

I) Cytokine receptor-like factor 2 and Dickkopf-related protein 2,

J) Dickkopf-related protein 2 and Interstitial collagenase,

K) CD15, Dickkopf-related protein 2 and Cytokine receptor-like factor 2, or

L) CD99 antigen, CD15, Myeloblastin, Dickkopf-related protein 2 and Cytokine receptor-like factor 2.

A combination of the aforementioned markers will increase sensitivity and specificity of the method.
In an embodiment, further biomarkers are determined in the sample in addition to the combinations given under A) to L) above. The further biomarkers may be one or more biomarkers selected from the protein biomarkers, male patient biomarkers, predictive biomarkers, diagnostic biomarkers, or combined predictive and diagnostic biomarkers.

## Method steps

The method of the present invention may essentially consist of the aforementioned steps

a. determining in a sample obtained from the subject the amount of at least one of the biomarkers of this invention, as well as combinations thereof and fragments, isoforms and variants thereof, and

b. comparing the amount of the biomarker with a reference amount for the biomarker.

The method may include further steps. In a preferred embodiment, the method is carried out ex vivo, i.e. not practiced on the human or animal body. The method can be assisted by automation.

In accordance with the method of the present invention, the risk of occurrence of AKI is predicted or AKI is diagnosed early in a subject. Preferably, AKI is predicted or diagnosed in the context of a medical intervention, which means that the method is carried out in the peri-operative setting. In the case of a surgical intervention, the "peri-operative setting" is the period between the admission of a subject at the hospital or ICU and the complete regeneration of the subject upon surgery, e.g. the subject's release from the hospital. Using the method of this invention, it can be predicted whether AKI will occur within 1 day, 2 days, 3 days, 4 days, 5 days, 6 days, 1 week, 10 days or 2 weeks or any intermittent time range after medical intervention.

The term "medical intervention" includes "surgical interventions" and other interventions. The other interventions may include the administration of drugs, including contrast agents or kidney stabilizing agents. Other interventions may also include avoiding administration of drugs including nephrotoxic substances.

## Definitions

The term 'solid organ' as used herein refers to an inner organ of the body, which has a firm tissue consistency and is neither hollow nor liquid. Heart, kidney, liver, lung, ovaries, spleen and pancreas are solid organs per definition.

The term 'solid organ transplantation' as used herein refers to a surgical intervention during which a solid organ is removed from the body of a subject to be placed into the body of a recipient subject in order to replace a missing or a damaged organ.

The term 'hematopoietic cell' as used herein refers to blood cells. Hematopoietic cells encompass hematopoietic stem cells, progenitor cells and different blood cell types.

The term 'hematopoeitic cell transplantation' as used herein refers to a surgical intervention during which hematopoietic cells usually derived from bone marrow, peripheral blood or umbilical cord blood are transplanted.

"Predicting the risk of occurrence of AKI" may include assessing the probability prior to a medical intervention according to which AKI will occur in a subject within the period after the medical intervention. More preferably, the risk/probability of occurrence of AKI within up to two weeks after completion of the medical intervention is predicted ("predictive window"). In a preferred embodiment, the predictive window is an interval of at least 1 day, at least 2 days, at least 3 days, at least 4 days, at least 5 days, at least 6 days, at least 1 week, at least 10 days, or at least 2 weeks, or any intermittent time range. In a particular preferred embodiment of the present invention, the predictive window, preferably, is an interval of up to 10 days, or more preferably, of up to 4 weeks. The predictive window may range until about 2 years after completion of the medical intervention, or until further medical intervention. Preferably, said predictive window is calculated from the completion of the medical intervention.

The term 'completion of the medical intervention' as used herein refers to a time point where the medical intervention is finished, e.g. release of the subject from the hospital. The medical intervention does not end with a release of the subject from the hospital in case medical treatment of the subject is needed beyond the time of release from the hospital. Alternatively, said predictive window is calculated from the time point at which the sample to be tested has been obtained.

As will be understood by those skilled in the art, such a prediction cannot be correct for 100% of the subjects. The term, however, requires that prediction can be made for a statistically significant portion of subjects in a proper and correct manner. Whether a portion is statistically significant can be determined by the person skilled in the art using various well-known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98%, or at least 99%. The p-values are, preferably, less than 0.1, less than 0.05, less than 0.01, less than 0.005, or less than 0.0001. Preferably, the probability envisaged by the present invention allows that the prediction of an increased, normal or decreased risk will be correct for at least 60%, at least 70%, at least 80%, or at least 90% of the subjects of a given cohort or population. The term, preferably, relates to predicting whether a subject is at elevated risk or reduced risk as compared to the average risk for the occurrence of AKI in a population of subjects.

Predicting the risk of occurrence of AKI as used herein may include that the subject to be analyzed by the method of the present invention is allocated either into the group of subjects being at risk of occurrence of AKI, or into the group of subjects being not at risk of occurrence of AKI. A risk of occurrence of AKI as referred to in accordance with the present invention means that the risk of occurrence of AKI is elevated (within the predictive window). Particularly, said risk may be elevated as compared to the average risk in a cohort of subjects undergoing medical intervention. A subject is considered not at risk of occurrence of AKI, if the risk of occurrence of AKI is reduced (within the predictive window). Particularly, said risk may be reduced as compared to the average risk in a cohort of subjects undergoing a medical intervention. A subject who is at risk of occurrence of AKI may have a risk of occurrence of AKI of 90% or larger, or of 75% or larger, particularly within a predictive window of 4 weeks. A subject who is not at risk of occurrence of AKI may have a risk of occurrence of AKI of lower than 50%, or lower than 10%, particularly within a predictive window of 4 weeks.

In an embodiment, "predicting the risk of occurrence of AKI" includes comparing the amount of at least one upregulated biomarker in a sample from a subject with a reference amount, wherein a subject is considered to be at risk of developing AKI, if the reference amount is exceeded in the sample. In an embodiment, "predicting the risk of occurrence of AKI" includes comparing the amount of at least one downregulated biomarker in a sample from a subject with a reference amount, wherein a subject is considered to be at risk of developing AKI, if the amount in the sample is less than the reference amount.

The term 'early diagnosis' as used herein refers to a timely diagnosis of AKI after intervention. More preferably, AKI should be diagnosed within 24 h after intervention.

The term "subject" as used herein relates to animals, preferably mammals, and, more preferably, humans. In an embodiment, the subjects are male subjects. The subject to be tested may undergo or may have undergone medical intervention. Preferably, the method is applied to a subject known to undergo surgical intervention. In an embodiment, the subject will undergo surgical intervention in the future, or has undergone surgical intervention at the time at which the sample is taken. For example, the subject may have undergone medical intervention 48 h, 24 h or less before the sample is obtained. In another embodiment, the sample is taken from a subject who will undergo medical intervention, e.g. within 48, or 24 hours.

## Preferred methods

In the method according to the present invention, at least one biomarker of the afore-mentioned group of biomarkers, and preferably of the proteins as shown in SEQ ID NOs: 1 to 252, or fragments or variants thereof, is determined. However, more preferably, a group of biomarkers will be determined in order to strengthen specificity and/or sensitivity of the assessment. Such a group, preferably, comprises at least 2, at least 3, at least 4, at least 5, at least 6, at least 7, at least 8, at least 9, at least 10 or up to all of the said biomarkers. In addition to the specific biomarkers recited in the specification, other biomarkers may be, preferably, determined as well in the methods of the present invention.

In a preferred embodiment of the method of the invention, said at least one biomarker is selected from the group of biomarkers of SEQ ID No. 1 to 252 as well as CD15 and CD139, or fragments or variants thereof.

In a preferred embodiment of the method of the invention, said at least one biomarker is selected from the group of biomarkers listed in SEQ ID No. 68, 105,106, 20, 148,149,150,151, 195, 196, 197, 213, 214, 215, 216, 217, 225, 226, 227, 228, 229, 230, 231, 238, 239, 240, 241, 242, 243, 244, 245, 246, 247, 248 as well as CD15, or fragments or variants thereof. A change in such a biomarker in a sample of a male test subject as compared to the reference is indicative for the risk of occurrence of AKI.

In a preferred embodiment of the method of the invention, said at least one biomarker is selected from the group of biomarkers listed in SEQ ID No. 68, 105,106, 20, 148,149,150,151, 195, 196, 197, 213, 214, 215, 216, 217, 225, 226, 227, 228, 229, 230, 231, 238, 239, 240, 241, 242, 243, 244, 245, 246, 247, 248 as well as CD15, or fragments or variants thereof. A change in such a biomarker in a sample of a male test subject as compared to the reference is indicative for the occurrence of AKI at an early time point.

The term 'medical intervention' as used herein refers to a medical action taken to analyze or improve the state of a subject potentially with a medical disorder. In the context of the present invention, an intervention can be the administration of a drug, avoiding a drug, the injection of contrast media or a surgical intervention.

The term 'change of therapy plan' as used herein refers to an adaptation of the patient's care in response to a prognosis or a diagnosis of AKI. Adaptation of the patient's care can consist in renal dialysis, avoiding nephrotoxic compounds, administration of kidney-stabilizing drugs, avoiding allogeneic blood transfusion, optimizing of the surgery strategy, stringent observation of the patient leading to earlier intervention.

The sample may be a sample of a body fluid, to a sample of separated cells, or to a sample from a tissue or an organ. Samples of body fluids can be obtained by well-known techniques and include, preferably, samples of urine or more preferably, samples of blood, plasma, or serum. Tissue or organ samples may be obtained from any tissue. Separated cells may be obtained from the body fluids or the tissues or organs by separating techniques such as centrifugation or cell sorting. Preferably, cell-, tissue- or organ samples are obtained from those cells, tissues or organs that express or produce the biomarkers referred to herein.

For the present invention, it is particularly preferred that the sample is plasma or serum obtained from the patient. More preferably, said sample is blood plasma obtained prior to surgical intervention for predicting the risk of AKI or up to 48 h, or up to 24 h upon medical intervention for early diagnosis of AKI.

The term 'blood product' as used herein refers to any therapeutic substance prepared from human blood. This includes blood components and plasma derivatives. For the present invention, red blood cells and fresh frozen plasma were considered for the analysis.

**Analyzing samples**

In an embodiment, the predictive method is used to determine before a medical intervention whether a subject may undergo medical intervention, and/or whether certain measures have to be taken prior or during the intervention to reduce the risk of AKI. For that purpose, the sample may be taken up to 6 months prior to a planned medical intervention.

The sample to be analyzed in the context of the methods of the present invention may be obtained prior or after a medical intervention, in particular prior or after the surgical intervention as described herein. A sample obtained prior to medical intervention is, preferably, obtained directly prior to the intervention. It is also contemplated to obtain a sample not more than six months, not more than three months, not more than six weeks, not more than two weeks, or not more than one week prior to medical intervention or at any time point prior medical intervention, such as within 2h, 6h, 12h, 24h, 36h, 48h, within 7 days before a medical intervention. A sample obtained after medical intervention preferably, may be obtained within 2h, 6h, 12h, 24h, 36h, 48h or within 7 days after completion of medical intervention.

Determining the amount of the biomarkers referred to in this specification relates to measuring the amount or concentration, preferably semi-quantitatively or quantitatively. Measuring can be done directly or indirectly. Direct measuring relates to measuring the amount or concentration of the biomarker based on a signal that is obtained from the biomarker itself and the intensity of which directly or indirectly correlates with the number of molecules of the polypeptide present in the sample. Such a signal - sometimes referred to herein as intensity signal may be obtained, e.g., by measuring an intensity value of a specific physical or chemical property of the polypeptide. Indirect measuring includes measuring of a signal obtained from a secondary component (i.e. a component not being the biomarker itself) or a biological read out system, e.g., measurable cellular responses, ligands, labels, or enzymatic reaction products.

In accordance with the present invention, determining the amount of a biomarker can be achieved by different means for determining the amount of a biomarker in a sample. Said means comprise immunoassay devices and methods that may utilize labeled molecules in various sandwich, competition, or other assay formats. Preferably, the immunoassay device is an antibody array, in particular a planar antibody microarray or a bead based antibody microarray. Also preferred are stripe tests. Said assays will develop a signal which is indicative for the presence or absence of the biomarker, e.g. a polypeptide biomarker.

Moreover, the signal strength can, preferably, be correlated directly or indirectly (e.g. proportional, or reverse-proportional) to the amount of biomarker present in a sample. Further suitable methods comprise measuring a physical or chemical property specific for the biomarker such as its precise molecular mass or NMR spectrum. Said methods comprise, preferably, biosensors, optical devices coupled to immunoassays, biochips, analytical devices such as mass-spectrometers, NMR- analyzers, or chromatography devices. Further, methods include micro-plate ELISA-based methods, fully-automated or robotic immunoassays, CBA (an enzymatic Cobalt Binding Assay), or latex agglutination assays. The determination of the amount of a biomarker can be performed in a medical laboratory or it can consist of a point-of-care testing.

Also preferably, determining the amount of a biomarker may comprise the step of measuring a specific intensity signal obtainable from the biomarker in the sample. As described above, such a signal may be the signal intensity observed at a mass to charge (m/z) variable specific for the biomarker observed in mass spectra or an NMR spectrum specific for the biomarker.

Determining the amount of a biomarker may, preferably, comprise the steps of

aa) contacting the biomarker with a specific ligand,

ab) optionally, removing non-bound ligand, and

ac) measuring the amount of bound ligand.

The bound ligand will generate an intensity signal. Binding includes both covalent and non-covalent binding. A ligand can be any compound, e.g., a peptide, polypeptide, nucleic acid, or small molecule, binding to the biomarker described herein. Preferred ligands include antibodies, nucleic acids, peptides or polypeptides such as receptors or binding partners for the biomarker and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab, scFv and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. Alternatively, chimeric mouse antibodies with rabbit Fc can be used. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the biomarker.

"Specific binding" according to the present invention means that the ligand or agent should not bind substantially to ("cross-react" with) another biomarker, polypeptide or substance present in the sample to be analyzed. Preferably, the specifically bound biomarker should be bound with at least 3 times higher, more preferably at least 10 times higher and even more preferably at least 50 times higher affinity than any other substance, biomarker or polypeptide in the sample. Non-specific binding may be tolerable, if it can still be distinguished and measured unequivocally, e.g. according to its size on a Western Blot, or by its relatively higher abundance in the sample.

Binding of the ligand can be measured by any method known in the art. Preferably, said method is semi-quantitative or quantitative. Suitable methods are described in the following. First, binding of a ligand may be measured directly, e.g. by mass spectroscopy, NMR or surface plasmon resonance. Second, if the ligand also serves as a substrate of an enzymatic activity of the biomarker of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/biomarker" complex or the ligand that was bound by the biomarker, respectively, may be contacted with a suitable substrate allowing detection by the generation of an intensity signal.

For measurement of enzymatic reaction products, preferably the amount of substrate is saturating. The substrate may also be labeled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand.

Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of a tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.).

The ligand or substrate may also be "tagged" with one or more tags. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT- BCIP (4-nitro blue tetrazolium chloride and 5-bromo-4-chloro-3-indolyl-phosphate, available as ready-made stock solution from Roche Diagnostics), CDP-Star(TM) (Amersham Biosciences), ECF(TM) (Amersham Biosciences). A suitable enzyme-substrate combination may result in a colored reaction product, fluorescence or chemo luminescence, which can be measured according to methods known in the art (e.g. using a light-sensitive film or a suitable camera system). As for measuring the enzymatic reaction, the criteria given above apply analogously.

Suitable fluorescent labels include fluorescent proteins (such as GFP and its derivatives), Cy3, Cy5, or scioDye 1, scioDye 2, scioDye 3, scioDye 4 (Sciomics, Germany) or Texas Red, Fluorescein, and the Alexa dyes (e.g. Alexa 568). Further fluorescent labels are available e.g. from Molecular Probes (Oregon) or Dyomics (Germany). Further, the use of quantum dots as fluorescent labels is contemplated. Suitable radioactive labels include $^{35}S$, $^{125}I$, $^{32}P$, $^{33}P$ and the like. A radioactive label can be detected by any method known and appropriate, e.g. a light-sensitive film or a phosphor imager. Suitable measurement methods include precipitation (particularly immunoprecipitation), electro-chemiluminescence (electro-generated chemiluminescence), RIA (radioimmunoassay), ELISA (enzyme-linked immunosorbent assay), sandwich enzyme immune tests, electro-chemiluminescence sandwich immunoassays (ECLIA), dissociation-enhanced lanthanide fluoro immuno assay (DELFIA), scintillation proximity assay (SPA), FRET based proximity assays (Pulli et al., 2005) or Ligation proximity assays (Fredriksson et al., 2002), turbidimetry, nephelometry, latex-enhanced turbidimetry or nephelometry, or solid phase immune tests. Further methods such as gel electrophoresis, 2D gel electrophoresis, SDS polyacrylamide gel electrophoresis (SDS-PAGE), Western Blotting, and mass spectrometry can be used alone or in combination with labeling or other detection methods as described above.

The amount of a biomarker may be, also preferably, determined as follows:

a1) contacting a solid support comprising a ligand for the biomarker as specified above with a sample comprising the biomarker,

a2) optionally, removing non-bound biomarker, and

a3) measuring the amount of biomarker which is bound to the support.

The ligand, preferably, chosen from the group consisting of nucleic acids, peptides, polypeptides, antibodies and aptamers, is preferably present on a solid support in immobilized form.

Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes, or combinations thereof.

The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention.

Suitable methods for fixing/immobilizing said ligand include, but are not limited to ionic, hydrophobic, covalent interactions and the like. It is also contemplated to use "suspension arrays" as arrays according to the present invention (Nolan & Sklar, 2002). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are disclosed in US 5,744,305, which is incorporated by reference as if fully set forth herein.

The term "amount" as used herein encompasses the absolute amount of a biomarker, the relative amount or concentration of the said biomarker as well as any value or parameter which correlates thereto or can be derived therefrom. Such values or parameters comprise intensity signal values from all specific physical or chemical properties obtained from the said biomarker by direct measurements, e.g., intensity values in mass spectra or NMR spectra or surface Plasmon resonance spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the biomarker comprised in the sample to be analyzed with an amount of a suitable reference source specified elsewhere in this description. It is to be understood that "comparing" as used herein refers to a comparison of corresponding parameters or values, e.g., an absolute amount is compared to an absolute reference amount, while a concentration is compared to a reference concentration, or an intensity signal obtained from a test sample is compared to the same type of intensity signal of a reference sample. Preferably, the reference amount is the amount of the biomarker in healthy subjects, e.g. the average amount of the respective biomarker in a group of 10 or more, 30 or more, 50 or more, or 100 or more healthy subjects.

The comparison of the method of the present invention may be carried out manually or computer assisted. For a computer assisted comparison, the value of the determined amount may be compared to values corresponding to suitable references, which are stored in a database by a computer program. The computer program may further evaluate the result of the comparison, i.e. automatically provide the desired assessment in a suitable output format. Based on the comparison of the amount determined in step a) and the reference amount, it is possible to predict the risk of occurrence of AKI and/or diagnose AKI in a subject after medical intervention.

The term "reference" as used herein refers to amounts of the biomarker which allow for predicting whether a subject is at risk of occurrence of AKI or diagnosing AKI at an early time point. Therefore, the reference may either be derived from

(i) a subject known to be at risk of occurrence of AKI or diagnosed with AKI (or from a group of said subjects) or

(ii) a subject known not to be at risk of occurrence of AKI or not diagnosed with AKI (or from a group of said subjects).

Preferably, said reference is derived from a sample of the aforementioned healthy subjects.

More preferably, a changed amount of the said at least one biomarker selected from the biomarkers according to SEQ ID No. 2-79, 84-97, 98-128, 135-185, 194-233, 237-248, 251-252 as well as CD15 (Table 2) compared to the reference is indicative for a subject being at risk or not being at risk of occurrence of AKI, whereas a changed amount of the said at least one biomarker selected from the biomarkers according to SEQ ID No. 1, 13, 80-83, 25-29, 129, 130, 18, 61-67, 131, 98, 99, 121, 132-134, 148-15, 112, 186-189, 93-97, 190, 52-60, 191-193, 234-250 as well as CD139 (Table 3) compared to the reference is indicative for a subject with AKI or without AKI at an early time point.

Preferably, the changes as referred to herein are statistically significant.

In the context of the methods of the present invention, the amount of more than one biomarker may be determined. Of course, the determined amounts may be compared to various reference amounts, i.e. to the reference amounts for the individual biomarker tested.

Moreover, the references, preferably, define threshold amounts or thresholds. Suitable reference amounts or threshold amounts may be determined by the method of the present invention from a reference sample to be analyzed together, i.e. simultaneously or subsequently, with the test sample. A preferred reference amount serving as a threshold may be derived from the upper limit of normal (ULN), i.e. the upper limit of the physiological amount to be found in a population of subjects (e.g. patients enrolled for a clinical trial). The ULN for a given population of subjects can be determined by well known techniques. A suitable technique may be to determine the median of the population for the biomarker amounts to be determined in the method of the present invention. Suitable threshold amounts can also be identified by ROC plots depicting the overlap between the two distributions by plotting the sensitivity versus 1 - specificity for the complete range of decision thresholds. On the y-axis is sensitivity, or the true-positive fraction, defined as (number of true-positive test results)/(number of true-positive + number of false-negative test results). This has also been referred to as positivity in the presence of a given disease. It is calculated solely from the affected subgroup. On the x-axis is the false-positive fraction, or 1 - specificity, defined as (number of false-positive results)/(number of true-negative + number of false-positive results). It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has a ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45 degrees diagonal line from the lower left corner to the upper right corner. Most plots fall in between these two extremes.

**Diagnosis**

Further preferred are the following diagnostic algorithms:

i) An essentially identical or an increased amount of the at least one biomarker as compared to the reference amount indicates that the subject is at risk of occurrence of AKI or is diagnosed with AKI, if the at least one biomarker is selected from the biomarkers shown in Table 5, and if the reference amount is derived from a subject known to be at risk of occurrence of AKI or diagnosed with AKI, and/or an essentially identical or a decreased amount of the at least one biomarker as compared to the reference amount indicates that the subject is at no risk of occurrence of AKI or diagnosed with no occurrence of AKI, if the at least one biomarker is selected from the biomarkers shown in Table 5, and if the reference amount is derived from a subject known to be not at risk of occurrence of AKI or diagnosed with no occurrence of AKI.

ii) An essentially identical or a decreased amount of the at least one biomarker as compared to the reference amount indicates that the subject is at risk of occurrence of AKI or is diagnosed with AKI, if the at least one biomarker is selected from the biomarkers shown in Table 6, and if the reference amount is derived from a subject known to be at risk of occurrence of AKI or is diagnosed with AKI, and/or an essentially identical or an increased amount of the at least one biomarker as compared to the reference amount indicates that the subject is not at risk of occurrence of AKI or diagnosed with no occurrence of AKI, if the at least one biomarker is selected from the biomarkers shown in Table 6, and if the reference amount is derived from a subject known to be not at risk of occurrence of AKI or diagnosed with no occurrence of AKI.

Advantageously, it has been found in the study underlying the present invention that the biomarkers according to SEQ ID No. 1 to 252 as well as CD15 and CD139 are reliable markers for predicting the occurrence of AKI and/or for early diagnosis of AKI in a subject undergoing medical intervention. Said prediction and early diagnosis is of high importance since AKI, in particular in the peri-operative setting, has a high incidence rate. AKI is associated with a high patient mortality and morbidity, with consequences such as chronic kidney disease (CKD) requiring life-long monitoring and treatment and resulting in high health care costs. The findings underlying the aforementioned method also allow for an improved clinical management of AKI since subjects can be identified which require a change in therapy plan.

Change in therapy

It is to be understood that a subject who is at risk of AKI occurrence or a subject who is diagnosed at an early time point for AKI may require a change in therapy plan compared to a subject who is not at risk of AKI occurrence.

Therefore, the aforementioned method, preferably, further comprises the step of change of therapy plan. A change in therapy plan includes, as discussed above, renal dialysis, avoiding nephrotoxic drugs, administration of kidney-stabilizing drugs, administration of drugs preventing AKI, drugs alleviating or reversing AKI effects or drugs preventing further development of AKI into CKD, avoiding anemia, optimization of the strategy of the surgical intervention, stringent observation of the patient possibly leading to earlier intervention.

**Definition**

The term 'dialysis' as used herein refers to a procedure required when a subject develops end-stage kidney failure, which corresponds to less than 15% of kidney function. Kidney failure can be acute as in AKI or the result of a slowly worsening kidney function as in CKD. Dialysis relies on the principle of osmosis of solutes and ultrafiltration of fluid across a semi-permeable membrane. The main purpose of dialysis is to remove excess water, solutes and toxins from the blood of a subject, thereby partially replacing the functions of a healthy kidney. Dialysis can be performed at an early time point if AKI is diagnosed within 24h upon medical intervention.

The phrase 'avoiding nephrotoxic drugs' as used herein refers to avoiding substances, which impair renal function if the subject is at risk of AKI or if AKI has been already diagnosed. These substances can be chemicals or medications and encompass analgesics, antibiotics, immunosuppressive drugs and contrast media, in particular radiocontrast media for example.

The phrase 'avoiding anemia' as used herein refers to avoiding a decrease in the total amount of red blood cells (RBCs) or hemoglobin in the blood, or a lowered ability of the blood to carry oxygen.

The phrase 'administration of kidney-stabilizing drugs' as used herein refers to the administration of putative drugs which support the physiological function of the kidney if the subject is at risk of AKI or if AKI has been already diagnosed.

The phrase 'administration of drugs preventing AKI' as used herein refers to the administration of putative drugs which prevent the onset of AKI if the subject is at risk of AKI.

The phrase 'drugs alleviating or reversing AKI effects' as used herein refers to the administration of putative drugs which reduce AKI related symptoms and severity of disease.

The phrase 'drugs preventing further development of AKI into CKD' as used herein refers to the administration of putative drugs which prevent the development of AKI into the chronic disease CKD.

The phrase 'optimization of the strategy of the surgical intervention' as used herein refers to the use of surgical methods to shorten the overall duration of the surgical intervention, thereby shortening the time during which a patient is relying on a heart-lung-machine. A heart-lung-machine is a mechanical device composed of a pump, an oxygenator and a heat exchanger to take over the heart and lung function during a surgical intervention.

The phrase 'stringent observation of the patient possibly leading to earlier intervention' as used herein refers to reducing the surveillance interval if the subject is at risk of AKI.

The definitions and explanations given herein above apply mutatis mutandis to the embodiments described herein below (except stated otherwise).

**Change in Therapy (details)**

In an embodiment, the method may include the step of changing the subject's therapy plan, if the method reveals that the subject is suffering from AKI or has a high risk that AKI might occur. It will be understood that a change in therapy plan is at least beneficial for such subject. As discussed above, the method of the present invention already allows identifying subjects at risk of AKI. Accordingly, such subjects may not be unambiguously identifiable based on their clinical symptoms.

Preferred changes in therapy plan are described herein above.

Preferably, the reference is derived from a subject or group of subjects known to be in need of a change in therapy plan, or from a subject or group of subjects known to be not in need of a change in therapy plan.

Preferably, the sample to be tested has been obtained after medical intervention. More preferably, the sample has been obtained after medical intervention, in particular, after surgical intervention. More preferably, the sample has been obtained after surgical intervention, in particular, after solid organ transplantation, cardiac surgery or knee or hip replacement surgery. More preferably, the sample has been obtained after solid organ transplantation, in particular, after lung transplantation, liver transplantation, heart transplantation or kidney transplantation. Even more preferably, the sample has been obtained after lung transplantation, in particular, after lung transplantation in male subjects.

Device for predicting AKI or early diagnosis of AKI

The present invention also relates to a device for predicting occurrence of AKI or for the early diagnosis of AKI in a sample of a subject comprising:

- an analyzing unit for the said sample of the subject comprising a detection agent for at least one biomarker of this invention, or variants or fragments thereof, said detection agent allowing for the determination of the amount of the said at least one biomarker in the sample, and

- an evaluation unit comprising a data processing unit and a data base, said data base comprising a stored reference and said data processing unit being capable of carrying out a comparison of the amount of the at least one biomarker determined by the analyzing unit and the stored reference thereby establishing the prediction.

The biomarker may be selected from any group of biomarkers disclosed herein, such as protein biomarkers, male patient biomarkers, predictive biomarkers, diagnostic biomarkers, or combined predictive and diagnostic biomarkers, as well as combinations thereof and fragments and variants thereof.

The biomarker may be selected from the group consisting of CD9 antigen, Prostaglandin G/H synthase 2, CD15, CD99 antigen, High affinity immunoglobulin epsilon receptor subunit alpha, Ligands of Tumor necrosis factor receptor superfamily member 1B including Tumor necrosis factor and Lymphotoxin-alpha, Tumor necrosis factor receptor superfamily member 6, Interferon alpha-1/13, Basigin, C-C motif, chemokine 7, Dickkopf-related protein 2, Hyaluronan mediated motility receptor, Interleukin-18, Interleukin-7, Major prion protein, Receptor-type tyrosine-protein phosphatase C, P-selectin glycoprotein ligand 1, Tumor necrosis factor ligand superfamily member 14, DNA topoisomerase 2-alpha, Brain-derived neurotrophic factor, Caspase-8, Eotaxin, C-C motif chemokine 3, C-C motif chemokine 5, Monocyte differentiation antigen CD14, Cytokine receptor-like factor 2, Lamin-B1, Cellular tumor antigen p53, Serine/threonine-protein kinase PAK 1, Caspase-9, Transforming growth factor-beta-induced protein ig-h3, Leukocyte surface antigen CD47, T-cell surface glycoprotein CD8 alpha chain, Dickkopf-related protein 3, Growth arrest-specific protein 6, Interleukin-15, Cytokine receptor common subunit beta, Keratin type II cytoskeletal 8, Leukosialin, MAP/microtubule affinity-regulating kinase 4, Melanophilin, Interstitial collagenase, Matrilysin, Prostaglandin G/H synthase 1, Myeloblastin, RNA-binding protein 3, Serum amyloid P-component, Tetraspanin-16, Urokinase-type plasminogen activator, CTP synthase 1, CD139, Max dimerization protein 4, Transmembrane protein 54, Actin cytoplasmic 1, Caspase-3, Complement decay-accelerating factor, High mobility group protein B2, Homeobox protein, Hox-C11, Intercellular adhesion molecule 1, Interleukin-12 subunit alpha, Krueppel-like factor 8, Galectin-4, Ragulator complex protein LAMTOR1, L-selectin, Mitogen-activated protein kinase 3, Mucin-5B, Nuclear factor of activated T-cells, Transforming growth factor beta-1 proprotein, Serine/threonine-protein kinase VRK1, Cyclin-dependent kinase inhibitor 3, Tissue factor pathway inhibitor 2, Microtubule-associated proteins 1A/1B light chain 3B, Phosphatidylinositol 3,4,5-trisphosphate 3-phosphatase and dual-specificity protein phosphatase PTEN, Interleukin-8, Rho guanine nucleotide exchange factor 2, CASP8 and FADD-like apoptosis regulator, CUE domain-containing protein 2, Death-associated protein kinase 1, Endothelin-1 receptor, Eukaryotic translation initiation factor 3 subunit B, DNA-binding protein inhibitor ID-2, Prelamin-A/C, CAD protein, Zinc finger protein 593, Mitogen-activated protein kinase 12, Cytochrome P450 1B1, Angiotensinogen, Adenomatous polyposis coli protein, POU domain class 2 transcription factor 1, Somatostatin receptor type 4, Tumor necrosis factor alpha-induced protein 3, and E3 ubiquitin-protein ligase TRIM22 (SEQ ID No. 1 to 252 as well as CD15 and CD139), and isoforms, fragments and variants thereof.

The term "device" as used herein relates to a system of means comprising at least the afore-mentioned analyzing unit and the evaluation unit operatively linked to each other as to allow the diagnosis. Preferred detection agents to be used for the device of the present invention are disclosed above in connection with the method of the invention. Preferably, detection agents are antibodies or aptamers. How to link the units of the device in an operating manner will depend on the type of units included into the device. For example, where units for automatically determining the amount of the biomarker are applied, the data obtained by said automatically operating unit can be processed by, e.g., a computer program in order to obtain the desired results. Preferably, the units are comprised by a single device in such a case. The computer unit, preferably, comprises a database including the stored reference(s) as well as a computer-implemented algorithm for carrying out a comparison of the determined amounts for the biomarkers with the stored reference of the database. "Computer-implemented" as used herein refers to a computer-readable program code tangibly included into the computer unit. The results may be given as output of raw data which need interpretation by the clinician. Preferably, the output of the device is, however, processed, i.e. evaluated, raw data the interpretation of which does not require a clinician.

In a preferred device of the invention, the detection agent, preferably, an antibody is immobilized on a solid support in an array format. It will be understood that a device according to the present invention can determine the amount of more than one biomarker simultaneously. To this end, the detection agents may be immobilized on a solid support and arranged in an array format, e.g., in a so called "microarray".

Kit

The present invention also relates to a kit comprising a detection agent for determining the amount of at least one biomarker of this invention, or variants or fragments thereof, and evaluation instructions for establishing the prediction or early diagnosis of AKI.

The biomarker may be selected from any group of biomarkers disclosed herein, such as protein biomarkers, male patient biomarkers, predictive biomarkers, diagnostic biomarkers, or combined predictive and diagnostic biomarkers, as well as combinations thereof and fragments and variants thereof.

In an embodiment, the biomarker is selected from the group consisting of CD9 antigen, Prostaglandin G/H synthase 2, CD15, CD99 antigen, High affinity immunoglobulin epsilon receptor subunit alpha, Ligands of Tumor necrosis factor receptor superfamily member 1B including Tumor necrosis factor and Lymphotoxin-alpha, Tumor necrosis factor receptor superfamily member 6, Interferon alpha-1/13, Basigin, C-C motif, chemokine 7, Dickkopf-related protein 2, Hyaluronan mediated motility receptor, Interleukin-18, Interleukin-7, Major prion protein, Receptor-type tyrosine-protein phosphatase C, P-selectin glycoprotein ligand 1, Tumor necrosis factor ligand superfamily member 14, DNA topoisomerase 2-alpha, Brain-derived neurotrophic factor, Caspase-8, Eotaxin, C-C motif chemokine 3, C-C motif chemokine 5, Monocyte differentiation antigen CD14, Cytokine receptor-like factor 2, Lamin-B1, Cellular tumor antigen p53, Serine/threonine-protein kinase PAK 1, Caspase-9, Transforming growth factor-beta-induced protein ig-h3, Leukocyte surface antigen CD47, T-cell surface glycoprotein CD8 alpha chain, Dickkopf-related protein 3, Growth arrest-specific protein 6, Interleukin-15, Cytokine receptor common subunit beta, Keratin type II cytoskeletal 8, Leukosialin, MAP/microtubule affinity-regulating kinase 4, Melanophilin, Interstitial collagenase, Matrilysin, Prostaglandin G/H synthase 1, Myeloblastin, RNA-binding protein 3, Serum amyloid P-component, Tetraspanin-16, Urokinase-type plasminogen activator, CTP synthase 1, CD139, Max dimerization protein 4, Transmembrane protein 54, Actin cytoplasmic 1, Caspase-3, Complement decay-accelerating factor, High mobility group protein B2, Homeobox protein, Hox-C11, Intercellular adhesion molecule 1, Interleukin-12 subunit alpha, Krueppel-like factor 8, Galectin-4, Ragulator complex protein LAMTOR1, L-selectin, Mitogen-activated protein kinase 3, Mucin-5B, Nuclear factor of activated T-cells, Transforming growth factor beta-1 proprotein, Serine/threonine-protein kinase VRK1, Cyclin-dependent kinase inhibitor 3, Tissue factor pathway inhibitor 2, Microtubule-associated proteins 1A/1B light chain 3B, Phosphatidylinositol 3,4,5-trisphosphate 3-phosphatase and dual-specificity protein phosphatase PTEN, Interleukin-8, Rho guanine nucleotide exchange factor 2, CASP8 and FADD-like apoptosis regulator, CUE domain-containing protein 2, Death-associated protein kinase 1, Endothelin-1 receptor, Eukaryotic translation initiation factor 3 subunit B, DNA-binding protein inhibitor ID-2, Prelamin-A/C, CAD protein, Zinc finger protein 593, Mitogen-activated protein kinase 12, Cytochrome P450 1B1, Angiotensinogen, Adenomatous polyposis coli protein, POU domain class 2 transcription factor 1, Somatostatin receptor type 4, Tumor necrosis factor alpha-induced protein 3, E3 ubiquitin-protein ligase TRIM22 (SEQ ID No. 1 to 250 as well as CD15, CD139 and Ligands of Tumor necrosis factor receptor superfamily member 1B including Tumor necrosis factor and Lymphotoxin-alpha), and isoforms fragments and variants thereof.

The term "kit" as used herein refers to a collection of the aforementioned agent and the instructions provided in a ready-to-use manner for determining the biomarker amount in a sample. The agent and the instructions are, preferably, provided in a single container. Preferably, the kit also comprises further components which are necessary for carrying out the determination of the amount of the biomarker. Such components may be auxiliary agents which are required for the detection of the biomarker or calibration standards. Moreover, the kit may, preferably, comprise agents for the detection of more than one biomarker.

Multiple groups have been described above that the biomarker of the present invention may preferably be selected from. These groups have in particular been described with respect to the method of the present invention. However, it is to be understood that these groups are not only relevant with respect to the methods of the invention but also with respect to the kits, devices, and uses of the present invention.

It is particularly envisaged that the detection agents, preferably, an antibody or fragment thereof, comprised by the aforementioned kits or compositions are immobilized on a solid support in an array format. In particular, the detection agents may be immobilized on a solid support and arranged in an array format, e.g., in a so-called "microarray". Accordingly, the present invention also envisaged a microarray comprising the aforementioned detection agents.

Preferably, the kit, the composition and the microarray is used for predicting the risk of occurrence of AKI and diagnosing AKI in a sample of a subject.

All references cited in this specification are herewith incorporated by reference with respect to their entire disclosure content and the disclosure content specifically mentioned in this specification.

Embodiments

The following is a list of embodiments within the scope of the present invention.

The invention includes a method for predicting the risk of occurrence of acute kidney injury (AKI) or for early diagnosis of AKI in a subject, comprising the steps of:

> a. determining in a sample obtained from the subject the amount of at least one biomarker having a logFC of less than -0.7 and being selected from the group consisting of CD15, CD9 antigen and Myeloblastin, as well as isoforms, fragments and variants thereof, and

> b. comparing the amount of said at least one biomarker with a reference amount for said at least one biomarker,

wherein the reference amount is the amount of the respective biomarker in healthy subjects, such as subjects who are not at risk of developing AKI and/or who are not having AKI.

The method includes embodiments, wherein a reduced amount of the at least one biomarker compared to the reference amount indicates that the subject has AKI or is at risk of developing AKI.

The method includes embodiments, comprising the step of predicting whether the subject is at risk of developing AKI and/or has AKI.

The method includes embodiments, including determining in the sample the amount of at least CD15 and Dickkopf-related protein 2, or isoforms, fragments or variants thereof.

The method includes embodiments, including determining in the sample the amount of at least CD9 antigen and Myeloblastin, or isoforms, fragments or variants thereof.

The method includes embodiments, including determining in the sample the amount of at least Dickkopf-related protein 2 and Interferon alpha-1/13, or isoforms, fragments or variants thereof.

The method includes embodiments, including determining in the sample the amount of Dickkopf-related protein 2 and Hyaluronan mediated motility receptor, or isoforms, fragments or variants thereof.

The method includes embodiments, wherein the sample is a urine, blood, plasma or serum sample.

The method includes embodiments, wherein the sample is taken prior to a planned medical intervention such as administration of a drug, avoiding administration of a drug, injection of contrast media or a surgical intervention.

The method includes embodiments, wherein one, two, three or more further biomarkers are determined in the sample, wherein further biomarkers are selected from one or more of the protein biomarkers, male patient biomarkers, predictive biomarkers, diagnostic biomarkers, or combined predictive and diagnostic biomarkers, wherein

The method includes embodiments, wherein the fragments, isoforms and/or variants of the biomarkers have at least 70%, at least 80%, at least 90%, at least 95%, at least 98%, or at least 99% sequence identity with the biomarker over the whole length of the sequence.

The method includes embodiments, wherein determining the amount of said at least one biomarker comprises using an immunoassay device, such as ELISA (enzyme-linked immunosorbent assay) or antibody array, in particular a planar antibody microarray or a bead based antibody microarray.

The invention includes a device for predicting the risk of occurrence of acute kidney injury (AKI) in a subject or for early diagnosis of AKI, comprising

> - an analyzing unit for the said sample of the subject comprising a detection agent for at least one biomarker, or variants or fragments thereof, said detection agent allowing for the determination of the amount of the said at least one biomarker in the sample, and

> - an evaluation unit comprising a data processing unit and a data base, said data base comprising a stored reference and said data processing unit being capable of carrying out a comparison of the amount of the at least one biomarker determined by the analyzing unit and the stored reference thereby establishing the prediction,

> - wherein the biomarker is at least one selected from the group consisting of CD15, CD9 antigen and Myeloblastin as well as isoforms, fragments and variants thereof.

The invention includes a kit comprising a detection agent for determining the amount of at least one biomarker, and evaluation instructions for establishing the prediction or early diagnosis of AKI, wherein the biomarker is at least one selected from the group consisting of CD15, CD9 antigen and Myeloblastin as well as isoforms, fragments and variants thereof.

The invention includes a kit, wherein the detection agent is selected from the group consisting of antibodies and aptamers.

Examples

**Method**

In the studies underlying this invention, plasma samples from subjects before and after LuTx (lung transplantation) were analyzed using antibody microarrays comprising 1130 antibodies against 930 different potential biomarkers. It was assessed whether there are differences between subjects in which AKI was diagnosed using the AKIN classification and subjects in whom AKI was not diagnosed using the AKIN classification. Differences in the marker amounts between subjects that turned out to be statistically significant are those of Table 1 below. These markers may be used as biomarkers for predicting the risk of occurrence of AKI and for early diagnosis of AKI.

In order to identify biomarkers with differential abundance in patients with and without an increased risk of AKI occurrence and between patients with and without AKI a study was performed utilizing complex antibody microarrays. In this study, the protein fraction of the samples was directly labeled by a fluorescent dye. A reference was established by pooling all samples comprised in the study and labeled with a second fluorescent dye. For incubation each sample was mixed with the reference sample and incubated on an antibody microarray in a competitive dual-color approach.

The antibody microarray applied in this study comprised 1130 antibodies that were directed at 900 different proteins. All antibodies were immobilized at least in duplicates. The studies involved a total of 135 samples with or without AKI. The samples were classified as no-AKI (AKIN0) or AKI (AKIN1,2,3).

After concentration measurement from the plasma samples by BCA assay, the samples were labeled at an adjusted protein concentration for one hour with scioDye-1 (Sciomics, Germany). Additionally, a common reference was prepared by pooling of samples and subsequent labeling with scioDye-2 (Sciomics, Germany). After one hour, the reaction was stopped by the addition of hydroxylamine. Excess dye was removed 30 min later and the buffer exchanged to PBS. All labeled protein samples were used immediately.

All samples were incubated on scioDiscover antibody arrays (Sciomics, Heidelberg, Germany). The arrays were blocked with scioBlock (Sciomics, Germany) and afterwards the samples were incubated competitively using a dual-colour approach. After incubation for three hours, the slides were thoroughly washed with 1x PBSTT, rinsed with 0.1x PBS as well as with water and subsequently dried with nitrogen.

Slide scanning was done on a Powerscanner (Tecan, Austria) using the identical instrument laser power and PMT. Spot segmentation was performed with GenePix Pro 6.0 (Molecular Devices, Union City, USA). Resulting data were analyzed using the LIMMA package of R-Bioconductor after uploading the mean signal and median background intensities. For normalization, an invariant Lowess normalization was applied. For differential analyses a linear model was fitted with LIMMA resulting in a two-sided t-test or F-test based on moderated statistics. All presented p-values were adjusted for multiple testing by controlling the false discovery rate according to Benjamini and Hochberg. Proteins were defined as differential for | logFC | > 0.3 and an adjusted p value < 0.05.

Using LIMMA analysis, 92 proteins were identified with differential abundance between AKI and non-AKI samples as differential, as defined above. The results of the aforementioned study are summarized in the following Tables. In the tables the difference of protein abundance in the two sample groups is given by the log fold change (logFC) calculated for the basis 2. The level of significance is indicated by the p-value adjusted for multiple testing as described above.

To assess the quality of the markers a Quality score (QS) was calculated for each marker taking into account logFC values, adjusted p-values as well as stripcharts (graphical representations of the distribution of abundance differences and discrimination power) as follows:

$$QS = QS\,[logFC] + QS\,[adjusted\ p\text{-value}] + QS\,[stripchart]$$

QS [logFC] is defined as "3" for |logFC| > 1.0.

QS [logFC] is defined as "2" for |logFC| > 0.7.

QS [logFC] is defined as "1" for |logFC| > 0.5.

QS [adjusted p-value] is defined as "3" for adjusted p-value < 0.0001.

QS [adjusted p-value] is defined as "2" for adjusted p-value < 0.001.

QS [adjusted p-value] is defined as "1" for adjusted p-value < 0.01.

For QS [stripchart] definition, discrimination power of stripcharts was classified after evaluation by expert scientists within a range of 0.5 to 2.0 as "excellent" = 2.0, "very good" = 1.0, "good" = 0.5.

QS [stripchart] is defined as "1" for discrimination power = excellent or very good.

A maximum Quality score of 7 can be reached by one marker.
A Quality score of > 5 is defined as "excellent",
A Quality score of 4 is defined as "very high",
A Quality score of 3 is defined as "high".
"logFC" is defined as the log fold change calculated for the basis 2 and represents the differences in protein abundance between AKI patients and patients without AKI. In the case of the predictive method, "AKI patient" refers to a subject who developed AKI. A logFC = 1 means that AKI patients have on average a $2^1$ = 2 fold higher signal as compared to patients without AKI. logFC = -1 stands for $2^{-1}$ = 1/2 of the signal in AKI patients compared to patients without AKI.
"adjusted p-values" are p-values adjusted for multiple testing and indicate the level of significance
"strip charts" are graphical representations of differential abundance distribution as well as discrimination power.

**Results**

The following tables identify the biomarkers identified by the inventors.
The present invention comprises in total 92 biomarkers as shown in Table 1. Table 1 comprises both predictive and diagnostic biomarkers.

*Table 1*

| No. | SEQ ID No. | Uniprot entry name | Pre/ diag | Qual score | logFC | adj.p-Val | Uniprot accession | Gene name | Protein name |
|---|---|---|---|---|---|---|---|---|---|
| **1** | 1 | PGH2_HUMAN | diag | 7 | -3.49 | 0.00000080 | P35354 | PTGS2 | Prostaglandin G/H synthase 2 |
| **2** | | CD15 (no protein) | pre | 6 | -0.86 | 0.00000004 | no entry | CD15 | |
| **3** | 2 | CD99_HUMAN | pre | 6 | -0.98 | 0.00000440 | P14209 | CD99 | CD99 antigen |
| | 3 | Isoform II of CD99_HUMAN | | | | | P14209-2 | | |
| | 4 | Isoform 3 of CD99_HUMAN | | | | | P14209-3 | | |
| **4** | 5 | FCERA_HUMAN | pre | 6 | -1.63 | 0.00049891 | P12319 | FCER1A | High affinity immunoglobulin epsilon receptor subunit alpha |
| **5** | 251-252 | No entry (Ligands of TNR1B_ HUMAN including TNFA_ HUMAN and TNFB_ HUMAN) | pre | 6 | -1.60 | 0.00000083 | P01375 and P01374 | TNF and LTA | Ligands of Tumor necrosis factor receptor superfamily member 1B including Tumor necrosis factor and Lymphotoxinalpha |
| **6** | 6 | TNR6_HUMAN | pre | 6 | -1.32 | 0.00003060 | P25445 | FAS | Tumor necrosis factor receptor superfamily member 6 |
| | 7 | Isoform 2 of TNR6_Human | | | | | P25445-2 | | |
| | 8 | Isoform 3 of TNR6_Human | | | | | P25445-3 | | |
| | 9 | Isoform 4 of TNR6_Human | | | | | P25445-4 | | |
| | 10 | Isoform 5 of TNR6_Human | | | | | P25445-5 | | |
| | 11 | Isoform 6 of TNR6_Human | | | | | P25445-6 | | |
| | 12 | Isoform 7 of TNR6_Human | | | | | P25445-7 | | |
| **7** | 13 | IFNA1_HUMAN | pre | 3 | -0.99 | 0.03511896 | P01562 | IFNA1; IFNA13 | Interferon alpha-1/13 |
| | | | diag | 6 | 1.09 | 0.00041027 | | | |
| **8** | 14 | BASI_HUMAN | pre | 5 | -1.00 | 0.00456896 | P35613 | BSG | Basigin |
| | 15 | Isoform 2 of BASI_Human | | | | | P35613-2 | | |
| | 16 | Isoform 3 of BASI_Human | | | | | P35613-3 | | |
| | 17 | Isoform 4 of BASI_Human | | | | | P35613-4 | | |

EP 3 904 883 A1

| No. | SEQ ID No. | Uniprot entry name | Pre/ diag | Qual score | logFC | adj.p-Val | Uniprot accession | Gene name | Protein name |
|---|---|---|---|---|---|---|---|---|---|
| 9 | 18 | CCL7_HUMAN | pre | 5 | -0.82 | 0.00082227 | P80098 | CCL7 | C-C motif chemokine 7 |
| | | | diag | 3 | 0.74 | 0.04367345 | | | |
| 10 | 19 | CD9_HUMAN | pre | 5 | -0.78 | 0.00077460 | P21926 | CD9 | CD9 antigen |
| 11 | 20 | DKK2_HUMAN | pre | 5 | -0.78 | 0.00077460 | Q9UBU2 | DKK2 | Dickkopf-related protein 2 |
| 12 | 21 | HMMR_HUMAN | pre | | | | O75330 | HMMR | Hyaluronan mediated motility receptor |
| | 22 | Isoform 2 of HMMR_HUMAN | | 5 | -1.02 | 0.00743181 | O75330-2 | | |
| | 23 | Isoform 3 of HMMR_HUMAN | | | | | O75330-3 | | |
| | 24 | Isoform 4 of HMMR_HUMAN | | | | | O75330-4 | | |
| 13 | 25 | IL18_HUMAN | pre | 5 | -1.03 | 0.00709074 | Q14116 | IL18 | Interleukin-18 |
| | | | diag | 4 | 1.00 | 0.01705270 | | | |
| | 26 | Isoform 2 of IL18_HUMAN | | | | | Q14116-2 | | |
| 14 | 27 | IL7_HUMAN | pre | 5 | -0.98 | 0.00077460 | P13232 | IL7 | Interleukin-7 |
| | | | diag | 4 | 1.02 | 0.01917485 | | | |
| | 28 | Isoform 2 of IL7_HUMAN | | | | | P13232-2 | | |
| | 29 | Isoform 3 of IL7_HUMAN | | | | | P13232-3 | | |
| 15 | 30 | PRIO_HUMAN | pre | 5 | -0.95 | 0.00077460 | P04156 | PRNP | Major prion protein |
| 16 | 31 | PTPRC_HUMAN | pre | 5 | -0.93 | 0.00021371 | P08575 | PTPRC | Receptor-type tyrosine-protein phosphatase C |
| | 32 | Isoform 2 of PTPRC_HUMAN | | | | | P08575-4 | | |
| | 33 | Isoform 3 of PTPRC_HUMAN | | | | | P08575-5 | | |
| | 34 | Isoform 4 of PTPRC_HUMAN | | | | | P08575-6 | | |
| | 35 | Isoform 5 of PTPRC_HUMAN | | | | | P08575-7 | | |

| No. | SEQ ID No. | Uniprot entry name | Pre/ diag | Qual score | logFC | adj.p-Val | Uniprot accession | Gene name | Protein name |
|---|---|---|---|---|---|---|---|---|---|
| | 36 | Isoform 6 of PTPRC_ HUMAN | | | | | P08575-8 | | |
| | 37 | Isoform 7 of PTPRC_ HUMAN | | | | | P08575-9 | | |
| | 38 | Isoform 8 of PTPRC_ HUMAN | | | | | P08575-10 | | |
| 17 | 39 | SELPL_HUMAN | pre | 5 | -0.96 | 0.00020190 | Q14242 | SELPLG | P-selectin glycoprotein ligand 1 |
| | 40 | Isoform 2 of SELPL_HUMAN | | | | | Q14242-2 | | |
| 18 | 41 | TNF14_HUMAN | pre | 5 | -1.23 | 0.00896522 | 043557 | TNFSF14 | Tumor necrosis factor ligand superfamily member 14 |
| | 42 | Isoform 2 of TNF14_HUMAN | | | | | 043557-2 | | |
| 19 | 43 | TOP2A_HUMAN | pre | 5 | -0.88 | 0.00077460 | P11388 | TOP2A | DNA topoisomerase 2-alpha |
| | 44 | Isoform 2 of TOP2A_ HUMAN | | | | | P11388-2 | | |
| | 45 | Isoform 3 of TOP2A_ HUMAN | | | | | P11388-3 | | |
| | 46 | Isoform 4 of TOP2A_ HUMAN | | | | | P11388-4 | | |
| 20 | 47 | BDNF_HUMAN | pre | 4 | -1.03 | 0.03226915 | P23560 | BDNF | Brain-derived neurotrophic factor |
| | 48 | Isoform 2 of BDNF_HUMAN | | | | | P23560-2 | | |
| | 49 | Isoform 3 of BDNF_HUMAN | | | | | P23560-3 | | |
| | 50 | Isoform 4 of BDNF_HUMAN | | | | | P23560-4 | | |
| | 51 | Isoform 5 of BDNF_HUMAN | | | | | P23560-5 | | |
| 21 | 52 | CASP8_HUMAN | pre | 4 | -0.98 | 0.00743181 | Q14790 | CASP8 | Caspase-8 |
| | | | diag | 2 | 0.89 | 0.04367345 | | | |
| | 53 | Isoform 2 of CASP8_HUMAN | | | | | Q14790-2 | | |

| No. | SEQ ID No. | Uniprot entry name | Pre/ diag | Qual score | logFC | adj.p-Val | Uniprot accession | Gene name | Protein name |
|---|---|---|---|---|---|---|---|---|---|
| | 54 | Isoform 3 of CASP8_HUMAN | | | | | Q14790-3 | | |
| | 55 | Isoform 4 of CASP8_HUMAN | | | | | Q14790-4 | | |
| | 56 | Isoform 5 of CASP8_HUMAN | | | | | Q14790-5 | | |
| | 57 | Isoform 6 of CASP8_HUMAN | | | | | Q14790-6 | | |
| | 58 | Isoform 7 of CASP8_HUMAN | | | | | Q14790-7 | | |
| | 59 | Isoform 8 of CASP8_HUMAN | | | | | Q14790-8 | | |
| | 60 | Isoform 9 of CASP8_HUMAN | | | | | Q14790-9 | | |
| 22 | 61 | CCL11_HUMAN | pre | 4 | -0.92 | 0.00743181 | P51671 | CCL11 | Eotaxin |
| | | | diag | 3 | 1.15 | 0.01633199 | | | |
| 23 | 62 | CCL3_HUMAN | pre | 4 | -1.00 | 0.03040191 | P10147 | CCL3 | C-C motif chemokine 3 |
| 24 | 63 | CCL5_HUMAN | pre | 4 | -1.07 | 0.00896522 | P13501 | CCL5 | C-C motif chemokine 5 |
| 25 | 64 | CD14_HUMAN | pre | 4 | -0.87 | 0.00975827 | P08571 | CD14 | Monocyte differentiation antigen CD14 |
| 26 | 65 | CRLF2_HUMAN | pre | 4 | -1.08 | 0.00866136 | Q9HC73 | CRLF2 | Cytokine receptor-like factor 2 |
| | | | diag | 3 | 1.01 | 0.01848700 | | | |
| | 66 | Isoform 2 of CRLF2_HUMAN | | | | | Q9HC73-2 | | |
| | 67 | Isoform 3 of CRLF2_HUMAN | | | | | Q9HC73-3 | | |
| 27 | 68 | LMNB1_HUMAN | pre | 4 | -0.78 | 0.00041269 | P20700 | LMNB1 | Lamin-BI |
| 28 | 69 | P53_HUMAN | pre | 4 | 0.81 | 0.00173864 | P04637 | TP53 | Cellular tumor antigen p53 |
| | 70 | Isoform 2 of P53_HUMAN | | | | | P04637-2 | | |

EP 3 904 883 A1

34

| No. | SEQ ID No. | Uniprot entry name | Pre/diag | Qual score | logFC | adj.p-Val | Uniprot accession | Gene name | Protein name |
|---|---|---|---|---|---|---|---|---|---|
| | 71 | Isoform 3 of P53_HUMAN | | | | | P04637-3 | | |
| | 72 | Isoform 4 of P53_HUMAN | | | | | P04637-4 | | |
| | 73 | Isoform 5 of P53_HUMAN | | | | | P04637-5 | | |
| | 74 | Isoform 6 of P53_HUMAN | | | | | P04637-6 | | |
| | 75 | Isoform 7 of P53_HUMAN | | | | | P04637-7 | | |
| | 76 | Isoform 8 of P53_HUMAN | | | | | P04637-8 | | |
| | 77 | Isoform 9 of P53_HUMAN | | | | | P04637-9 | | |
| 29 | 78 | PAK1_HUMAN | pre | 4 | -0.74 | 0.00901111 | Q13153 | PAK1 | Serine/threonine-protein kinase PAK 1 |
| | 79 | Isoform 2 of PAK1_HUMAN | | | | | Q13153-2 | | |
| 30 | 80 | CASP9_HUMAN | diag | 4 | 1.26 | 0.01917485 | P55211 | CASP9 | Caspase-9 |
| | 81 | Isoform 2 of CASP9_HUMAN | | | | | P55211-2 | | |
| | 82 | Isoform 3 of CASP9_HUMAN | | | | | P55211-3 | | |
| | 83 | Isoform 4 of CASP9_HUMAN | | | | | P55211-4 | | |
| 31 | 84 | BGH3_HUMAN | pre | 3 | -0.79 | 0.00768649 | Q15582 | TGFBI | Transforming growth factor-beta-induced protein ig-h3 |
| 32 | 85 | CD47_HUMAN | pre | 3 | -0.54 | 0.00118575 | Q08722 | CD47 | Leukocyte surface antigen CD47 |
| | 86 | Isoform OA3-293 of CD47_HUMAN | | | | | Q08722-2 | | |
| | 87 | Isoform OA3-305 of CD47_HUMAN | | | | | Q08722-3 | | |
| | 88 | Isoform OA3-312 of CD47_HUMAN | | | | | Q08722-4 | | |
| 33 | 89 | CD8A_HUMAN | pre | 3 | -0.93 | 0.02217931 | P01732 | CD8A | T-cell surface glycoprotein CD8 alpha chain |

| No. | SEQ ID No. | Uniprot entry name | Pre/diag | Qual score | logFC | adj.p-Val | Uniprot accession | Gene name | Protein name |
|---|---|---|---|---|---|---|---|---|---|
| | 90 | Isoform 2 of CD8A_HUMAN | | | | | P01732-2 | | |
| | 91 | Isoform 3 of CD8A_HUMAN | | | | | P01732-3 | | |
| 34 | 92 | DKK3_HUMAN | pre | 3 | -0.77 | 0.03511896 | Q9UBP4 | DKK3 | Dickkopf-related protein 3 |
| 35 | 93 | GAS6_HUMAN | pre | 3 | -0.83 | 0.01370348 | Q14393 | GAS6 | Growth arrest-specific protein 6 |
| | | | diag | 2 | 0.79 | 0.01633199 | | | |
| | 94 | Isoform 2 of GAS6_HUMAN | | | | | Q14393-1 | | |
| | 95 | Isoform 3 of GAS6_HUMAN | | | | | Q14393-3 | | |
| | 96 | Isoform 4 of GAS6_HUMAN | | | | | Q14393-4 | | |
| | 97 | Isoform 5 of GAS6_HUMAN | | | | | Q14393-5 | | |
| 36 | 98 | IL15_HUMAN | pre | 3 | -0.94 | 0.01585528 | P40933 | IL15 | Interleukin-15 |
| | | | diag | 3 | 1.08 | 0.01242697 | | | |
| | 99 | Isoform IL15-S21AA of IL15_HUMAN | | | | | P40933-2 | | |
| 37 | 100 | IL3RB_HUMAN | pre | 3 | -0.80 | 0.01834127 | P32927 | CSF2RB | Cytokine receptor common subunit beta |
| | 101 | Isoform 2 o-fIL3RB_HUMAN | | | | | P32927-2 | | |
| 38 | 102 | K2C8_HUMAN | pre | 3 | 0.97 | 0.03749303 | P05787 | KRT8 | Keratin, type II cytoskeletal 8 |
| | 103 | Isoform 2 of K2C8_HUMAN | | | | | P05787-2 | | |
| 39 | 104 | LEUK_HUMAN | | 3 | -0.59 | 0.00095308 | P16150 | SPN | Leukosialin |
| 40 | 105 | MARK4_HUMAN | pre | 3 | -0.79 | 0.00167421 | Q96L34 | MARK4 | MAP/microtubule affinity-regulating kinase 4 |
| | 106 | Isoform 2 of MARK4_HUMAN | | | | | Q96L34-2 | | |
| 41 | 107 | MELPH_HUMAN | pre | 3 | -0.76 | 0.03511896 | Q9BV36 | MLPH | Melanophilin |
| | 108 | Isoform 2 of MELPH_ HUMAN | | | | | Q9BV36-2 | | |

EP 3 904 883 A1

| No. | SEQ ID No. | Uniprot entry name | Pre/diag | Qual score | logFC | adj.p-Val | Uniprot accession | Gene name | Protein name |
|---|---|---|---|---|---|---|---|---|---|
| | 109 | Isoform 3 of MELPH_HUMAN | | | | | Q9BV36-3 | | |
| | 110 | Isoform 4 of MELPH_HUMAN | | | | | Q9BV36-4 | | |
| | 111 | Isoform 5 of MELPH_HUMAN | | | | | Q9BV36-5 | | |
| 42 | 112 | MMP1_HUMAN | pre | 3 | -0.59 | 0.00208582 | P03956 | MMP1 | Interstitial collagenase |
| | | | diag | 2 | 0.71 | 0.01633199 | | | |
| 43 | 113 | MMP7_HUMAN | pre | 3 | -0.96 | 0.03511896 | P09237 | MMP7 | Matrilysin |
| 44 | 114 | PGH1_HUMAN | pre | 3 | -0.81 | 0.00801786 | P23219 | PTGS1 | Prostaglandin G/H synthase 1 |
| | 115 | Isoform 2 of PGH1_HUMAN | | | | | P23219-2 | | |
| | 116 | Isoform 3 of PGH1_HUMAN | | | | | P23219-3 | | |
| | 117 | Isoform 4 of PGH1_HUMAN | | | | | P23219-4 | | |
| | 118 | Isoform 5 of PGH1_HUMAN | | | | | P23219-5 | | |
| | 119 | Isoform 6 of PGH1_HUMAN | | | | | P23219-6 | | |
| 45 | 120 | PRTN3_HUMAN | pre | 3 | -0.76 | 0.02563658 | P24158 | PRTN3 | Myeloblastin |
| 46 | 121 | RBM3_HUMAN | pre | 3 | -0.88 | 0.00173864 | P98179 | RBM3 | RNA-binding protein 3 |
| | | | diag | 3 | 0.96 | 0.01754186 | | | |
| 47 | 122 | SAMP_HUMAN | pre | 3 | 1.60 | 0.04927846 | P02743 | APCS | Serum amyloid P-component |
| 48 | 123 | TSN16_HUMAN | pre | 3 | -0.95 | 0.01860133 | Q9UKR8 | TSPAN16 | Tetraspanin-16 |
| | 124 | Isoform 2 of TSN16_HUMAN | | | | | Q9UKR8-2 | | |
| | 125 | Isoform 3 of TSN16_HUMAN | | | | | Q9UKR8-3 | | |
| | 126 | Isoform 4 of TSN16_HUMAN | | | | | Q9UKR8-4 | | |
| 49 | 127 | UROK_HUMAN | pre | 3 | 1.05 | 0.01289580 | P00749 | PLAU | Urokinase-type plasminogen activator |
| | 128 | Isoform 2 of UROK_HUMAN | | | | | P00749-2 | | |

| No. | SEQ ID No. | Uniprot entry name | Pre/ diag | Qual score | logFC | adj.p-Val | Uniprot accession | Gene name | Protein name |
|---|---|---|---|---|---|---|---|---|---|
| 50 | 129 | PYRG1_HUMAN | diag | 3 | 1.03 | 0.04367345 | P17812 | CTPS1 | CTP synthase 1 |
| | 130 | Isoform 2 of PYRG1_HUMAN | | | | | P17812-2 | | |
| 51 | no ID | CD139 | diag | 3 | -1.22 | 0.03468974 | no entry | CD139 | |
| 52 | 131 | MAD4_HUMAN | diag | 3 | 1.09 | 0.01633199 | Q14582 | MXD4 | Max dimerization protein 4 |
| 53 | 132 | TMM54_HUMAN | diag | 3 | 0.80 | 0.01242697 | Q969K7 | TMEM54 | Transmembrane protein 54 |
| | 133 | Isoform 2 of TMM54_HUMAN | | | | | Q969K7-2 | | |
| | 134 | Isoform 3 of TMM54_HUMAN | | | | | Q969K7-3 | | |
| 54 | 135 | ACTB_HUMAN | pre | 2 | -0.66 | 0.01989421 | P60709 | ACTB | Actin, cytoplasmic 1 |
| 55 | 136 | CASP3_HUMAN | pre | 2 | -0.77 | 0.01672936 | P42574 | CASP3 | Caspase-3 |
| 56 | 137 | DAF_HUMAN | pre | 2 | 0.51 | 0.02261542 | P08174 | CD55 | Complement decay-accelerating factor |
| | 138 | Isoform 1 of DAF_HUMAN | | | | | P08174-2 | | |
| | 139 | Isoform 3 of DAF_HUMAN | | | | | P08174-3 | | |
| | 140 | Isoform 4 of DAF_HUMAN | | | | | P08174-4 | | |
| | 141 | Isoform 5 of DAF_HUMAN | | | | | P08174-5 | | |
| | 142 | Isoform 6 of DAF_HUMAN | | | | | P08174-6 | | |
| | 143 | Isoform 7 of DAF_HUMAN | | | | | P08174-7 | | |
| 57 | 144 | HMGB2_HUMAN | pre | 2 | 0.72 | 0.03511896 | P26583 | HMGB2 | High mobility group protein B2 |
| 58 | 145 | HXC11_HUMAN | pre | 2 | 0.91 | 0.03511896 | 043248 | HOXC11 | Homeobox protein Hox-C11 |
| 59 | 146 | ICAM1_HUMAN | pre | 2 | 0.71 | 0.02564186 | P05362 | ICAM1 | Intercellular adhesion molecule 1 |
| 60 | 147 | IL12A_HUMAN | pre | 2 | -0.71 | 0.03511896 | P29459 | IL12A | Interleukin-12 subunit alpha |
| 61 | 148 | KLF8_HUMAN | pre | 2 | -0.67 | 0.01886910 | 095600 | KLF8 | Krueppel-like factor 8 |
| | | | diag | 2 | -0.93 | 0.04479542 | | | |

EP 3 904 883 A1

38

| No. | SEQ ID No. | Uniprot entry name | Pre/diag | Qual score | logFC | adj.p-Val | Uniprot accession | Gene name | Protein name |
|---|---|---|---|---|---|---|---|---|---|
| | 149 | Isoform 2 of KLF8_HUMAN | | | | | 095600-3 | | |
| | 150 | Isoform 3 of KLF8_HUMAN | | | | | 095600-4 | | |
| | 151 | Isoform 4 of KLF8_HUMAN | | | | | 095600-5 | | |
| 62 | 152 | LEG4_HUMAN | pre | 2 | -0.80 | 0.03511896 | P56470 | LGALS4 | Galectin-4 |
| 63 | 153 | LTOR1_HUMAN | pre | 2 | 0.83 | 0.04600786 | Q6IAA8 | LAMTOR1 | Ragulator complex protein LAMTOR1 |
| 64 | 154 | LYAM1_HUMAN | pre | 2 | -0.62 | 0.03226915 | P14151 | SELL | L-selectin |
| | 155 | Isoform 2 of LYAM1_HUMAN | | | | | P14151-2 | | |
| 65 | 156 | MK03_HUMAN | pre | 2 | -0.77 | 0.01886910 | P27361 | MAPK3 | Mitogen-activated protein kinase 3 |
| | 157 | Isoform 2 of MK03_HUMAN | | | | | P27361-2 | | |
| | 158 | Isoform 3 of MK03_HUMAN | | | | | P27361-3 | | |
| 66 | 159 | MUC5B_HUMAN | pre | 2 | -0.80 | 0.02047452 | Q9HC84 | MUC5B | Mucin-5B |
| 67 | 160 | NFAC4_HUMAN | pre | 2 | -0.50 | 0.01281061 | Q14934 | NFATC4 | Nuclear factor of activated T-cells |
| | 161 | Isoform 2 of NFAC4_HUMAN | | | | | Q14934-2 | | |
| | 162 | Isoform 3 of NFAC4_HUMAN | | | | | Q14934-3 | | |
| | 163 | Isoform 4 of NFAC4_HUMAN | | | | | Q14934-4 | | |
| | 164 | Isoform 5 of NFAC4_HUMAN | | | | | Q14934-5 | | |
| | 165 | Isoform 6 of NFAC4_HUMAN | | | | | Q14934-6 | | |
| | 166 | Isoform 7 of NFAC4_HUMAN | | | | | Q14934-7 | | |
| | 167 | Isoform 8 of NFAC4_HUMAN | | | | | Q14934-8 | | |

| No. | SEQ ID No. | Uniprot entry name | Pre/ diag | Qual score | logFC | adj.p-Val | Uniprot accession | Gene name | Protein name |
|---|---|---|---|---|---|---|---|---|---|
| | 168 | Isoform 9 of NFAC4_HUMAN | | | | | Q14934-9 | | |
| | 169 | Isoform 10 of NFAC4_HUMAN | | | | | Q14934-10 | | |
| | 170 | Isoform 11 of NFAC4_HUMAN | | | | | Q14934-11 | | |
| | 171 | Isoform 12 of NFAC4_HUMAN | | | | | Q14934-12 | | |
| | 172 | Isoform 13 of NFAC4_HUMAN | | | | | Q14934-13 | | |
| | 173 | Isoform 14 of NFAC4_HUMAN | | | | | Q14934-14 | | |
| | 174 | Isoform 15 of NFAC4_HUMAN | | | | | Q14934-15 | | |
| | 175 | Isoform 16 of NFAC4_HUMAN | | | | | Q14934-16 | | |
| | 176 | Isoform 17 of NFAC4_HUMAN | | | | | Q14934-17 | | |
| | 177 | Isoform 18 of NFAC4_HUMAN | | | | | Q14934-18 | | |
| | 178 | Isoform 19 of NFAC4_HUMAN | | | | | Q14934-19 | | |
| | 179 | Isoform 20 of NFAC4_HUMAN | | | | | Q14934-20 | | |
| | 180 | Isoform 21 of NFAC4_HUMAN | | | | | Q14934-21 | | |
| | 181 | Isoform 22 of NFAC4_HUMAN | | | | | Q14934-22 | | |

| No. | SEQ ID No. | Uniprot entry name | Pre/diag | Qual score | logFC | adj.p-Val | Uniprot accession | Gene name | Protein name |
|---|---|---|---|---|---|---|---|---|---|
| | 182 | Isoform 23 of NFAC4_HUMAN | | | | | Q14934-23 | | |
| | 183 | Isoform 24 of NFAC4_HUMAN | | | | | Q14934-24 | | |
| 68 | 184 | TGFB1_HUMAN | pre | 2 | 0.81 | 0.02912596 | P01137 | TGFB1 | Transforming growth factor beta-1 pro-protein |
| 69 | 185 | VRK1_HUMAN | pre | 2 | -0.87 | 0.04772231 | Q99986 | VRK1 | Serine/threonine-protein kinase VRK1 |
| 70 | 186 | CDKN3_HUMAN | diag | 2 | -0.71 | 0.01978279 | Q16667 | CDKN3 | Cyclin-dependent kinase inhibitor 3 |
| | 187 | Isoform 2 of CDKN3_HUMAN | | | | | Q16667-2 | | |
| 71 | 188 | TFPI2_HUMAN | diag | 2 | -0.79 | 0.03075045 | P48307 | TFPI2 | Tissue factor pathway inhibitor 2 |
| | 189 | Isoform 2 of TFPI2_HUMAN | | | | | P48307-2 | | |
| 72 | 190 | MLP3B_HUMAN | diag | 2 | 0.99 | 0.01633199 | Q9GZQ8 | MAP1LC3B | Microtubule-associated proteins 1A/1B light chain 3B |
| 73 | 191 | PTEN_HUMAN | diag | 2 | 0.93 | 0.01633199 | P60484 | PTEN | Phosphatidylinositol 3,4,5-trisphosphate 3-phosphatase and dual-specificity protein phosphatase PTEN |
| | 192 | Isoform alpha of PTEN_HUMAN | | | | | P60484-2 | | |
| | 193 | Isoform 3 of PTEN_HUMAN | | | | | P60484-3 | | |
| 74 | 194 | IL8_HUMAN | pre | 2 | -0.83 | 0.03511896 | P10145 | CXCL8 | Interleukin-8 |
| 75 | 195 | ARHG2_HUMAN | pre | 1 | -0.41 | 0.03565782 | Q92974 | ARHGEF2 | Rho guanine nucleotide exchange factor 2 |
| | 196 | Isoform 2 of ARHG2_HUMAN | | | | | Q92974-2 | | |
| | 197 | Isoform 3 of ARHG2_HUMAN | | | | | Q92974-3 | | |
| 76 | 198 | CFLAR_HUMAN | pre | 1 | 0.52 | 0.03006471 | 015519 | CFLAR | CASP8 and FADD-like apoptosis |

| No. | SEQ ID No. | Uniprot entry name | Pre / diag | Qual score | logFC | adj.p-Val | Uniprot accession | Gene name | Protein name |
|---|---|---|---|---|---|---|---|---|---|
| | | | | | | | | | regulator |
| | 199 | Isoform 2 of CFLAR_ HUMAN | | | | | 015519-2 | | |
| | 200 | Isoform 3 of CFLAR_ HUMAN | | | | | O15519-3 | | |
| | 201 | Isoform 4 of CFLAR_ HUMAN | | | | | 015519-4 | | |
| | 202 | Isoform 5 of CFLAR_ HUMAN | | | | | O15519-5 | | |
| | 203 | Isoform 6 of CFLAR_ HUMAN | | | | | 015519-6 | | |
| | 204 | Isoform 7 of CFLAR_ HUMAN | | | | | O15519-7 | | |
| | 205 | Isoform 8 of CFLAR_ HUMAN | | | | | 015519-8 | | |
| | 206 | Isoform 9 of CFLAR_ HUMAN | | | | | O15519-9 | | |
| | 207 | Isoform 10 of CFLAR_ HUMAN | | | | | 015519-10 | | |
| | 208 | Isoform 11 of CFLAR_ HUMAN | | | | | O15519-11 | | |
| | 209 | Isoform 12 of CFLAR_ HUMAN | | | | | 015519-12 | | |
| | 210 | Isoform 13 of CFLAR_ HUMAN | | | | | 015519-13 | | |
| | 211 | Isoform 14 of CFLAR_ HUMAN | | | | | O15519-14 | | |
| | 212 | Isoform 15 of CFLAR_ HUMAN | | | | | 015519-15 | | |

EP 3 904 883 A1

| No. | SEQ ID No. | Uniprot entry name | Pre / diag | Qual score | logFC | adj.p-Val | Uniprot accession | Gene name | Protein name |
|---|---|---|---|---|---|---|---|---|---|
| 77 | 213 | CUED2_HUMAN | pre | 1 | -0.70 | 0.01007946 | Q9H467 | CUEDC2 | CUE domain-containing protein 2 |
| 78 | 214 | DAPK1_HUMAN | pre | 1 | -0.65 | 0.02261542 | P53355 | DAPK1 | Death-associated protein kinase 1 |
| | 215 | Isoform 2 of DAPK1_HUMAN | | | | | P53355-2 | | |
| | 216 | Isoform 3 of DAPK1_HUMAN | | | | | P53355-3 | | |
| | 217 | Isoform 4 of DAPK1_HUMAN | | | | | P53355-4 | | |
| 79 | 218 | EDNRA_HUMAN | pre | 1 | 0.58 | 0.02900873 | P25101 | EDNRA | Endothelin-1 receptor |
| | 219 | Isoform 2 of EDNRA_ HUMAN | | | | | P25101-2 | | |
| | 220 | Isoform 3 of EDNRA_ HUMAN | | | | | P25101-3 | | |
| | 221 | Isoform 4 of EDNRA_ HUMAN | | | | | P25101-4 | | |
| | 222 | Isoform 5 of EDNRA_ HUMAN | | | | | P25101-5 | | |
| 80 | 223 | EIF3B_HUMAN | pre | 1 | 0.69 | 0.01829644 | P55884 | EIF3B | Eukaryotic translation initiation factor 3 subunit B |
| | 224 | Isoform 2 of EIF3B_HUMAN | | | | | P55884-2 | | |
| 81 | 225 | ID2_HUMAN | pre | 1 | 0.32 | 0.03707986 | Q02363 | ID2 | DNA-binding protein inhibitor ID-2 |
| 82 | 226 | LMNA_HUMAN | pre | 1 | -0.63 | 0.03353007 | P02545 | LMNA | Prelamin-A/C |
| | 227 | Isoform C of LMNA_HUMAN | | | | | P02545-2 | | |
| | 228 | Isoform ADelta10 of LMNA_ HUMAN | | | | | P02545-3 | | |
| | 229 | Isoform 4 of LMNA_HUMAN | | | | | P02545-4 | | |
| | 230 | Isoform 5 of LMNA_HUMAN | | | | | P02545-5 | | |

43

| No. | SEQ ID No. | Uniprot entry name | Pre / diag | Qual score | logFC | adj.p-Val | Uniprot accession | Gene name | Protein name |
|---|---|---|---|---|---|---|---|---|---|
| | 231 | Isoform 6 of LMNA_HUMAN | | | | | P02545-6 | | |
| 83 | 232 | PYR1_HUMAN | pre | 1 | -0.65 | 0.01284425 | P27708 | CAD | CAD protein |
| 84 | 233 | ZN593_HUMAN | pre | 1 | 0.62 | 0.01284425 | O00488 | ZNF593 | Zinc finger protein 593 |
| 85 | 234 | MK12_HUMAN | diag | 1 | 0.69 | 0.04367345 | P53778 | MAPK12 | Mitogen-activated protein kinase 12 |
| | 235 | Isoform 2 of MK12_HUMAN | | | | | P53778-2 | | |
| 86 | 236 | CP1B1_HUMAN | diag | 1 | -0.56 | 0.01978279 | Q16678 | CYP1B1 | Cytochrome P450 1B1 |
| 87 | 237 | ANGT_HUMAN | | 0 | -0.42 | 0.04109223 | P01019 | AGT | Angiotensinogen |
| 88 | 238 | APC_HUMAN | pre | 0 | -0.42 | 0.02825626 | P25054 | APC | Adenomatous polyposis coli protein |
| | 239 | Isoform 2 of APC_HUMAN | | | | | P25054-2 | | |
| | 240 | Isoform 1B of APC_HUMAN | | | | | P25054-3 | | |
| 89 | 241 | P02F1_HUMAN | pre | 0 | 0.31 | 0.02825626 | P14859 | POU2F1 | POU domain, class 2, transcription factor 1 |
| | 242 | Isoform 2 of P02F1_HUMAN | | | | | P14859-2 | | |
| | 243 | Isoform 3 of P02F1_HUMAN | | | | | P14859-3 | | |
| | 244 | Isoform 6 of P02F1_HUMAN | | | | | P14859-6 | | |
| | 245 | Isoform 4 of P02F1_HUMAN | | | | | P14859-4 | | |
| | 246 | Isoform 5 of P02F1_HUMAN | | | | | P14859-5 | | |
| 90 | 247 | SSR4_HUMAN | pre | 0 | -0.40 | 0.02825626 | P31391 | SSTR4 | Somatostatin receptor type 4 |
| 91 | 248 | TNAP3_HUMAN | pre | 0 | -0.49 | 0.03707986 | P21580 | TNFAIP3 | Tumor necrosis factor alpha-induced protein 3 |
| 92 | 249 | TRI22_HUMAN | diag | 0 | 0.44 | 0.04624814 | Q8IYM9 | TRIM22 | E3 ubiquitin-protein ligase TRIM22 |
| | 250 | Isoform 2 of TRI22_HUMAN | | | | | Q8IYM9-2 | | |

EP 3 904 883 A1

44

The biomarkers of the present invention were identified by binding to immobilized antibodies, except ligands of the Tumor necrosis factor receptor superfamily member 1B (Marker No. 5 in Table 1, Marker No. 4 in Table 2, Marker No. 5 in Table 6). Here, an immobilized Fc-Tumor necrosis factor receptor superfamily member 1B-fusion protein was used for capturing ligands of this receptor including Tumor necrosis factor and Lymphotoxin-alpha.

"Qual score" indicates a quality score that takes into account logFC-values, adjusted p-values as well as stripcharts (graphical representations of differential abundance distribution as well as discrimination power).

LogFC-values and adjusted p-values are shown in Table 1 as well. Positive logFC-values indicate that the respective biomarker is upregulated in AKI patients as compared to patients without AKI. Negative logFC-values indicate that the respective biomarker is downregulated in AKI patients as compared to patients without AKI.

The Uniprot entry name and the Uniprot accession number are indicated if applicable. Not all biomarkers of the invention are proteins so that not every biomarker has a UniProt entry.

Table 1, Table 5 and Table 6 comprise a combination of predictive and diagnostic biomarkers, Table 4 comprises biomarkers identified as predictive as well as diagnostic. Biomarkers identified in the predictive assay are indicated by "pre", biomarkers identified in the diagnostic assay are indicated by "diag" within these tables.

Table 2 shows the 78 predictive biomarkers of the present invention.

*Table 2*

| No. | SEQ ID NO | Uniprot entry name | Qual score | logFC | adj.p-Val | Uniprot accession | Gene name | Protein name |
|---|---|---|---|---|---|---|---|---|
| **1** | no ID | CD15 (no entry, no protein) | 6 | -0.86 | 0.00000004 | no entry | CD15 | |
| **2** | 2 | CD99_HUMAN | 6 | -0.98 | 0.00000440 | P14209 | CD99 | CD99 antigen |
| | 3 | Isoform II of CD99_HUMAN | | | | P14209-2 | | |
| | 4 | Isoform 3 of CD99_HUMAN | | | | P14209-3 | | |
| **3** | 5 | FCERA_HUMAN | 6 | -1.63 | 0.00049891 | P12319 | FCER1A | High affinity immunoglobulin epsilon receptor subunit alpha |
| **4** | 251-252 | No entry (Ligands of TNR1B_HUMAN including TNFA_HUMAN and TNFB_HUMAN) | 6 | -1.60 | 0.00000083 | P01375 and P01374 | TNF and LTA | Ligands of Tumor necrosis factor receptor superfamily member 1B including Tumor necrosis factor and Lymphotoxin-alpha |
| **5** | 6 | TNR6_HUMAN | 6 | -1.32 | 0.00003060 | P25445 | FAS | Tumor necrosis factor receptor superfamily member 6 |
| | 7 | Isoform 2 of TNR6_Human | | | | P25445-2 | | |
| | 8 | Isoform 3 of TNR6_Human | | | | P25445-3 | | |
| | 9 | Isoform 4 of TNR6_Human | | | | P25445-4 | | |
| | 10 | Isoform 5 of TNR6_Human | | | | P25445-5 | | |
| | 11 | Isoform 6 of TNR6_Human | | | | P25445-6 | | |
| | 12 | Isoform 7 of TNR6_Human | | | | P25445-7 | | |
| **6** | 14 | BASI_HUMAN | 5 | -1.00 | 0.00456896 | P35613 | BSG | Basigin |
| | 15 | Isoform 2 of BASI_Human | | | | P35613-2 | | |
| | 16 | Isoform 3 of BASI_Human | | | | P35613-3 | | |
| | 17 | Isoform 4 of BASI_Human | | | | P35613-4 | | |
| **7** | 18 | CCL7_HUMAN | 5 | -0.82 | 0.00082227 | P80098 | CCL7 | C-C motif chemokine 7 |
| **8** | 19 | CD9_HUMAN | 5 | -0.78 | 0.00077460 | P21926 | CD9 | CD9 antigen |
| **9** | 20 | DKK2_HUMAN | 5 | -0.78 | 0.00077460 | Q9UBU2 | DKK2 | Dickkopf-related protein 2 |
| **10** | 21 | HMMR_HUMAN | | | | 075330-1 | HMMR | Hyaluronan mediated motility receptor |

(continued)

| No. | SEQ ID NO | Uniprot entry name | Qual score | logFC | adj.p-Val | Uniprot accession | Gene name | Protein name |
|---|---|---|---|---|---|---|---|---|
| | 22 | Isoform 2 of HMMR_HUMAN | 5 | -1.02 | 0.00743181 | 075330-2 | | |
| | 23 | Isoform 3 of HMMR_HUMAN | | | | 075330-3 | | |
| | 24 | Isoform 4 of HMMR_HUMAN | | | | 075330-4 | | |
| 11 | 25 | IL18_HUMAN | 5 | -1.03 | 0.00709074 | Q14116 | IL18 | Interleukin-18 |
| | 26 | Isoform 2 of IL18_HUMAN | | | | | | |
| 12 | 27 | IL7_HUMAN | 5 | -0.98 | 0.00077460 | P13232 | IL7 | Interleukin-7 |
| | 28 | Isoform 2 of IL7_HUMAN | | | | P13232-2 | | |
| | 29 | Isoform 3 of IL7_HUMAN | | | | P13232-3 | | |
| 13 | 30 | PRIO_HUMAN | 5 | -0.95 | 0.00077460 | P04156 | PRNP | Major prion protein |
| 14 | 31 | PTPRC_HUMAN | 5 | -0.93 | 0.00021371 | P08575 | PTPRC | Receptor-type tyrosine-protein phosphatase C |
| | 32 | Isoform 2 of PTPRC_HUMAN | | | | P08575-4 | | |
| | 33 | Isoform 3 of PTPRC_HUMAN | | | | P08575-5 | | |
| | 34 | Isoform 4 of PTPRC_HUMAN | | | | P08575-6 | | |
| | 35 | Isoform 5 of PTPRC_HUMAN | | | | P08575-7 | | |
| | 36 | Isoform 6 of PTPRC_HUMAN | | | | P08575-8 | | |
| | 37 | Isoform 7 of PTPRC_HUMAN | | | | P08575-9 | | |
| | 38 | Isoform 8 of PTPRC_HUMAN | | | | P08575-10 | | |
| 15 | 39 | SELPL_HUMAN | 5 | -0.96 | 0.00020190 | Q14242 | SELPLG | P-selectin glycoprotein ligand 1 |
| | 40 | Isoform 2 of SELPL_HUMAN | | | | Q14242-2 | | |
| 16 | 41 | TNF14_HUMAN | 5 | -1.23 | 0.00896522 | O43557 | TNFSF14 | Tumor necrosis factor ligand superfamily member 14 |
| | 42 | Isoform 2 of TNF14_HUMAN | | | | O43557-2 | | |
| 17 | 43 | TOP2A_HUMAN | 5 | -0.88 | 0.00077460 | P11388 | TOP2A | DNA topoisomerase 2-alpha |
| | 44 | Isoform 2 of TOP2A_HUMAN | | | | P11388-2 | | |
| | 45 | Isoform 3 of TOP2A_HUMAN | | | | P11388-3 | | |

| No. | SEQ ID NO | Uniprot entry name | Qual score | logFC | adj.p-Val | Uniprot accession | Gene name | Protein name |
|---|---|---|---|---|---|---|---|---|
|  | 46 | Isoform 4 of TOP2A_HUMAN |  |  |  | P11388-4 |  |  |
| 18 | 47 | BDNF_HUMAN | 4 | -1.03 | 0.03226915 | P23560 | BDNF | Brain-derived neurotrophic factor |
|  | 48 | Isoform 2 of BDNF_HUMAN |  |  |  | P23560-2 |  |  |
|  | 49 | Isoform 3 of BDNF_HUMAN |  |  |  | P23560-3 |  |  |
|  | 50 | Isoform 4 of BDNF_HUMAN |  |  |  | P23560-4 |  |  |
|  | 51 | Isoform 5 of BDNF_HUMAN |  |  |  | P23560-5 |  |  |
| 19 | 52 | CASP8_HUMAN | 4 | -0.98 | 0.00743181 | Q14790 | CASP8 | Caspase-8 |
|  | 53 | Isoform 2 of CASP8_HUMAN |  |  |  | Q14790-2 |  |  |
|  | 54 | Isoform 3 of CASP8_HUMAN |  |  |  | Q14790-3 |  |  |
|  | 55 | Isoform 4 of CASP8_HUMAN |  |  |  | Q14790-4 |  |  |
|  | 56 | Isoform 5 of CASP8_HUMAN |  |  |  | Q14790-5 |  |  |
|  | 57 | Isoform 6 of CASP8_HUMAN |  |  |  | Q14790-6 |  |  |
|  | 58 | Isoform 7 of CASP8_HUMAN |  |  |  | Q14790-7 |  |  |
|  | 59 | Isoform 8 of CASP8_HUMAN |  |  |  | Q14790-8 |  |  |
|  | 60 | Isoform 9 of CASP8_HUMAN |  |  |  | Q14790-9 |  |  |
| 20 | 61 | CCL11_HUMAN | 4 | -0.92 | 0.00743181 | P51671 | CCL11 | Eotaxin |
| 21 | 62 | CCL3_HUMAN | 4 | -1.00 | 0.03040191 | P10147 | CCL3 | C-C motif chemokine 3 |
| 22 | 63 | CCL5_HUMAN | 4 | -1.07 | 0.00896522 | P13501 | CCL5 | C-C motif chemokine 5 |
| 23 | 64 | CD14_HUMAN | 4 | -0.87 | 0.00975827 | P08571 | CD14 | Monocyte differentiation antigen CD14 |
| 24 | 65 | CRLF2_HUMAN | 4 | -1.08 | 0.00866136 | Q9HC73 | CRLF2 | Cytokine receptor-like factor 2 |
|  | 66 | Isoform 2 of CRLF2_HUMAN |  |  |  | Q9HC73-2 |  |  |
|  | 67 | Isoform 3 of CRLF2_HUMAN |  |  |  | Q9HC73-3 |  |  |
| 25 | 68 | LMNB1_HUMAN | 4 | -0.78 | 0.00041269 | P20700 | LMNB1 | Lamin-Bl |
| 26 | 69 | P53_HUMAN | 4 | 0.81 | 0.00173864 | P04637 | TP53 | Cellular tumor antigen p53 |

| No. | SEQ ID NO | Uniprot entry name | Qual score | logFC | adj.p-Val | Uniprot accession | Gene name | Protein name |
|---|---|---|---|---|---|---|---|---|
| | 70 | Isoform 2 of P53_HUMAN | | | | P04637-2 | | |
| | 71 | Isoform 3 of P53_HUMAN | | | | P04637-3 | | |
| | 72 | Isoform 4 of P53_HUMAN | | | | P04637-4 | | |
| | 73 | Isoform 5 of P53_HUMAN | | | | P04637-5 | | |
| | 74 | Isoform 6 of P53_HUMAN | | | | P04637-6 | | |
| | 75 | Isoform 7 of P53_HUMAN | | | | P04637-7 | | |
| | 76 | Isoform 8 of P53_HUMAN | | | | P04637-8 | | |
| | 77 | Isoform 9 of P53_HUMAN | | | | P04637-9 | | |
| 27 | 78 | PAK1_HUMAN | 4 | -0.74 | 0.00901111 | Q13153 | PAK1 | Serine/threonine-protein kinase PAK 1 |
| | 79 | Isoform 2 of PAK1_HUMAN | | | | Q13153-2 | | |
| 28 | 84 | BGH3_HUMAN | 3 | -0.79 | 0.00768649 | Q15582 | TGFBI | Transforming growth factor-beta-induced protein ig-h3 |
| 29 | 85 | CD47_HUMAN | 3 | -0.54 | 0.00118575 | Q08722 | CD47 | Leukocyte surface antigen CD47 |
| | 86 | Isoform OA3-293 of CD47_HUMAN | | | | Q08722-2 | | |
| | 87 | Isoform OA3-305 of CD47_HUMAN | | | | Q08722-3 | | |
| | 88 | Isoform OA3-312 of CD47_HUMAN | | | | Q08722-4 | | |
| 30 | 89 | CD8A_HUMAN | 3 | -0.93 | 0.02217931 | P01732 | CD8A | T-cell surface glycoprotein CD8 alpha chain |
| | 90 | Isoform 2 of CD8A_HUMAN | | | | P01732-2 | | |
| | 91 | Isoform 3 of CD8A_HUMAN | | | | P01732-3 | | |
| 31 | 92 | DKK3_HUMAN | 3 | -0.77 | 0.03511896 | Q9UBP4 | DKK3 | Dickkopf-related protein 3 |
| 32 | 93 | GAS6_HUMAN | 3 | -0.83 | 0.01370348 | Q14393 | GAS6 | Growth arrest-specific protein 6 |
| | 94 | Isoform 2 of GAS6_HUMAN | | | | Q14393-1 | | |
| | 95 | Isoform 3 of GAS6_HUMAN | | | | Q14393-3 | | |

| No. | SEQ ID NO | Uniprot entry name | Qual score | logFC | adj.p-Val | Uniprot accession | Gene name | Protein name |
|---|---|---|---|---|---|---|---|---|
| | 96 | Isoform 4 of GAS6_HUMAN | | | | Q14393-4 | | |
| | 97 | Isoform 5 of GAS6_HUMAN | | | | Q14393-5 | | |
| 33 | 13 | IFNA1_HUMAN | 3 | -0.99 | 0.03511896 | P01562 | IFNA1; IFNA13 | Interferon alpha-1/13 |
| 34 | 98 | IL15_HUMAN | 3 | -0.94 | 0.01585528 | P40933 | IL15 | Interleukin-15 |
| | 99 | Isoform IL15-S21AA of IL15_HUMAN | | | | P40933-2 | | |
| 35 | 100 | IL3RB_HUMAN | 3 | -0.80 | 0.01834127 | P32927 | CSF2RB | Cytokine receptor common subunit beta |
| | 101 | Isoform 2 of IL3RB_HUMAN | | | | P32927-2 | | |
| 36 | 102 | K2C8_HUMAN | 3 | 0.97 | 0.03749303 | P05787 | KRT8 | Keratin, type II cytoskeletal 8 |
| | 103 | Isoform 2 of K2C8_HUMAN | | | | P05787-2 | | |
| 37 | 104 | LEUK_HUMAN | 3 | -0.59 | 0.00095308 | P16150 | SPN | Leukosialin |
| 38 | 105 | MARK4_HUMAN | 3 | -0.79 | 0.00167421 | Q96L34 | MARK4 | MAP/microtubule affinity-regulating kinase 4 |
| | 106 | Isoform 2 of MARK4_HUMAN | | | | Q96L34-2 | | |
| 39 | 107 | MELPH_HUMAN | 3 | -0.76 | 0.03511896 | Q9BV36 | MLPH | Melanophilin |
| | 108 | Isoform 2 of MELPH_HUMAN | | | | Q9BV36-2 | | |
| | 109 | Isoform 3 of MELPH_HUMAN | | | | Q9BV36-3 | | |
| | 110 | Isoform 4 of MELPH_HUMAN | | | | Q9BV36-4 | | |
| | 111 | Isoform 5 of MELPH_HUMAN | | | | Q9BV36-5 | | |
| 40 | 112 | MMP1_HUMAN | 3 | -0.59 | 0.00208582 | P03956 | MMP1 | Interstitial collagenase |
| 41 | 113 | MMP7_HUMAN | 3 | -0.96 | 0.03511896 | P09237 | MMP7 | Matrilysin |
| 42 | 114 | PGH1_HUMAN | 3 | -0.81 | 0.00801786 | P23219 | PTGS1 | Prostaglandin G/H synthase 1 |
| | 115 | Isoform 2 of PGH1_HUMAN | | | | P23219-2 | | |
| | 116 | Isoform 3 of PGH1_HUMAN | | | | P23219-3 | | |
| | 117 | Isoform 4 of PGH1_HUMAN | | | | P23219-4 | | |

| No. | SEQ ID NO | Uniprot entry name | Qual score | logFC | adj.p-Val | Uniprot accession | Gene name | Protein name |
|---|---|---|---|---|---|---|---|---|
|  | 118 | Isoform 5 of PGH1_HUMAN |  |  |  | P23219-5 |  |  |
|  | 119 | Isoform 6 of PGH1_HUMAN |  |  |  | P23219-6 |  |  |
| 43 | 120 | PRTN3_HUMAN | 3 | -0.76 | 0.02563658 | P24158 | PRTN3 | Myeloblastin |
| 44 | 121 | RBM3_HUMAN | 3 | -0.88 | 0.00173864 | P98179 | RBM3 | RNA-binding protein 3 |
| 45 | 122 | SAMP_HUMAN | 3 | 1.60 | 0.04927846 | P02743 | APCS | Serum amyloid P-component |
| 46 | 123 | TSN16_HUMAN | 3 | -0.95 | 0.01860133 | Q9UKR8 | TSPAN16 | Tetraspanin-16 |
|  | 124 | Isoform 2 of TSN16_HUMAN |  |  |  | Q9UKR8-2 |  |  |
|  | 125 | Isoform 3 of TSN16_HUMAN |  |  |  | Q9UKR8-3 |  |  |
|  | 126 | Isoform 4 of TSN16_HUMAN |  |  |  | Q9UKR8-4 |  |  |
| 47 | 127 | UROK_HUMAN | 3 | 1.05 | 0.01289580 | P00749 | PLAU | Urokinase-type plasminogen activator |
|  | 128 | Isoform 2 of UROK_HUMAN |  |  |  | P00749-2 |  |  |
| 48 | 135 | ACTB_HUMAN | 2 | -0.66 | 0.01989421 | P60709 | ACTB | Actin, cytoplasmic 1 |
| 49 | 136 | CASP3_HUMAN | 2 | -0.77 | 0.01672936 | P42574 | CASP3 | Caspase-3 |
| 50 | 137 | DAF_HUMAN | 2 | 0.51 | 0.02261542 | P08174 | CD55 | Complement decay-accelerating factor |
|  | 138 | Isoform 1 of DAF_HUMAN |  |  |  | P08174-2 |  |  |
|  | 139 | Isoform 3 of DAF_HUMAN |  |  |  | P08174-3 |  |  |
|  | 140 | Isoform 4 of DAF_HUMAN |  |  |  | P08174-4 |  |  |
|  | 141 | Isoform 5 of DAF_HUMAN |  |  |  | P08174-5 |  |  |
|  | 142 | Isoform 6 of DAF_HUMAN |  |  |  | P08174-6 |  |  |
|  | 143 | Isoform 7 of DAF_HUMAN |  |  |  | P08174-7 |  |  |
| 51 | 144 | HMGB2_HUMAN | 2 | 0.72 | 0.03511896 | P26583 | HMGB2 | High mobility group protein B2 |
| 52 | 145 | HXC11_HUMAN | 2 | 0.91 | 0.03511896 | O43248 | HOXC11 | Homeobox protein Hox-C11 |
| 53 | 146 | ICAM1_HUMAN | 2 | 0.71 | 0.02564186 | P05362 | ICAM1 | Intercellular adhesion molecule 1 |
| 54 | 147 | IL12A_HUMAN | 2 | -0.71 | 0.03511896 | P29459 | IL12A | Interleukin-12 subunit alpha |

| No. | SEQ ID NO | Uniprot entry name | Qual score | logFC | adj.p-Val | Uniprot accession | Gene name | Protein name |
|---|---|---|---|---|---|---|---|---|
| 55 | 194 | IL8_HUMAN | 2 | -0.83 | 0.03511896 | P10145 | CXCL8 | Interleukin-8 |
| 56 | 148 | KLF8_HUMAN | 2 | -0.67 | 0.01886910 | O95600 | KLF8 | Krueppel-like factor 8 |
| | 149 | Isoform 2 of KLF8_HUMAN | | | | O95600-3 | | |
| | 150 | Isoform 3 of KLF8_HUMAN | | | | O95600-4 | | |
| | 151 | Isoform 4 of KLF8_HUMAN | | | | O95600-5 | | |
| 57 | 152 | LEG4_HUMAN | 2 | -0.80 | 0.03511896 | P56470 | LGALS4 | Galectin-4 |
| 58 | 153 | LTOR1_HUMAN | 2 | 0.83 | 0.04600786 | Q6IAA8 | LAMTOR1 | Ragulator complex protein LAMTOR1 |
| 59 | 154 | LYAM1_HUMAN | 2 | -0.62 | 0.03226915 | P14151 | SELL | L-selectin |
| | 155 | Isoform 2 of LYAM1_HUMAN | | | | P14151-2 | | |
| 60 | 156 | MK03_HUMAN | 2 | -0.77 | 0.01886910 | P27361 | MAPK3 | Mitogen-activated protein kinase 3 |
| | 157 | Isoform 2 of MK03_HUMAN | | | | P27361-2 | | |
| | 158 | Isoform 3 of MK03_HUMAN | | | | P27361-3 | | |
| 61 | 159 | MUC5B_HUMAN | 2 | -0.80 | 0.02047452 | Q9HC84 | MUC5B | Mucin-5B |
| 62 | 160 | NFAC4_HUMAN | 2 | -0.50 | 0.01281061 | Q14934 | NFATC4 | Nuclear factor of activated T-cells, cytoplasmic 4 |
| | 161 | Isoform 2 of NFAC4_HUMAN | | | | Q14934-2 | | |
| | 162 | Isoform 3 of NFAC4_HUMAN | | | | Q14934-3 | | |
| | 163 | Isoform 4 of NFAC4_HUMAN | | | | Q14934-4 | | |
| | 164 | Isoform 5 of NFAC4_HUMAN | | | | Q14934-5 | | |
| | 165 | Isoform 6 of NFAC4_HUMAN | | | | Q14934-6 | | |
| | 166 | Isoform 7 of NFAC4_HUMAN | | | | Q14934-7 | | |
| | 167 | Isoform 8 of NFAC4_HUMAN | | | | Q14934-8 | | |
| | 168 | Isoform 9 of NFAC4_HUMAN | | | | Q14934-9 | | |
| | 169 | Isoform 10 of NFAC4_HUMAN | | | | Q14934-10 | | |
| | 170 | Isoform 11 of NFAC4_HUMAN | | | | Q14934-11 | | |

(continued)

| No. | SEQ ID NO | Uniprot entry name | Qual score | logFC | adj.p-Val | Uniprot accession | Gene name | Protein name |
|---|---|---|---|---|---|---|---|---|
| | 171 | Isoform 12 of NFAC4_HUMAN | | | | Q14934-12 | | |
| | 172 | Isoform 13 of NFAC4_HUMAN | | | | Q14934-13 | | |
| | 173 | Isoform 14 of NFAC4_HUMAN | | | | Q14934-14 | | |
| | 174 | Isoform 15 of NFAC4_HUMAN | | | | Q14934-15 | | |
| | 175 | Isoform 16 of NFAC4_HUMAN | | | | Q14934-16 | | |
| | 176 | Isoform 17 of NFAC4_HUMAN | | | | Q14934-17 | | |
| | 177 | Isoform 18 of NFAC4_HUMAN | | | | Q14934-18 | | |
| | 178 | Isoform 19 of NFAC4_HUMAN | | | | Q14934-19 | | |
| | 179 | Isoform 20 of NFAC4_HUMAN | | | | Q14934-20 | | |
| | 180 | Isoform 21 of NFAC4_HUMAN | | | | Q14934-21 | | |
| | 181 | Isoform 22 of NFAC4_HUMAN | | | | Q14934-22 | | |
| | 182 | Isoform 23 of NFAC4_HUMAN | | | | Q14934-23 | | |
| | 183 | Isoform 24 of NFAC4_HUMAN | | | | Q14934-24 | | |
| 63 | 184 | TGFB1_HUMAN | 2 | 0.81 | 0.02912596 | P01137 | TGFB1 | Transforming growth factor beta-1 proprotein |
| 64 | 185 | VRK1_HUMAN | 2 | -0.87 | 0.04772231 | Q99986 | VRK1 | Serine/threonine-protein kinase VRK1 |
| 65 | 195 | ARHG2_HUMAN | 1 | -0.41 | 0.03565782 | Q92974 | ARHGEF2 | Rho guanine nucleotide exchange factor |
| | 196 | Isoform 2 of ARHG2_HUMAN | | | | Q92974-2 | | |
| | 197 | Isoform 3 of ARHG2_HUMAN | | | | Q92974-3 | | |
| 66 | 198 | CFLAR_HUMAN | 1 | 0.52 | 0.03006471 | O15519 | CFLAR | CASP8 and FADD-like apoptosis regulator |
| | 199 | Isoform 2 of CFLAR_HUMAN | | | | O15519-2 | | |
| | 200 | Isoform 3 of CFLAR_HUMAN | | | | O15519-3 | | |
| | 201 | Isoform 4 of CFLAR_HUMAN | | | | O15519-4 | | |
| | 202 | Isoform 5 of CFLAR_HUMAN | | | | O15519-5 | | |
| | 203 | Isoform 6 of CFLAR_HUMAN | | | | O15519-6 | | |

| No. | SEQ ID NO | Uniprot entry name | Qual score | logFC | adj.p-Val | Uniprot accession | Gene name | Protein name |
|---|---|---|---|---|---|---|---|---|
|  | 204 | Isoform 7 of CFLAR_HUMAN |  |  |  | O15519-7 |  |  |
|  | 205 | Isoform 8 of CFLAR_HUMAN |  |  |  | O15519-8 |  |  |
|  | 206 | Isoform 9 of CFLAR_HUMAN |  |  |  | O15519-9 |  |  |
|  | 207 | Isoform 10 of CFLAR_HUMAN |  |  |  | O15519-10 |  |  |
|  | 208 | Isoform 11 of CFLAR_HUMAN |  |  |  | O15519-11 |  |  |
|  | 209 | Isoform 12 of CFLAR_HUMAN |  |  |  | O15519-12 |  |  |
|  | 210 | Isoform 13 of CFLAR_HUMAN |  |  |  | O15519-13 |  |  |
|  | 211 | Isoform 14 of CFLAR_HUMAN |  |  |  | O15519-14 |  |  |
|  | 212 | Isoform 15 of CFLAR_HUMAN |  |  |  | O15519-15 |  |  |
| 67 | 213 | CUED2_HUMAN | 1 | -0.70 | 0.01007946 | Q9H467 | CUEDC2 | CUE domain-containing protein 2 |
| 68 | 214 | DAPK1_HUMAN | 1 | -0.65 | 0.02261542 | P53355 | DAPK1 | Death-associated protein kinase 1 |
|  | 215 | Isoform 2 of DAPK1_HUMAN |  |  |  | P53355-2 |  |  |
|  | 216 | Isoform 3 of DAPK1_HUMAN |  |  |  | P53355-3 |  |  |
|  | 217 | Isoform 4 of DAPK1_HUMAN |  |  |  | P53355-4 |  |  |
| 69 | 218 | EDNRA_HUMAN | 1 | 0.58 | 0.02900873 | P25101 | EDNRA | Endothelin-1 receptor |
|  | 219 | Isoform 2 of EDNRA_HUMAN |  |  |  | P25101-2 |  |  |
|  | 220 | Isoform 3 of EDNRA_HUMAN |  |  |  | P25101-3 |  |  |
|  | 221 | Isoform 4 of EDNRA_HUMAN |  |  |  | P25101-4 |  |  |
|  | 222 | Isoform 5 of EDNRA_HUMAN |  |  |  | P25101-5 |  |  |
| 70 | 223 | EIF3B_HUMAN | 1 | 0.69 | 0.01829644 | P55884 | EIF3B | Eukaryotic translation initiation factor 3 subunit |
|  | 224 | Isoform 2 of EIF3B_HUMAN |  |  |  | P55884-2 |  |  |
| 71 | 225 | ID2_HUMAN | 1 | 0.32 | 0.03707986 | Q02363 | ID2 | DNA-binding protein inhibitor ID-2 |
| 72 | 226 | LMNA_HUMAN | 1 | -0.63 | 0.03353007 | P02545 | LMNA | Prelamin-A/C |
|  | 227 | Isoform C of LMNA_HUMAN |  |  |  | P02545-2 |  |  |

(continued)

| No. | SEQ ID NO | Uniprot entry name | Qual score | logFC | adj.p-Val | Uniprot accession | Gene name | Protein name |
|---|---|---|---|---|---|---|---|---|
| | 228 | Isoform ADelta10 of LMNA_HUMAN | | | | P02545-3 | | |
| | 229 | Isoform 4 of LMNA_HUMAN | | | | P02545-4 | | |
| | 230 | Isoform 5 of LMNA_HUMAN | | | | P02545-5 | | |
| | 231 | Isoform 6 of LMNA_HUMAN | | | | P02545-6 | | |
| 73 | 232 | PYR1_HUMAN | 1 | -0.65 | 0.01284425 | P27708 | CAD | CAD protein |
| 74 | 233 | ZN593_HUMAN | 1 | 0.62 | 0.01284425 | O00488 | ZNF593 | Zinc finger protein 593 |
| 75 | 237 | ANGT_HUMAN | 0 | -0.42 | 0.04109223 | P01019 | AGT | Angiotensinogen |
| 76 | 238 | APC_HUMAN | 0 | -0.42 | 0.02825626 | P25054 | APC | Adenomatous polyposis coli protein |
| | 239 | Isoform 2 of APC_HUMAN | | | | P25054-2 | | |
| | 240 | Isoform 1B of APC_HUMAN | | | | P25054-3 | | |
| 77 | 241 | P02F1_HUMAN | 0 | 0.31 | 0.02825626 | P14859 | POU2F1 | POU domain, class 2, transcription factor 1 |
| | 242 | Isoform 2 of P02F1_HUMAN | | | | P14859-2 | | |
| | 243 | Isoform 3 of P02F1_HUMAN | | | | P14859-3 | | |
| | 244 | Isoform 6 of P02F1_HUMAN | | | | P14859-6 | | |
| | 245 | Isoform 4 of P02F1_HUMAN | | | | P14859-4 | | |
| | 246 | Isoform 5 of P02F1_HUMAN | | | | P14859-5 | | |
| 78 | 247 | SSR4_HUMAN | 0 | -0.40 | 0.02825626 | P31391 | SSTR4 | Somatostatin receptor type 4 |
| 79 | 248 | TNAP3_HUMAN | 0 | -0.49 | 0.03707986 | P21580 | TNFAIP3 | Tumor necrosis factor alpha-induced protein 3 |

Table 3 shows the 26 diagnostic biomarkers of the present invention.

*Table 3*

| No. | SEQ ID No. | Uniprot entry name | Qual score | logFC | adj.p-Val | Uniprot accession | Gene name | Protein name |
|---|---|---|---|---|---|---|---|---|
| **1** | 1 | PGH2_HUMAN | 7 | -3.49 | 0.00000080 | P35354 | PTGS2 | Prostaglandin G/H synthase 2 |
| **2** | 13 | IFNA1_HUMAN | 6 | 1.09 | 0.00041027 | P01562 | IFNA1; IFNA13 | Interferon alpha-1/13 |
| **3** | 80 | CASP9_HUMAN | 4 | 1.26 | 0.01917485 | P55211 | CASP9 | Caspase-9 |
| | 81 | Isoform 2 of CASP9_HUMAN | | | | P55211-2 | | |
| | 82 | Isoform 3 of CASP9_HUMAN | | | | P55211-3 | | |
| | 83 | Isoform 4 of CASP9_HUMAN | | | | P55211-4 | | |
| **4** | 25 | IL18_HUMAN | 4 | 1.00 | 0.01705270 | Q14116 | IL18 | Interleukin-18 |
| | 26 | Isoform 2 of IL18_HUMAN | | | | Q14116-2 | | |
| **5** | 27 | IL7_HUMAN | 4 | 1.02 | 0.01917485 | P13232 | IL7 | Interleukin-7 |
| | 28 | Isoform 2 of IL7_HUMAN | | | | P13232-2 | | |
| | 29 | Isoform 3 of IL7_HUMAN | | | | P13232-3 | | |
| **6** | 129 | PYRG1_HUMAN | 3 | 1.03 | 0.04367345 | P17812 | CTPS1 | CTP synthase 1 |
| | 130 | Isoform 2 of PYRG1_HUMAN | | | | P17812-2 | | |
| **7** | 18 | CCL7_HUMAN | 3 | 0.74 | 0.04367345 | P80098 | CCL7 | C-C motif chemokine 7 |
| **8** | no ID | CD139 (no entry, no protein) | 3 | -1.22 | 0.03468974 | no entry | | |
| **9** | 61 | CCL11_HUMAN | 3 | 1.15 | 0.01633199 | P51671 | CCL11 | Eotaxin |
| **10** | 65 | CRLF2_HUMAN | 3 | 1.01 | 0.01848700 | Q9HC73 | CRLF2 | Cytokine receptor-like factor 2 |

| No. | SEQ ID No. | Uniprot entry name | Qual score | logFC | adj.p-Val | Uniprot accession | Gene name | Protein name |
|---|---|---|---|---|---|---|---|---|
| | 66 | Isoform 2 of CRLF2_HUMAN | | | | Q9HC73-2 | | |
| | 67 | Isoform 3 of CRLF2_HUMAN | | | | Q9HC73-3 | | |
| 11 | 131 | MAD4_HUMAN | 3 | 1.09 | 0.01633199 | Q14582 | MXD4 | Max dimerization protein 4 |
| 12 | 98 | IL15_HUMAN | 3 | 1.08 | 0.01242697 | P40933 | IL15 | Interleukin-15 |
| | 99 | Isoform IL15-S21AA of IL15_HUMAN | | | | P40933-2 | | |
| 13 | 121 | RBM3_HUMAN | 3 | 0.96 | 0.01754186 | P98179 | RBM3 | RNA-binding protein 3 |
| 14 | 132 | TMM54_HUMAN | 3 | 0.80 | 0.01242697 | Q969K7 | TMEM54 | Transmembrane protein 54 |
| | 133 | Isoform 2 of TMM54_HUMAN | | | | Q969K7-2 | | |
| | 134 | Isoform 3 of TMM54_HUMAN | | | | Q969K7-3 | | |
| 15 | 148 | KLF8_HUMAN | 2 | -0.93 | 0.04479542 | O95600 | KLF8 | Krueppel-like factor 8 |
| | 149 | Isoform 2 of KLF8_HUMAN | | | | O95600-3 | | |
| | 150 | Isoform 3 of KLF8_HUMAN | | | | O95600-4 | | |
| | 151 | Isoform 4 of KLF8_HUMAN | | | | O95600-5 | | |
| 16 | 112 | MMP1_HUMAN | 2 | 0.71 | 0.01633199 | P03956 | MMP1 | Interstitial collagenase |
| 17 | 186 | CDKN3_HUMAN | 2 | -0.71 | 0.01978279 | Q16667 | CDKN3 | Cyclin-dependent kinase inhibitor 3 |
| | 187 | Isoform 2 of CDKN3_HUMAN | | | | Q16667-2 | | |
| 18 | 188 | TFPI2_HUMAN | 2 | -0.79 | 0.03075045 | P48307 | TFPI2 | Tissue factor pathway inhibitor 2 |

| No. | SEQ ID No. | Uniprot entry name | Qual score | logFC | adj.p-Val | Uniprot accession | Gene name | Protein name |
|---|---|---|---|---|---|---|---|---|
| | 189 | Isoform 2 of TFPI2_HUMAN | | | | P48307-2 | | |
| **19** | 93 | GAS6_HUMAN | 2 | 0.79 | 0.01633199 | Q14393 | GAS6 | Growth arrest-specific protein 6 |
| | 94 | Isoform 2 of GAS6_HUMAN | | | | Q14393-1 | | |
| | 95 | Isoform 3 of GAS6_HUMAN | | | | Q14393-3 | | |
| | 96 | Isoform 4 of GAS6_HUMAN | | | | Q14393-4 | | |
| | 97 | Isoform 5 of GAS6_HUMAN | | | | Q14393-5 | | |
| **20** | 190 | MLP3B_HUMAN | 2 | 0.99 | 0.01633199 | Q9GZQ8 | MAP1LC3B | Microtubule-associated proteins 1A/1B light chain 3B |
| **21** | 52 | CASP8_HUMAN | 2 | 0.89 | 0.04367345 | Q14790 | CASP8 | Caspase-8 |
| | 53 | Isoform 2 of CASP8_HUMAN | | | | Q14790-2 | | |
| | 54 | Isoform 3 of CASP8_HUMAN | | | | Q14790-3 | | |
| | 55 | Isoform 4 of CASP8_HUMAN | | | | Q14790-4 | | |
| | 56 | Isoform 5 of CASP8_HUMAN | | | | Q14790-5 | | |
| | 57 | Isoform 6 of CASP8_HUMAN | | | | Q14790-6 | | |
| | 58 | Isoform 7 of CASP8_HUMAN | | | | Q14790-7 | | |
| | 59 | Isoform 8 of CASP8_HUMAN | | | | Q14790-8 | | |

(continued)

| No. | SEQ ID No. | Uniprot entry name | Qual score | logFC | adj.p-Val | Uniprot accession | Gene name | Protein name |
|---|---|---|---|---|---|---|---|---|
| | 60 | Isoform 9 of CASP8_HUMAN | | | | Q14790-9 | | |
| 22 | 191 | PTEN_HUMAN | 2 | 0.93 | 0.01633199 | P60484 | PTEN | Phosphatidylinositol 3,4,5-trisphosphate 3-phosphatase and dual-specificity protein phosphatase PTEN |
| | 192 | Isoform alpha of PTEN_HUMAN | | | | P60484-2 | | |
| | 193 | Isoform 3 of PTEN_HUMAN | | | | P60484-3 | | |
| 23 | 234 | MK12_HUMAN | 1 | 0.69 | 0.04367345 | P53778 | MAPK12 | Mitogen-activated protein kinase 12 |
| | 235 | Isoform 2 of MK12_HUMAN | | | | P53778-2 | | |
| 24 | 236 | CP1B1_HUMAN | 1 | -0.56 | 0.01978279 | Q16678 | CYP1B1 | Cytochrome P450 1B1 |
| 25 | 249 | TRI22_HUMAN | 0 | 0.44 | 0.04624814 | Q8IYM9 | TRIM22 | E3 ubiquitin-protein ligase TRIM22 |
| | 250 | Isoform 2 of TRI22_HUMAN | | | | Q8IYM9-2 | | |

12 biomarkers have been found to be both predictive and diagnostic biomarkers. Therefore, the 78 predictive biomarkers and the 26 diagnostic biomarkers sum up to 92 biomarkers in total.

The 12 biomarkers being both predictive and diagnostic are shown in Table 4.

*Table 4*

| No. | SEQ ID No. | Uniprot entry name | Pre / diag | Qual score | logFC | adj.p-Val | Uniprot accession | Gene name | Protein name |
|---|---|---|---|---|---|---|---|---|---|
| **1** | 52 | CASP8_HUMAN | pre | 4 | -0.98 | 0.00743181 | Q14790 | CASP8 | Caspase-8 |
| | | | diag | 2 | 0.89 | 0.04367345 | Q14790 | | |
| | 53 | Isoform 2 of CASP8_HUMAN | | | | | Q14790-2 | | |
| | 54 | Isoform 3 of CASP8_HUMAN | | | | | Q14790-3 | | |
| | 55 | Isoform 4 of CASP8_HUMAN | | | | | Q14790-4 | | |
| | 56 | Isoform 5 of CASP8_HUMAN | | | | | Q14790-5 | | |
| | 57 | Isoform 6 of CASP8_HUMAN | | | | | Q14790-6 | | |
| | 58 | Isoform 7 of CASP8_HUMAN | | | | | Q14790-7 | | |
| | 59 | Isoform 8 of CASP8_HUMAN | | | | | Q14790-8 | | |
| | 60 | Isoform 9 of CASP8_HUMAN | | | | | Q14790-9 | | |
| **2** | 61 | CCL11_HUMAN | pre | 4 | -0.92 | 0.00743181 | P51671 | CCL11 | Eotaxin |
| | | | diag | 3 | 1.15 | 0.01633199 | P51671 | | |
| **3** | 18 | CCL7_HUMAN | pre | 5 | -0.82 | 0.00082227 | P80098 | CCL7 | C-C motif chemokine 7 |
| | | | diag | 3 | 0.74 | 0.04367345 | P80098 | | |
| **4** | 65 | CRLF2_HUMAN | pre | 4 | -1.08 | 0.00866136 | Q9HC73 | CRLF2 | Cytokine receptor-like factor 2 |
| | | | diag | 3 | 1.01 | 0.01848700 | Q9HC73 | | |
| | 66 | Isoform 2 of CRLF2_HUMAN | | | | | Q9HC73-2 | | |
| | 67 | Isoform 3 of CRLF2_HUMAN | | | | | Q9HC73-3 | | |
| **5** | 93 | GAS6_HUMAN | pre | 3 | -0.83 | 0.01370348 | Q14393 | GAS6 | Growth arrest-specific protein 6 |
| | | | diag | 2 | 0.79 | 0.01633199 | Q14393 | | |
| | 94 | Isoform 2 of GAS6_HUMAN | | | | | Q14393-1 | | |
| | 95 | Isoform 3 of GAS6_HUMAN | | | | | Q14393-3 | | |
| | 96 | Isoform 4 of GAS6_HUMAN | | | | | Q14393-4 | | |

| No. | SEQ ID No. | Uniprot entry name | Pre / diag | Qual score | logFC | adj.p-Val | Uniprot accession | Gene name | Protein name |
|---|---|---|---|---|---|---|---|---|---|
| | 97 | Isoform 5 of GAS6_HUMAN | | | | | Q14393-5 | | |
| 6 | 13 | IFNA1_HUMAN | pre | 3 | -0.99 | 0.03511896 | P01562 | IFNA1; IFNA13 | Interferon alpha-1/13 |
| | | | diag | 6 | 1.09 | 0.00041027 | P01562 | | |
| 7 | 98 | IL15_HUMAN | pre | 3 | -0.94 | 0.01585528 | P40933 | IL15 | Interleukin-15 |
| | | | diag | 3 | 1.08 | 0.01242697 | P40933 | | |
| | 99 | Isoform IL15-S21AA of IL15_HUMAN | | | | | P40933-2 | | |
| 8 | 25 | IL18_HUMAN | pre | 5 | -1.03 | 0.00709074 | Q14116 | IL18 | Interleukin-18 |
| | | | diag | 4 | 1.00 | 0.01705270 | Q14116 | | |
| | 26 | Isoform 2 of IL18_HUMAN | | | | | Q14116-2 | | |
| 9 | 27 | IL7_HUMAN | pre | 5 | -0.98 | 0.00077460 | P13232 | IL7 | |
| | | | diag | 4 | 1.02 | 0.01917485 | P13232 | | Interleukin-7 |
| | 28 | Isoform 2 of IL7_HUMAN | | | | | P13232-2 | | |
| | 29 | Isoform 3 of IL7_HUMAN | | | | | P13232-3 | | |
| 10 | 148 | KLF8_HUMAN | pre | 2 | -0.67 | 0.01886910 | 095600 | KLF8 | Krueppel-like factor 8 |
| | | | diag | 2 | -0.93 | 0.04479542 | 095600 | | |
| | 149 | Isoform 2 of KLF8_HUMAN | | | | | 095600-3 | | |
| | 150 | Isoform 3 of KLF8_HUMAN | | | | | 095600-4 | | |
| | 151 | Isoform 4 of KLF8_HUMAN | | | | | 095600-5 | | |
| 11 | 112 | MMP1_HUMAN | pre | 3 | -0.59 | 0.00208582 | P03956 | MMP1 | Interstitial collagenase |
| | | | diag | 2 | 0.71 | 0.01633199 | P03956 | | |
| 12 | 121 | RBM3_HUMAN | pre | 3 | -0.88 | 0.00173864 | P98179 | RBM3 | RNA-binding protein 3 |
| | | | diag | 3 | 0.96 | 0.01754186 | P98179 | | |

The 35 biomarkers showing higher abundance in AKI patients are shown in Table 5.

*Table 5*

| No. | SEQ ID No. | Uniprot entry name | Pre / diag | Qual score | logFC | adj.p-Val | Uniprot accession | Gene name | Protein name |
|---|---|---|---|---|---|---|---|---|---|
| 1 | 13 | IFNA1_HUMAN | diag | 6 | 1.09 | 0.00041027 | P01562 | IFNA1; IFNA13 | Interferon alpha-1/13 |
| 2 | 69-77 | P53_HUMAN | pre | 4 | 0.81 | 0.00173864 | P04637 | TP53 | Cellular tumor antigen p53 |
| 3 | 80-83 | CASP9_HUMAN | diag | 4 | 1.26 | 0.01917485 | P55211 | CASP9 | Caspase-9 |
| 4 | 27-29 | IL7_HUMAN | diag | 4 | 1.02 | 0.01917485 | P13232 | IL7 | Interleukin-7 |
| 5 | 25-26 | IL18_HUMAN | diag | 4 | 1.00 | 0.01705270 | Q14116 | IL18 | Interleukin-18 |
| 6 | 122 | SAMP_HUMAN | pre | 3 | 1.60 | 0.04927846 | P02743 | APCS | Serum amyloid P-component |
| 7 | 127-128 | UROK_HUMAN | pre | 3 | 1.05 | 0.01289580 | P00749 | PLAU | Urokinase-type plasminogen activator |
| 8 | 102-103 | K2C8_HUMAN | pre | 3 | 0.97 | 0.03749303 | P05787 | KRT8 | Keratin, type II cytoskeletal 8 |
| 9 | 61 | CCL11_HUMAN | diag | 3 | 1.15 | 0.01633199 | P51671 | CCL11 | Eotaxin |
| 10 | 131 | MAD4_HUMAN | diag | 3 | 1.09 | 0.01633199 | Q14582 | MXD4 | Max dimerization protein 4 |
| 11 | 98-99 | IL15_HUMAN | diag | 3 | 1.08 | 0.01242697 | P40933 | IL15 | Interleukin-15 |
| 12 | 129-130 | PYRG1_HUMAN | diag | 3 | 1.03 | 0.04367345 | P17812 | CTPS1 | CTP synthase 1 |
| 13 | 65-67 | CRLF2_HUMAN | diag | 3 | 1.01 | 0.01848700 | Q9HC73 | CRLF2 | Cytokine receptor-like factor 2 |
| 14 | 121 | RBM3_HUMAN | diag | 3 | 0.96 | 0.01754186 | P98179 | RBM3 | RNA-binding protein 3 |
| 15 | 132-134 | TMM54_HUMAN | diag | 3 | 0.80 | 0.01242697 | Q969K7 | TMEM54 | Transmembrane protein 54 |
| 16 | 18 | CCL7_HUMAN | diag | 3 | 0.74 | 0.04367345 | P80098 | CCL7 | C-C motif chemokine 7 |
| 17 | 145 | HXC11_HUMAN | pre | 2 | 0.91 | 0.03511896 | 043248 | HOXC11 | Homeobox protein Hox-C11 |
| 18 | 153 | LTOR1_HUMAN | pre | 2 | 0.83 | 0.04600786 | Q6IAA8 | LAMTOR1 | Ragulator complex protein LAMTOR1 |
| 19 | 184 | TGFB1_HUMAN | pre | 2 | 0.81 | 0.02912596 | P01137 | TGFB1 | Transforming growth factor beta-1 proprotein |
| 20 | 144 | HMGB2_HUMAN | pre | 2 | 0.72 | 0.03511896 | P26583 | HMGB2 | High mobility group protein B2 |
| 21 | 146 | ICAM1_HUMAN | pre | 2 | 0.71 | 0.02564186 | P05362 | ICAM1 | Intercellular adhesion molecule 1 |
| 22 | 137-143 | DAF_HUMAN | pre | 2 | 0.51 | 0.02261542 | P08174 | CD55 | Complement decay-accelerating factor |
| 23 | 190 | MLP3B_HUMAN | diag | 2 | 0.99 | 0.01633199 | Q9GZQ8 | MAP1LC3B | Microtubule-associated proteins 1A/1B light chain 3B |

| No. | SEQ ID No. | Uniprot entry name | Pre / diag | Qual score | logFC | adj.p-Val | Uniprot accession | Gene name | Protein name |
|---|---|---|---|---|---|---|---|---|---|
| 24 | 191-193 | PTEN_HUMAN | diag | 2 | 0.93 | 0.01633199 | P60484 | PTEN | Phosphatidylinositol 3,4,5-trisphosphate 3-phosphatase and dual-specificity protein phosphatase PTEN |
| 25 | 52-60 | CASP8_HUMAN | diag | 2 | 0.89 | 0.04367345 | Q14790 | CASP8 | Caspase-8 |
| 26 | 93-97 | GAS6_HUMAN | diag | 2 | 0.79 | 0.01633199 | Q14393 | GAS6 | Growth arrestspecific protein 6 |
| 27 | 112 | MMP1_HUMAN | diag | 2 | 0.71 | 0.01633199 | P03956 | MMP1 | Interstitial collagenase |
| 28 | 223-224 | EIF3B_HUMAN | pre | 1 | 0.69 | 0.01829644 | P55884 | EIF3B | Eukaryotic translation initiation factor 3 subunit B |
| 29 | 233 | ZN593_HUMAN | pre | 1 | 0.62 | 0.01284425 | 000488 | ZNF593 | Zinc finger protein 593 |
| 30 | 218-222 | EDNRA_HUMAN | pre | 1 | 0.58 | 0.02900873 | P25101 | EDNRA | Endothelin-1 receptor |
| 31 | 198-212 | CFLAR_HUMAN | pre | 1 | 0.52 | 0.03006471 | 015519 | CFLAR | CASP8 and FADD-like apoptosis regulator |
| 32 | 225 | ID2_HUMAN | pre | 1 | 0.32 | 0.03707986 | Q02363 | ID2 | DNA-binding protein inhibitor ID-2 |
| 33 | 234-235 | MK12_HUMAN | diag | 1 | 0.69 | 0.04367345 | P53778 | MAPK12 | Mitogen-activated protein kinase 12 |
| 34 | 241-246 | P02F1_HUMAN | pre | 0 | 0.31 | 0.02825626 | P14859 | POU2F1 | POU domain, class 2, transcription factor 1 |
| 35 | 249-250 | TRI22_HUMAN | diag | 0 | 0.44 | 0.04624814 | Q8IYM9 | TRIM22 | E3 ubiquitin-protein ligase TRIM22 |

68 biomarkers showing lower abundance in AKI patients are shown in Table 6.

*Table 6*

| No. | SEQ ID No. | Uniprot entry name | Pre / diag | Qual score | logFC | adj.p-Val | Uniprot accession | Gene name | Protein name |
|---|---|---|---|---|---|---|---|---|---|
| 1 | 1 | PGH2_HUMAN | diag | 7 | -3.49 | 0.00000080 | P35354 | PTGS2 | Prostaglandin G/H synthase 2 |
| 2 | no ID | CD15 (no entry, no protein) | pre | 6 | -0.86 | 0.00000004 | no entry | CD15 | CD15 |
| 3 | 2-4 | CD99_HUMAN | pre | 6 | -0.98 | 0.00000440 | P14209 | CD99 | CD99 antigen |
| 4 | 6-12 | TNR6_HUMAN | pre | 6 | -1.32 | 0.00003060 | P25445 | FAS | Tumor necrosis factor receptor superfamily member 6 |
| 5 | 251-252 | No entry (Ligands of TNR1B_HUMAN including TNFA_HUMAN and TNFB_HUMAN) | pre | 6 | -1.60 | 0.00000083 | | | Ligands of TNR1B including TNF-alpha and lymphotoxin-alpha |
| 6 | 5 | FCERA_HUMAN | pre | 6 | -1.63 | 0.00049891 | P12319 | FCER1A | High affinity immunoglobulin epsilon receptor subunit alpha |
| 7 | 19 | CD9_HUMAN | pre | 5 | -0.78 | 0.00077460 | P21926 | CD9 | CD9 antigen |
| 8 | 20 | DKK2_HUMAN | pre | 5 | -0.78 | 0.00077460 | Q9UBU2 | DKK2 | Dickkopf-related protein 2 |
| 9 | 18 | CCL7_HUMAN | pre | 5 | -0.82 | 0.00082227 | P80098 | CCL7 | C-C motif chemokine 7 |
| 10 | 43-46 | TOP2A_HUMAN | pre | 5 | -0.88 | 0.00077460 | P11388 | TOP2A | DNA topoisomerase 2-alpha |
| 11 | 31-38 | PTPRC_HUMAN | pre | 5 | -0.93 | 0.00021371 | P08575 | PTPRC | Receptor-type tyrosine-protein phosphatase C |
| 12 | 30 | PRIO_HUMAN | pre | 5 | -0.95 | 0.00077460 | P04156 | PRNP | Major prion protein |
| 13 | 39-40 | SELPL_HUMAN | pre | 5 | -0.96 | 0.00020190 | Q14242 | SELPLG | P-selectin glycoprotein ligand 1 |
| 14 | 27-29 | IL7_HUMAN | pre | 5 | -0.98 | 0.00077460 | P13232 | IL7 | Interleukin-7 |
| 15 | 14-17 | BASI_HUMAN | pre | 5 | -1.00 | 0.00456896 | P35613 | BSG | Basigin |
| 16 | 21-24 | HMMR_HUMAN | pre | 5 | -1.02 | 0.00743181 | O75330 | HMMR | Hyaluronan mediated motility receptor |
| 17 | 25-26 | IL18_HUMAN | pre | 5 | -1.03 | 0.00709074 | Q14116 | IL18 | Interleukin-18 |
| 18 | 41-42 | TNF14_HUMAN | pre | 5 | -1.23 | 0.00896522 | O43557 | TNFSF14 | Tumor necrosis factor ligand superfamily member 14 |
| 19 | 78-79 | PAK1_HUMAN | pre | 4 | -0.74 | 0.00901111 | Q13153 | PAK1 | Serine/threonine-protein kinase PAK 1 |

| No. | SEQ ID No. | Uniprot entry name | Pre / diag | Qual score | logFC | adj.p-Val | Uniprot accession | Gene name | Protein name |
|---|---|---|---|---|---|---|---|---|---|
| 20 | 68 | LMNB1_HUMAN | pre | 4 | -0.78 | 0.00041269 | P20700 | LMNB1 | Lamin-Bl |
| 21 | 64 | CD14_HUMAN | pre | 4 | -0.87 | 0.00975827 | P08571 | CD14 | Monocyte differentiation antigen CD14 |
| 22 | 61 | CCL11_HUMAN | pre | 4 | -0.92 | 0.00743181 | P51671 | CCL11 | Eotaxin |
| 23 | 52-60 | CASP8_HUMAN | pre | 4 | -0.98 | 0.00743181 | Q14790 | CASP8 | Caspase-8 |
| 24 | 62 | CCL3_HUMAN | pre | 4 | -1.00 | 0.03040191 | P10147 | CCL3 | C-C motif chemokine 3 |
| 25 | 47-51 | BDNF_HUMAN | pre | 4 | -1.03 | 0.03226915 | P23560 | BDNF | Brain-derived neurotrophic factor |
| 26 | 63 | CCL5_HUMAN | pre | 4 | -1.07 | 0.00896522 | P13501 | CCL5 | C-C motif chemokine 5 |
| 27 | 65-67 | CRLF2_HUMAN | pre | 4 | -1.08 | 0.00866136 | Q9HC73 | CRLF2 | Cytokine receptor-like factor 2 |
| 28 | 85-88 | CD47_HUMAN | pre | 3 | -0.54 | 0.00118575 | Q08722 | CD47 | Leukocyte surface antigen CD47 |
| 29 | 104 | LEUK_HUMAN | pre | 3 | -0.59 | 0.00095308 | P16150 | SPN | Leukosialin |
| 30 | 112 | MMP1_HUMAN | pre | 3 | -0.59 | 0.00208582 | P03956 | MMP1 | Interstitial collagenase |
| 31 | 107-111 | MELPH_HUMAN | pre | 3 | -0.76 | 0.03511896 | Q9BV36 | MLPH | Melanophilin |
| 32 | 120 | PRTN3_HUMAN | pre | 3 | -0.76 | 0.02563658 | P24158 | PRTN3 | Myeloblastin |
| 33 | 92 | DKK3_HUMAN | pre | 3 | -0.77 | 0.03511896 | Q9UBP4 | DKK3 | Dickkopf-related protein 3 |
| 34 | 105-106 | MARK4_HUMAN | pre | 3 | -0.79 | 0.00167421 | Q96L34 | MARK4 | MAP/microtubule affinity-regulating kinase 4 |
| 35 | 84 | BGH3_HUMAN | pre | 3 | -0.79 | 0.00768649 | Q15582 | TGFBI BIGH3 | Transforming growth factor-beta-induced protein ig-h3 |
| 36 | 100-101 | IL3RB_HUMAN | pre | 3 | -0.80 | 0.01834127 | P32927 | CSF2RB | Cytokine receptor common subunit beta |
| 37 | 114-119 | PGH1_HUMAN | pre | 3 | -0.81 | 0.00801786 | P23219 | PTGS1 | Prostaglandin G/H synthase 1 |
| 38 | 93-97 | GAS6_HUMAN | pre | 3 | -0.83 | 0.01370348 | Q14393 | GAS6 | Growth arrest-specific protein 6 |
| 39 | 121 | RBM3_HUMAN | pre | 3 | -0.88 | 0.00173864 | P98179 | RBM3 | RNA-binding protein 3 |

| No. | SEQ ID No. | Uniprot entry name | Pre / diag | Qual score | logFC | adj.p-Val | Uniprot accession | Gene name | Protein name |
|---|---|---|---|---|---|---|---|---|---|
| 40 | 89-91 | CD8A_HUMAN | pre | 3 | -0.93 | 0.02217931 | P01732 | CD8A | T-cell surface glycoprotein CD8 alpha chain |
| 41 | 98 | IL15_HUMAN | pre | 3 | -0.94 | 0.01585528 | P40933 | IL15 | Interleukin-15 |
| 42 | 123-126 | TSN16_HUMAN | pre | 3 | -0.95 | 0.01860133 | Q9UKR8 | TSPAN16 | Tetraspanin-16 |
| 43 | 113 | MMP7_HUMAN | pre | 3 | -0.96 | 0.03511896 | P09237 | MMP7 | Matrilysin |
| No. | SEQ ID No. | Uniprot entry name | Pre/ diag | Qual score | logFC | adj.p-Val | Uniprot accession | Gene name | Protein name |
| 44 | 13 | IFNA1_HUMAN | pre | 3 | -0.99 | 0.03511896 | P01562 | IFNA1; IFNA13 | Interferon alpha-1/13 |
| 45 | no ID | CD139 (no entry, no protein) | diag | 3 | -1.22 | 0.03468974 | no entry | CD139 | CD139 |
| 46 | 160-183 | NFAC4_HUMAN | pre | 2 | -0.50 | 0.01281061 | Q14934 | NFATC4 | Nuclear factor of activated T-cells |
| 47 | 154-155 | LYAM1_HUMAN | pre | 2 | -0.62 | 0.03226915 | P14151 | SELL | L-selectin |
| 48 | 135 | ACTB_HUMAN | pre | 2 | -0.66 | 0.01989421 | P60709 | ACTB | Actin, cytoplasmic 1 |
| 49 | 148-151 | KLF8_HUMAN | pre | 2 | -0.67 | 0.01886910 | O95600 | KLF8 | Krueppel-like factor 8 |
|  |  |  | diag | 2 | -0.93 | 0.04479542 |  |  |  |
| 50 | 147 | IL12A_HUMAN | pre | 2 | -0.71 | 0.03511896 | P29459 | IL12A | Interleukin-12 subunit alpha |
| 51 | 194 | IL8_HUMAN | pre | 2 | -0.83 | 0.03511896 | P10145 | CXCL8 | Interleukin-8 |
| 52 | 156-158 | MK03_HUMAN | pre | 2 | -0.77 | 0.01886910 | P27361 | MAPK3 | Mitogen-activated protein kinase 3 |
| 53 | 136 | CASP3_HUMAN | pre | 2 | -0.77 | 0.01672936 | P42574 | CASP3 | Caspase-3 |
| 54 | 152 | LEG4_HUMAN | pre | 2 | -0.80 | 0.03511896 | P56470 | LGALS4 | Galectin-4 |
| 55 | 159 | MUC5B_HUMAN | pre | 2 | -0.80 | 0.02047452 | Q9HC84 | MUC5B | Mucin-5B |
| 56 | 185 | VRK1_HUMAN | pre | 2 | -0.87 | 0.04772231 | Q99986 | VRK1 | Serine/threonine-protein kinase VRK1 |

| No. | SEQ ID No. | Uniprot entry name | Pre/diag | Qual score | logFC | adj.p-Val | Uniprot accession | Gene name | Protein name |
|---|---|---|---|---|---|---|---|---|---|
| 57 | 186 | CDKN3_HUMAN | diag | 2 | -0.71 | 0.01978279 | Q16667 | CDKN3 | Cyclin-dependent kinase inhibitor 3 |
| 58 | 188-189 | TFPI2_HUMAN | diag | 2 | -0.79 | 0.03075045 | P48307 | TFPI2 | Tissue factor pathway inhibitor 2 |
| 59 | 195-197 | ARHG2_HUMAN | pre | 1 | -0.41 | 0.03565782 | Q92974 | ARHGEF2 | Rho guanine nucleotide exchange factor 2 |
| 60 | 226-231 | LMNA_HUMAN | pre | 1 | -0.63 | 0.03353007 | P02545 | LMNA | Prelamin-A/C |
| 61 | 232 | PYR1_HUMAN | pre | 1 | -0.65 | 0.01284425 | P27708 | CAD | CAD protein |
| 62 | 214-217 | DAPK1_HUMAN | pre | 1 | -0.65 | 0.02261542 | P53355 | DAPK1 | Death-associated protein kinase 1 |
| 63 | 213 | CUED2_HUMAN | pre | 1 | -0.70 | 0.01007946 | Q9H467 | CUEDC2 | CUE domain-containing protein 2 |
| 64 | 236 | CP1B1_HUMAN | diag | 1 | -0.56 | 0.01978279 | Q16678 | CYP1B1 | Cytochrome P450 1B1 |
| 65 | 247 | SSR4_HUMAN | pre | 0 | -0.40 | 0.02825626 | P31391 | SSTR4 | Somatostatin receptor type 4 |
| 66 | 237 | ANGT_HUMAN | pre | 0 | -0.42 | 0.04109223 | P01019 | AGT | Angiotensinogen |
| 67 | 238-240 | APC_HUMAN | pre | 0 | -0.42 | 0.02825626 | P25054 | APC | Adenomatous polyposis coli protein |
| 68 | 248 | TNAP3_HUMAN | pre | 0 | -0.49 | 0.03707986 | P21580 | TNFAIP3 | Tumor necrosis factor alpha-induced protein 3 |

**Clinical example**

AKI is a major complication in critically ill patients and after major surgeries. Particularly in cardiac surgery, patients are usually older and often have pre-existing conditions such as hypertension and diabetes mellitus. Both are risk factors for renal insufficiency. These patients are particularly at risk of developing acute kidney injury (AKI) with a frequency up to 40%. In many cases, the deterioration of kidney function is recognized with a delay.

A concrete example of everyday clinical practice is a 72-year-old female patient suffering from mitral and tricuspid valve insufficiency with long-term hypertension as the underlying disease. Before surgery the patient had a reduced renal function of 45% and the operation had to be performed with the use of a heart-lung machine and the administration of blood products is required. After surgery the patient had a volume surplus through the intravascular fluid administration of about 5 L meaning an increase of creatinine as a common clinical parameter was not recognized and thus made an early diagnosis of AKI extremely difficult. By analyzing patient's plasma before surgery using a predictive test e.g. an immuno-based Point-of-care device containing a biomarker combination of 5-10 markers, the risk profile could be determined in advance and alternative procedures for valve replacement can be discussed to prevent AKI development. Furthermore, it could be considered in the case of blood product administration whether short-term stored red cell concentrates are indicated showing low amount of free heme as a kidney damaging hemoglobin degradation product. After surgery it took 7 days of dehydration to re-assess creatinine as a renal parameter and diagnose AKI in this patient. Using a diagnostic biomarker combination shortly after surgery in a time window of 6 to 24 hours post OP the onset and severity of AKI could be detected at an early stage and medication adjusted to the renal situation. One could consider kidney stabilizing drugs and/or avoid or adjust dosages of nephrotoxic substances like antibiotics thereby improving patients outcome and preventing secondary conditions like chronic kidney disease.

Literature

Basile, D. P., Anderson, M. D., & Sutton, T. A. (2012). Pathophysiology of acute kidney injury. Comprehensive Physiology, 2(2), 1303-1353. https://doi.org/10.1002/cphy.c110041

Bellomo, R., Ronco, C., Kellum, J. A., Mehta, R. L., Palevsky, P., & Acute Dialysis Quality Initiative workgroup. (2004). Acute renal failure - definition, outcome measures, animal models, fluid therapy and information technology needs: the Second International Consensus Conference of the Acute Dialysis Quality Initiative (ADQI) Group. Critical Care (London, England), 8(4), R204-212. https://doi.org/10.1186/cc2872

Brown, J. R., Rezaee, M. E., Nichols, E. L., Marshall, E. J., Siew, E. D., & Matheny, M. E. (2016). Incidence and In-Hospital Mortality of Acute Kidney Injury (AKI) and Dialysis-Requiring AKI (AKI-D)

After Cardiac Catheterization in the National Inpatient Sample. Journal of the American Heart Association, 5(3), e002739. https://doi.org/10.1161/JAHA.115.002739

Charrin, S., Jouannet, S., Boucheix, C., & Rubinstein, E. (2014). Tetraspanins at a glance. Journal of Cell Science, 127(Pt 17), 3641-3648. https://doi.org/10.1242/jcs.154906

Chuang, P. Y., & He, J. C. (2010). JAK/STAT signaling in renal diseases. Kidney International, 78(3), 231-234. https://doi.org/10.1038/ki.2010.158

Dowdy, S.M., & Wearden, S. (1983) Statistics for research. John Wiley & Sons

Drubin, D. G., & Nelson, W. J. (1996). Origins of Cell Polarity. Cell, 84(3), 335-344. https://doi.org/10.1016/S0092-8674(00)81278-7

Feng, D.-F., & Doolittle, R. F. (1987). Progressive sequence alignment as a prerequisiteto correct phylogenetic trees. Journal of Molecular Evolution, 25(4), 351-360. https://doi.org/10.1007/BF02603120

Fredriksson, S., Gullberg, M., Jarvius, J., Olsson, C., Pietras, K., Gústafsdóttir, S. M., Östman, A., & Landegren, U. (2002). Protein detection using proximity-dependent DNA ligation assays. Nature Biotechnology, 20(5), 473-477. https://doi.org/10.1038/nbt0502-473

Fuhrman, D. Y., Kane-Gill, S., Goldstein, S. L., Priyanka, P., & Kellum, J. A. (2018). Acute kidney injury epidemiology, risk factors, and outcomes in critically ill patients 16-25 years of age treated in an adult intensive care unit. Annals of Intensive Care, 8(1). https://doi.org/10.1186/s13613-018-0373-y

Higgins, D. G., & Sharp, P. M. (1989). Fast and sensitive multiple sequence alignments on a microcomputer. Bioinformatics, 5(2), 151-153. https://doi.org/10.1093/bioinformatics/5.2.151

Hobson, C., Singhania, G., & Bihorac, A. (2015). Acute Kidney Injury in the Surgical Patient. Critical Care Clinics, 31(4), 705-723. https://doi.org/10.1016/j.ccc.2015.06.007

Hsu, M.-F., Bettaieb, A., Ito, Y., Graham, J., Havel, P. J., & Haj, F. G. (2017). Protein tyrosine phosphatase Shp2 deficiency in podocytes attenuates lipopolysaccharide-induced proteinuria. Scientific Reports, 7(1), 461. https://doi.org/10.1038/s41598-017-00564-3

Kalisvaart, M., Schlegel, A., Umbro, I., de Haan, J. E., Scalera, I., Polak, W. G., IJzermans, J. N. M., Mirza, D. F., Perera, M. T. P. R., Isaac, J. I., Ferguson, J., Mitterhofer, A. P., de Jonge, J., & Muiesan, P. (2018). The Impact of Combined Warm Ischemia Time on Development of Acute Kidney

Injury in Donation After Circulatory Death Liver Transplantation: Stay Within the Golden Hour. Transplantation, 102(5), 783-793. https://doi.org/10.1097/TP.0000000000002085

Kinsey, G. R., & Okusa, M. D. (2012). Role of leukocytes in the pathogenesis of acute kidney injury. Critical Care (London, England), 16(2), 214. https://doi.org/10.1186/cc11228

Kogon, A., & Hingorani, S. (2010). Acute Kidney Injury in Hematopoietic Cell Transplantation. Seminars in Nephrology, 30(6), 615-626. https://doi.org/10.1016/j.semnephrol.2010.09.009

Lopes, J. A., & Jorge, S. (2013). The RIFLE and AKIN classifications for acute kidney injury: A critical and comprehensive review. Clinical Kidney Journal, 6(1), 8-14. https://doi.org/10.1093/ckj/sfs160

McEver, Rodger P. 2015. "Selectins: Initiators of Leucocyte Adhesion and Signalling at the Vascular Wall." Cardiovascular Research 107(3):331-39.

Mehta, R. L., Kellum, J. A., Shah, S. V., Molitoris, B. A., Ronco, C., Warnock, D. G., Levin, A., & Acute Kidney Injury Network. (2007). Acute Kidney Injury Network: report of an initiative to improve outcomes in acute kidney injury. Critical Care (London, England), 11(2), R31. https://doi.org/10.1186/cc5713

Needleman, S. B., & Wunsch, C. D. (1970). A general method applicable to the search for similarities in the amino acid sequence of two proteins. Journal of Molecular Biology, 48(3), 443-453. https://doi.org/10.1016/0022-2836(70)90057-4

Nezvitsky, L., Tremblay, M. L., Takano, T., Papillon, J., & Cybulsky, A. V. (2014). Complement-mediated glomerular injury is reduced by inhibition of protein-tyrosine phosphatase 1B. American Journal of Physiology-Renal Physiology, 307(5), F634-F647. https://doi.org/10.1152/ajprenal.00191.2014

Nolan, J. P., & Sklar, L. A. (2002). Suspension array technology: Evolution of the flat-array paradigm. Trends in Biotechnology, 20(1), 9-12. https://doi.org/10.1016/S0167-7799(01)01844-3

Pang, M., Ma, L., Gong, R., Tolbert, E., Mao, H., Ponnusamy, M., Chin, Y. E., Yan, H., Dworkin, L. D., & Zhuang, S. (2010). A novel STAT3 inhibitor, S3I-201, attenuates renal interstitial fibroblast activation and interstitial fibrosis in obstructive nephropathy. Kidney International, 78(3), 257-268. https://doi.org/10.1038/ki.2010.154

Pulli, T., Höyhtyä, M., Söderlund, H., & Takkinen, K. (2005). One-Step Homogeneous Immunoassay for Small Analytes. Analytical Chemistry, 77(8), 2637-2642. https://doi.org/10.1021/ac048379I

Satoh, N., Nakamura, M., Suzuki, M., Suzuki, A., Seki, G., & Horita, S. (2015). Roles of Akt and SGK1 in the Regulation of Renal Tubular Transport. BioMed Research International, 2015, 1-8. https://doi.org/10.1155/2015/971697

Smith, T. F., & Waterman, M. S. (1981). Comparison of biosequences. Advances in Applied Mathematics, 2(4), 482-489. https://doi.org/10.1016/0196-8858(81)90046-4

Wehbe, E., Brock, R., Budev, M., Xu, M., Demirjian, S., Schreiber, M. J., & Stephany, B. (2012). Short-term and long-term outcomes of acute kidney injury after lung transplantation. The Journal of Heart and Lung Transplantation, 31(3), 244-251. https://doi.org/10.1 016/j.healun.2011.08.016

Wehbe, E., Duncan, A. E., Dar, G., Budev, M., & Stephany, B. (2013). Recovery from AKI and short-and long-term outcomes after lung transplantation. Clinical Journal of the American Society of Nephrology: CJASN, 8(1), 19-25. https://doi.org/10.2215/CJN.04800512

**Claims**

1. A method for predicting the risk of occurrence of acute kidney injury (AKI) or for early diagnosis of AKI in a subject, comprising the steps of:

   a. determining in a sample obtained from the subject the amount of at least one biomarker having a logFC of less than -0.7 and being selected from the group consisting of CD15, CD9 antigen and Myeloblastin, as well as isoforms, fragments and variants thereof, and
   b. comparing the amount of said at least one biomarker with a reference amount for said at least one biomarker, wherein the reference amount is the amount of the respective biomarker in healthy subjects, such as subjects who are not at risk of developing AKI and/or who are not having AKI.

2. The method according to claim 1, wherein a reduced amount of the at least one biomarker compared to the reference amount indicates that the subject has AKI or is at risk of developing AKI.

3. The method according to claim 1 or 2, comprising the step of predicting whether the subject is at risk of developing AKI and/or has AKI.

4. The method according to at least one of the preceding claims, including determining in the sample the amount of at least CD15 and Dickkopf-related protein 2, or isoforms, fragments or variants thereof.

5. The method according to at least one of the preceding claims, including determining in the sample the amount of at least CD9 antigen and Myeloblastin, or isoforms, fragments or variants thereof.

6. The method according to at least one of the preceding claims, including determining in the sample the amount of at least Dickkopf-related protein 2 and Interferon alpha-1/13, or isoforms, fragments or variants thereof.

7. The method according to at least one of the preceding claims, including determining in the sample the amount of Dickkopf-related protein 2 and Hyaluronan mediated motility receptor, or isoforms, fragments or variants thereof.

8. The method according to at least one of the preceding claims, wherein the sample is a urine, blood, plasma or serum sample.

9. The method according to at least one of the preceding claims, wherein the sample is taken prior to a planned medical intervention such as administration of a drug, avoiding administration of a drug, injection of contrast media or a surgical intervention.

10. The method according to at least one of the preceding claims, wherein one, two, three or more further biomarkers are determined in the sample, wherein further biomarkers are selected from one or more of the protein biomarkers, male patient biomarkers, predictive biomarkers, diagnostic biomarkers, or combined predictive and diagnostic biomarkers, wherein

    - the protein biomarkers are selected from one or more of CD9 antigen, Prostaglandin G/H synthase 2, CD15, CD99 antigen, High affinity immunoglobulin epsilon receptor subunit alpha, ligands of Tumor necrosis factor receptor superfamily member 1B including Tumor necrosis factor and Lymphotoxin-alpha, Tumor necrosis factor receptor superfamily member 6, Interferon alpha-1/13, Basigin, C-C motif, chemokine 7, Dickkopf-related protein 2, Hyaluronan mediated motility receptor, Interleukin-18, Interleukin-7, Major prion protein, Receptor-type tyrosine-protein phosphatase C, P-selectin glycoprotein ligand 1, Tumor necrosis factor ligand superfamily member 14, DNA topoisomerase 2-alpha, Brain-derived neurotrophic factor, Caspase-8, Eotaxin, C-C motif chemokine 3, C-C motif chemokine 5, Monocyte differentiation antigen CD14, Cytokine receptor-like factor 2, Lamin-B1, Cellular tumor antigen p53, Serine/threonine-protein kinase PAK 1, Caspase-9, Transforming growth factor-beta-induced protein ig-h3, Leukocyte surface antigen CD47, T-cell surface glycoprotein CD8 alpha chain, Dickkopf-related protein 3, Growth arrest-specific protein 6, Interleukin-15, Cytokine receptor common subunit beta, Keratin, type II cytoskeletal 8, Leukosialin, MAP/microtubule affinity-regulating kinase 4, Melanophilin, Interstitial collagenase, Matrilysin, Prostaglandin G/H synthase 1, Myeloblastin, RNA-binding protein 3, Serum amyloid P-component, Tetraspanin-16, Urokinase-type plasminogen activator, CTP synthase 1, CD139, Max dimerization protein 4, Transmembrane protein 54, Actin, cytoplasmic 1, Caspase-3, Complement decay-accelerating factor, High mobility group protein B2, Homeobox protein, Hox-C11, Intercellular adhesion molecule 1, Interleukin-12 subunit alpha, Krueppel-like factor 8, Galectin-4, Ragulator complex protein LAMTOR1, L-selectin, Mitogen-activated protein kinase 3, Mucin-5B, Nuclear factor of activated T-cells, Transforming growth factor beta-1 proprotein, Serine/threonine-protein kinase VRK1, Cyclin-dependent kinase inhibitor 3, Tissue factor pathway inhibitor 2, Microtubule-associated proteins 1A/1B light chain 3B, Phosphatidylinositol 3,4,5-trisphosphate 3-phosphatase and dual-specificity protein phosphatase PTEN, Interleukin-8, Rho guanine nucleotide exchange factor 2, CASP8 and FADD-like apoptosis regulator, CUE domain-containing protein 2, Death-associated protein kinase 1, Endothelin-1 receptor, Eukaryotic translation initiation factor 3 subunit B, DNA-binding protein inhibitor ID-2, Prelamin-A/C, CAD protein, Zinc finger protein 593, Mitogen-activated protein kinase 12, Cytochrome P450 1B1, Angiotensinogen, Adenomatous polyposis coli protein, POU domain class 2 transcription factor 1, Somatostatin receptor type 4, Tumor necrosis factor alpha-induced protein 3, and E3 ubiquitin-protein ligase TRIM22 as well as isoforms, fragments and variants thereof;

- the male patient biomarkers are selected from one or more of CD15, ligands of Tumor necrosis factor receptor superfamily member 1B including Tumor necrosis factor and Lymphotoxin-alpha, Lamin-B1, MAP/microtubule affinity-regulating kinase 4, Dickkopf-related protein 2, Krueppel-like factor 8, Rho guanine nucleotide exchange factor 2, CUE domain-containing protein 2, Death-associated protein kinase 1, DNA-binding protein inhibitor ID-2, Prelamin-A/C, Adenomatous polyposis coli protein, POU domain class 2 transcription factor 1, Somatostatin receptor type 4, and Tumor necrosis factor alpha-induced protein 3 as well as isoforms, fragments and variants thereof;

- the predictive biomarkers are selected from one or more of CD15, CD99 antigen, High affinity immunoglobulin epsilon receptor subunit alpha, Ligands of Tumor necrosis factor receptor superfamily member 1B including Tumor necrosis factor and Lymphotoxin-alpha, Tumor necrosis factor receptor superfamily member 6, Basigin, C-C motif chemokine 7, CD9 antigen, Dickkopf-related protein 2, Hyaluronan mediated motility receptor, Interleukin-18, Interleukin-7, Major prion protein, Receptor-type tyrosine-protein phosphatase C, P-selectin glycoprotein ligand 1, Tumor necrosis factor ligand superfamily member 14, DNA topoisomerase 2-alpha, Brain-derived neurotrophic factor, Caspase-8, Eotaxin, C-C motif chemokine 3, C-C motif chemokine 5, Monocyte differentiation antigen CD14, Cytokine receptor-like factor 2, Lamin-B1, Cellular tumor antigen p53, Serine/threonine-protein kinase PAK 1, Transforming growth factor-beta-induced protein ig-h3, Leukocyte surface antigen CD47, T-cell surface glycoprotein CD8 alpha chain, Dickkopf-related protein 3, Growth arrest-specific protein 6, Interferon alpha-1/13, Interleukin-15, Cytokine receptor common subunit beta, Keratin type II cytoskeletal 8, Leukosialin, MAP/microtubule affinity-regulating kinase 4, Melanophilin, Interstitial collagenase, Matrilysin, Prostaglandin G/H synthase 1, Myeloblastin, RNA-binding protein 3, Serum amyloid P-component, Tetraspanin-16, Urokinase-type plasminogen activator, Actin cytoplasmic 1, Caspase-3, Complement decay-accelerating factor, High mobility group protein B2, Homeobox protein Hox-C11, Intercellular adhesion molecule 1, Interleukin-12 subunit alpha, Interleukin-8, Krueppel-like factor 8, Galectin-4, Ragulator complex protein, LAMTOR1, L-selectin, Mitogen-activated protein kinase 3, Mucin-5B, Nuclear factor of activated T-cells cytoplasmic 4, Transforming growth factor beta-1 proprotein, Serine/threonine-protein kinase VRK1, Rho guanine nucleotide exchange factor, CASP8 and FADD-like apoptosis regulator, CUE domain-containing protein 2, Death-associated protein kinase 1, Endothelin-1 receptor, Eukaryotic translation initiation factor 3 subunit, DNA-binding protein inhibitor ID-2, Prelamin-A/C, CAD protein, Zinc finger protein 593, Angiotensinogen, Adenomatous polyposis coli protein, POU domain, class 2, transcription factor 1, Somatostatin receptor type 4, and Tumor necrosis factor alpha-induced protein 3 as well as isoforms, fragments and variants thereof;

- the diagnostic biomarkers are selected from one or more of Cytokine receptor-like factor 2, Prostaglandin G/H synthase 2, Interferon alpha-1/13, Caspase-9, Interleukin-18, Interleukin-7, CTP synthase 1, C-C motif chemokine 7, CD139, Eotaxin, Max dimerization protein 4, Interleukin-15, RNA-binding protein 3, Transmembrane protein 54, Krueppel-like factor 8, Interstitial collagenase, Cyclin-dependent kinase inhibitor 3, Tissue factor pathway inhibitor 2, Growth arrest-specific protein 6, Microtubule-associated proteins 1A/1B light chain 3B, Caspase-8, Phosphatidylinositol 3,4,5-trisphosphate 3-phosphatase and dual-specificity protein phosphatase PTEN, Mitogen-activated protein kinase 12, Cytochrome P450 1B1, and E3 ubiquitin-protein ligase TRIM22 as well as isoforms, fragments and variants thereof;

- the combined predictive and diagnostic biomarkers are selected from one or more of Cytokine receptor-like factor 2, Caspase-8, Eotaxin, C-C motif chemokine 7, Growth arrest-specific protein 6, Interferon alpha-1/13, Interleukin-15, Interleukin-18, Interleukin-7, Krueppel-like factor 8, Interstitial collagenase, and RNA-binding protein 3 as well as isoforms, fragments and variants thereof.

11. The method according to at least one of the preceding claims, wherein the fragments, isoforms and/or variants of the biomarkers have at least 70%, at least 80%, at least 90%, at least 95%, at least 98%, or at least 99% sequence identity with the biomarker over the whole length of the sequence.

12. The method according to at least one of the preceding claims, wherein determining the amount of said at least one biomarker comprises

using an immunoassay device, such as ELISA (enzyme-linked immunosorbent assay) or antibody array, in particular a planar antibody microarray or a bead based antibody microarray.

13. Device for predicting the risk of occurrence of acute kidney injury (AKI) in a subject or for early diagnosis of AKI, comprising

- an analyzing unit for the said sample of the subject comprising a detection agent for at least one biomarker, or variants or fragments thereof, said detection agent allowing for the determination of the amount of the said at least one biomarker in the sample, and
- an evaluation unit comprising a data processing unit and a data base, said data base comprising a stored reference and said data processing unit being capable of carrying out a comparison of the amount of the at least one biomarker determined by the analyzing unit and the stored reference thereby establishing the prediction,
- wherein the biomarker is at least one selected from the group consisting of CD15, CD9 antigen and Myeloblastin as well as isoforms, fragments and variants thereof.

14. A kit comprising a detection agent for determining the amount of at least one biomarker, and evaluation instructions for establishing the prediction or early diagnosis of AKI, wherein the biomarker is at least one selected from the group consisting of CD15, CD9 antigen and Myeloblastin as well as isoforms, fragments and variants thereof.

15. Kit according to claim 14, wherein the detection agent is selected from the group consisting of antibodies and aptamers.

**Europäisches Patentamt**
**European Patent Office**
**Office européen des brevets**

# EUROPEAN SEARCH REPORT

Application Number

EP 20 16 8527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROHIT J. KATE ET AL: "Prediction and detection models for acute kidney injury in hospitalized older adults", BMC MEDICAL INFORMATICS AND DECISION MAKING, vol. 16, no. 1, 29 March 2016 (2016-03-29), XP055622649, DOI: 10.1186/s12911-016-0277-4 * (p 2, col 2, para 3 ff; fig 5) * | 1-15 | INV. G01N33/68 |
| A | SIMONA POZZOLI ET AL: "Predicting acute kidney injury: current status and future challenges", JOURNAL OF NEPHROLOGY : JN, vol. 31, no. 2, 17 April 2018 (2018-04-17), pages 209-223, XP055535728, IT ISSN: 1121-8428, DOI: 10.1007/s40620-017-0416-8 * table 1 * | 1-15 | |
| A | JOANA GAMEIRO ET AL: "Artificial Intelligence in Acute Kidney Injury Risk Prediction", JOURNAL OF CLINICAL MEDICINE, vol. 9, no. 3, 3 March 2020 (2020-03-03), page 678, XP055713546, DOI: 10.3390/jcm9030678 * (p p 1-2, bridging paragraph) * | 1-15 | TECHNICAL FIELDS SEARCHED (IPC) G01N |
| A | WO 2013/041913 A1 (UNIV DE LOS ANDES [CL]; IRARRAZABAL MUNOZ CARLOS ERNESTO [CL]) 28 March 2013 (2013-03-28) * abstract; claims 1-7; 13 * | 1-15 | |

-/--

The present search report has been drawn up for all claims

| Place of search | Date of completion of the search | Examiner |
|---|---|---|
| Munich | 13 July 2020 | Bigot-Maucher, Cora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

## EUROPEAN SEARCH REPORT

Application Number

EP 20 16 8527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/115885 A1 (CARIS LIFE SCIENCES LUXEMBOURG HOLDINGS S A R L [LU] ET AL.) 30 August 2012 (2012-08-30) | 14,15 | |
| A | * paragraph [0257] * | 1-13 | |
| X | MAHDI SALIH ET AL: "Urinary extracellular vesicles and the kidney: biomarkers and beyond", AMERICAN JOURNAL OF PHYSIOLOGY: RENAL PHYSIOLOGY, vol. 306, no. 11, 1 June 2014 (2014-06-01) , pages F1251-F1259, XP055701235, United States ISSN: 1931-857X, DOI: 10.1152/ajprenal.00128.2014 | 14,15 | |
| A | * page 48, paragraph 1 * | 1-13 | |
| X | HONG IN-KEE ET AL: "Homophilic interactions of tetraspanin CD151 up-regulate motility and matrix metalloproteinase-9 expression of human melanoma cells through adhesion-dependent c-Jun activation signaling pathways", JOURNAL OF BIOLOGICAL CHEMISTRY, AMERICAN SOCIETY FOR BIOCHEMISTRY AND MOLECULAR BIOLOGY, US, vol. 281, no. 34, 1 August 2006 (2006-08-01), pages 24279-24292, XP009138925, ISSN: 0021-9258, DOI: 10.1074/JBC.M601209200 | 14,15 | |
| A | * page 24280, column 1, last paragraph * | 1-13 | |
| X | WO 2004/007685 A2 (UNIV TENNESSEE RES FOUNDATION [US]) 22 January 2004 (2004-01-22) | 14,15 | TECHNICAL FIELDS SEARCHED (IPC) |
| A | * page 13, paragraph 2 * | 1-13 | |

-/--

The present search report has been drawn up for all claims

| Place of search | Date of completion of the search | Examiner |
|---|---|---|
| Munich | 13 July 2020 | Bigot-Maucher, Cora |

EPO FORM 1503 03.82 (P04C01)

page 2 of 3

Europäisches
Patentamt

European
Patent Office

Office européen
des brevets

## EUROPEAN SEARCH REPORT

Application Number

EP 20 16 8527

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BORIES D ET AL: "Down-regulation of a serine protease, myeloblastin, causes growth arrest and differentiation of promyelocytic leukemia cells", CELL, ELSEVIER, AMSTERDAM, NL, vol. 59, no. 6, 22 December 1989 (1989-12-22), pages 959-968, XP027461592, ISSN: 0092-8674, DOI: 10.1016/0092-8674(89)90752-6 [retrieved on 1989-12-22] | 14,15 | |
| A | * p 961, col 2, last para * | 1-13 | |
| X | WO 91/09865 A1 (UNIV COLUMBIA [US]) 11 July 1991 (1991-07-11) | 14,15 | |
| A | * abstract * | 1-13 | |
| X | PO-TSANG LEE ET AL: "Mouse Kidney Progenitor Cells Accelerate Renal Regeneration and Prolong Survival After Ischemic Injury", STEM CELLS (MIAMISBURG), 1 January 2010 (2010-01-01), pages N/A-N/A, XP055701241, ISSN: 1066-5099, DOI: 10.1002/stem.310 | 14,15 | TECHNICAL FIELDS SEARCHED (IPC) |
| A | * p 574, col 2, last lines * | 1-13 | |

The present search report has been drawn up for all claims

| Place of search | Date of completion of the search | Examiner |
|---|---|---|
| Munich | 13 July 2020 | Bigot-Maucher, Cora |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 3 of 3

## ANNEX TO THE EUROPEAN SEARCH REPORT
## ON EUROPEAN PATENT APPLICATION NO.

EP 20 16 8527

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2020

| Patent document cited in search report | | Publication date | Patent family member(s) | | Publication date |
|---|---|---|---|---|---|
| WO 2013041913 | A1 | 28-03-2013 | AU | 2011377379 A1 | 10-04-2014 |
| | | | BR | 112014006741 A2 | 28-03-2017 |
| | | | CA | 2849577 A1 | 28-03-2013 |
| | | | CN | 103917869 A | 09-07-2014 |
| | | | EP | 2758774 A1 | 30-07-2014 |
| | | | HK | 1199754 A1 | 17-07-2015 |
| | | | JP | 5893742 B2 | 23-03-2016 |
| | | | JP | 2014528076 A | 23-10-2014 |
| | | | KR | 20140076571 A | 20-06-2014 |
| | | | RU | 2014115999 A | 27-10-2015 |
| | | | US | 2014329334 A1 | 06-11-2014 |
| | | | WO | 2013041913 A1 | 28-03-2013 |
| WO 2012115885 | A1 | 30-08-2012 | AU | 2012220872 A1 | 12-09-2013 |
| | | | CA | 2827894 A1 | 30-08-2012 |
| | | | CN | 103492590 A | 01-01-2014 |
| | | | EP | 2678448 A1 | 01-01-2014 |
| | | | JP | 2014507160 A | 27-03-2014 |
| | | | US | 2014141986 A1 | 22-05-2014 |
| | | | WO | 2012115885 A1 | 30-08-2012 |
| WO 2004007685 | A2 | 22-01-2004 | AU | 2003253918 A1 | 02-02-2004 |
| | | | US | 2004136985 A1 | 15-07-2004 |
| | | | WO | 2004007685 A2 | 22-01-2004 |
| WO 9109865 | A1 | 11-07-1991 | AU | 7247891 A | 24-07-1991 |
| | | | US | 5180819 A | 19-01-1993 |
| | | | WO | 9109865 A1 | 11-07-1991 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5744305 A

**Non-patent literature cited in the description**

- *UniProt release,* 13 February 2019
- *The UniProt Consortium,* 2017
- **DOWDY ; WEARDEN.** Statistics for Research. John Wiley & Sons, 1983
- **BASILE, D. P. ; ANDERSON, M. D. ; SUTTON, T. A.** Pathophysiology of acute kidney injury. *Comprehensive Physiology,* 2012, vol. 2 (2), 1303-1353, https://doi.org/10.1002/cphy.c110041
- **BELLOMO, R. ; RONCO, C. ; KELLUM, J. A. ; MEHTA, R. L. ; PALEVSKY, P.** Acute Dialysis Quality Initiative workgroup. (2004). Acute renal failure - definition, outcome measures, animal models, fluid therapy and information technology needs: the Second International Consensus Conference of the Acute Dialysis Quality Initiative (ADQI) Group. *Critical Care,* vol. 8 (4), R204-212, https://doi.org/10.1186/cc2872
- **BROWN, J. R. ; REZAEE, M. E. ; NICHOLS, E. L. ; MARSHALL, E. J. ; SIEW, E. D. ; MATHENY, M. E.** *Incidence and In-Hospital Mortality of Acute Kidney Injury (AKI) and Dialysis-Requiring AKI (AKI-D),* 2016
- After Cardiac Catheterization in the National Inpatient Sample. *Journal of the American Heart Association,* vol. 5 (3), e002739, https://doi.org/10.1161/JAHA.115.002739
- **CHARRIN, S. ; JOUANNET, S. ; BOUCHEIX, C. ; RUBINSTEIN, E.** Tetraspanins at a glance. *Journal of Cell Science,* 2014, vol. 127, 3641-3648, https://doi.org/10.1242/jcs.154906
- **CHUANG, P. Y. ; HE, J. C.** JAK/STAT signaling in renal diseases. *Kidney International,* 2010, vol. 78 (3), 231-234, https://doi.org/10.1038/ki.2010.158
- **DOWDY, S.M. ; WEARDEN, S.** Statistics for research. John Wiley & Sons, 1983
- **DRUBIN, D. G. ; NELSON, W. J.** Origins of Cell Polarity. *Cell,* 1996, vol. 84 (3), 335-344, https://doi.org/10.1016/S0092-8674(00)81278-7

- **FENG, D.-F. ; DOOLITTLE, R. F.** Progressive sequence alignment as a prerequisitetto correct phylogenetic trees. *Journal of Molecular Evolution,* 1987, vol. 25 (4), 351-360, https://doi.org/10.1007/BF02603120
- **FREDRIKSSON, S. ; GULLBERG, M. ; JARVIUS, J. ; OLSSON, C. ; PIETRAS, K. ; GÚSTAFSDÓTTIR, S. M. ; ÖSTMAN, A. ; LANDEGREN, U.** Protein detection using proximity-dependent DNA ligation assays. *Nature Biotechnology,* 2002, vol. 20 (5), 473-477, https://doi.org/10.1038/nbt0502-473
- **FUHRMAN, D. Y. ; KANE-GILL, S. ; GOLDSTEIN, S. L. ; PRIYANKA, P. ; KELLUM, J. A.** Acute kidney injury epidemiology, risk factors, and outcomes in critically ill patients 16-25 years of age treated in an adult intensive care unit. *Annals of Intensive Care,* 2018, vol. 8 (1, https://doi.org/10.1186/s13613-018-0373-y
- **HIGGINS, D. G. ; SHARP, P. M.** Fast and sensitive multiple sequence alignments on a microcomputer. *Bioinformatics,* 1989, vol. 5 (2), 151-153, https://doi.org/10.1093/bioinformatics/5.2.151
- **HOBSON, C. ; SINGHANIA, G. ; BIHORAC, A.** Acute Kidney Injury in the Surgical Patient. *Critical Care Clinics,* 2015, vol. 31 (4), 705-723, https://doi.org/10.1016/j.ccc.2015.06.007
- **HSU, M.-F. ; BETTAIEB, A. ; ITO, Y. ; GRAHAM, J. ; HAVEL, P. J. ; HAJ, F. G.** Protein tyrosine phosphatase Shp2 deficiency in podocytes attenuates lipopolysaccharide-induced proteinuria. *Scientific Reports,* 2017, vol. 7 (1), 461, https://doi.org/10.1038/s41598-017-00564-3
- **KALISVAART, M. ; SCHLEGEL, A. ; UMBRO, I. ; DE HAAN, J. E. ; SCALERA, I. ; POLAK, W. G. ; IJZERMANS, J. N. M. ; MIRZA, D. F. ; PERERA, M. T. P. R. ; ISAAC, J. I.** *The Impact of Combined Warm Ischemia Time on Development of Acute Kidney,* 2018
- Injury in Donation After Circulatory Death Liver Transplantation: Stay Within the Golden Hour. *Transplantation,* vol. 102 (5), 783-793, https://doi.org/10.1097/TP.0000000000002085

- **KINSEY, G. R. ; OKUSA, M. D.** Role of leukocytes in the pathogenesis of acute kidney injury. *Critical Care (London, England),* 2012, vol. 16 (2), 214, https://doi.org/10.1186/cc11228
- **KOGON, A. ; HINGORANI, S.** Acute Kidney Injury in Hematopoietic Cell Transplantation. *Seminars in Nephrology,* 2010, vol. 30 (6), 615-626, https://doi.org/10.1016/j.semnephrol.2010.09.009
- **LOPES, J. A. ; JORGE, S.** The RIFLE and AKIN classifications for acute kidney injury: A critical and comprehensive review. *Clinical Kidney Journal,* 2013, vol. 6 (1), 8-14, https://doi.org/10.1093/ckj/sfs160
- **MCEVER, RODGER P.** Selectins: Initiators of Leucocyte Adhesion and Signalling at the Vascular Wall. *Cardiovascular Research,* 2015, vol. 107 (3), 331-39
- **MEHTA, R. L. ; KELLUM, J. A. ; SHAH, S. V. ; MOLITORIS, B. A. ; RONCO, C. ; WARNOCK, D. G. ; LEVIN, A.** Acute Kidney Injury Network. (2007). Acute Kidney Injury Network: report of an initiative to improve outcomes in acute kidney injury. *Critical Care (London, England),* vol. 11 (2), R31, https://doi.org/10.1186/cc5713
- **NEEDLEMAN, S. B. ; WUNSCH, C. D.** A general method applicable to the search for similarities in the amino acid sequence of two proteins. *Journal of Molecular Biology,* 1970, vol. 48 (3), 443-453, https://doi.org/10.1016/0022-2836(70)90057-4
- **NEZVITSKY, L. ; TREMBLAY, M. L. ; TAKANO, T. ; PAPILLON, J. ; CYBULSKY, A. V.** Complement-mediated glomerular injury is reduced by inhibition of protein-tyrosine phosphatase 1B. *American Journal of Physiology-Renal Physiology,* 2014, vol. 307 (5), F634-F647, https://doi.org/10.1152/ajprenal.00191.2014
- **NOLAN, J. P. ; SKLAR, L. A.** Suspension array technology: Evolution of the flat-array paradigm. *Trends in Biotechnology,* 2002, vol. 20 (1), 9-12, https://doi.org/10.1016/S0167-7799(01)01844-3
- **PANG, M. ; MA, L. ; GONG, R. ; TOLBERT, E. ; MAO, H. ; PONNUSAMY, M. ; CHIN, Y. E. ; YAN, H. ; DWORKIN, L. D. ; ZHUANG, S.** A novel STAT3 inhibitor, S3I-201, attenuates renal interstitial fibroblast activation and interstitial fibrosis in obstructive nephropathy. *Kidney International,* 2010, vol. 78 (3), 257-268, https://doi.org/10.1038/ki.2010.154
- **PULLI, T. ; HÖYHTYÄ, M. ; SÖDERLUND, H. ; TAKKINEN, K.** One-Step Homogeneous Immunoassay for Small Analytes. *Analytical Chemistry,* 2005, vol. 77 (8), 2637-2642, https://doi.org/10.1021/ac048379I
- **SATOH, N. ; NAKAMURA, M. ; SUZUKI, M. ; SUZUKI, A. ; SEKI, G. ; HORITA, S.** Roles of Akt and SGK1 in the Regulation of Renal Tubular Transport. *BioMed Research International,* 2015, 1-8, https://doi.org/10.1155/2015/971697
- **SMITH, T. F. ; WATERMAN, M. S.** Comparison of biosequences. *Advances in Applied Mathematics,* 1981, vol. 2 (4), 482-489, https://doi.org/10.1016/0196-8858(81)90046-4
- **WEHBE, E. ; BROCK, R. ; BUDEV, M. ; XU, M. ; DEMIRJIAN, S. ; SCHREIBER, M. J. ; STEPHANY, B.** Short-term and long-term outcomes of acute kidney injury after lung transplantation. *The Journal of Heart and Lung Transplantation,* 2012, vol. 31 (3), 244-251, https://doi.org/10.1016/j.healun.2011.08.016
- **WEHBE, E. ; DUNCAN, A. E. ; DAR, G. ; BUDEV, M. ; STEPHANY, B.** Recovery from AKI and short-and long-term outcomes after lung transplantation. *Clinical Journal of the American Society of Nephrology: CJASN,* 2013, vol. 8 (1), 19-25, https://doi.org/10.2215/CJN.04800512